# EUROPÄISCHE PATENTSCHRIFT

(11) **EP 1 504 002 B1**
(45) Veröffentlichungstag und Bekanntmachung des Hinweises auf die Patenterteilung: **19.12.2012**
(21) Anmeldenummer: 03720507.7
(22) Anmeldetag: 22.04.2003
(51) Int. Cl.: C07D 401/06

(54) **VERFAHREN ZUR HERSTELLUNG OPTISCH AKTIVER BETA-AMINOKETONE UND OPTISCH AKTIVER 1.3-AMINOALKOHOLE**
METHOD FOR THE PRODUCTION OF OPTICALLY ACTIVE BETA-AMINOKETONES AND OPTICALLY ACTIVE 1,3-AMINOALCOHOLS
PROCEDE DE PREPARATION DE BETA-AMINOCETONES OPTIQUEMENT ACTIVES ET D' 1,3-AMINOALCOOLS OPTIQUEMENT ACTIFS

(30) Priorität: 03.05.2002 DE 10219987
(43) Veröffentlichungstag der Anmeldung: 09.02.2005
(73) Patentinhaber: Sanofi-Aventis Deutschland GmbH, 65929 Frankfurt am Main (DE)
(72) Erfinder: JENDRALLA, Heiner, 65931 Frankfurt (DE); SCHWAB, Wilfried, 65207 Wiesbaden-Naurod (DE); STUEDEMANN, Thomas, 65779 Kelkheim (DE)
(86) Internationale Anmeldenummer: PCT/EP2003/004127
(87) Internationale Veröffentlichungsnummer: WO 2003/093259

(56) Entgegenhaltungen:
- WO-A-00/20392
- WO-A-00/20393

## Beschreibung

Aminoalkylierungen von CH-aciden Verbindungen sind seit ca. 100 Jahren bekannt. Sie werden als Mannich-Reaktionen bezeichnet und gehören zu den bedeutendsten C-C-Verknüpfungsreaktionen der Organischen Chemie.

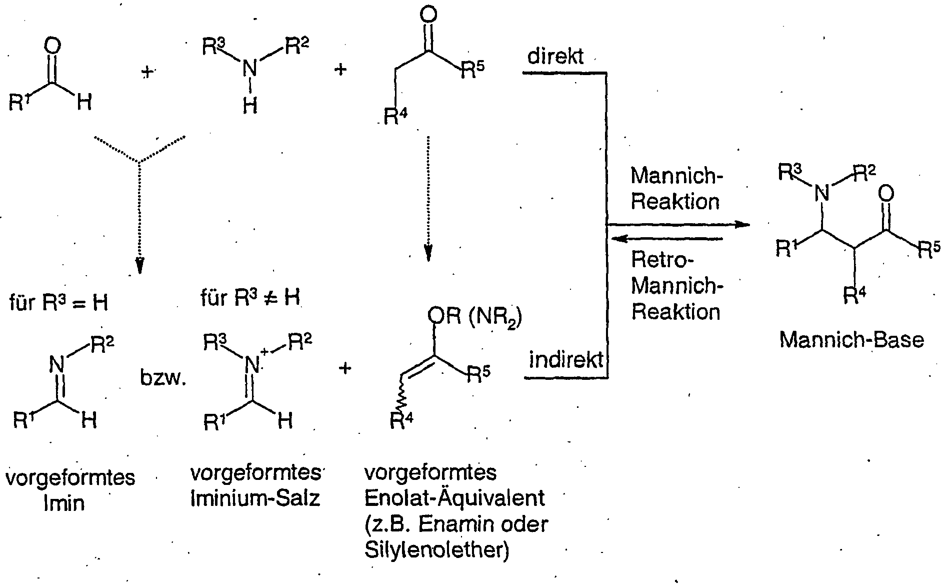

In seiner ursprünglichen und bekanntesten Form wird die Mannich-Reaktion mit drei Edukten in Form einer "Dreikomponenten-Kupplung" durchgeführt: Ein enolisierbares Keton, ein nicht enolisierbarer Aldehyd (häufig Formaldehyd oder ein Arylaldehyd) und eine Amin-Komponente (Ammoniak oder ein primäres oder sekundäres Amin) reagieren miteinander unter Bildung eines β-Aminoketons. In dieser "Mannich-Base" ist der aktive Wasserstoff des enolisierbaren Ketons durch einen Aminoalkyl-Substituenten ersetzt worden. Diese direkte Variante der Mannich-Reaktion ist industriell besonders attraktiv, denn die drei genannten Edukte sind meistens gut verfügbar und preiswert, zumindest aber sehr gut zugänglich. Außerdem sind diese Edukte in der Regel unempfindlich (d.h. gut lagerbar) und erlauben deshalb ein einfaches Handling. Schließlich stellt die direkte Dreikomponenten-Kupplung kommerzieller Edukte eine einstufige, d.h. denkbar kürzeste, Synthese von β-Aminoketonen dar.

Daneben gibt es industriell weniger attraktive indirekte Varianten der Mannich-Reaktion, in denen vorgeformte Enolat-Äquivalente (meistens Enamine oder Silylenolether) eingesetzt werden. Diese Verbindungen sind in der Regel nicht kommerziell erhältlich oder sind teuer. Ihre vorherige Herstellung stellt eine zusätzliche Synthesestufe dar. Weiterhin sind insbesondere die Trimethylsilylenolether und in geringerem Maße auch die Enamine säure- und hydrolyseempfindlich, schlecht lagerbar und schwierig zu handhaben. Silylenolether mit bestimmten anderen Silylgruppen sind zwar etwas stabiler, aber teuer in der Herstellung. Die hohe Nucleophilie der vorgeformten Enolat-Äquivalente hat Vor- und Nachteile. Sie erlaubt einerseits häufig milde Reaktionsbedingungen und ermöglicht so gelegentlich Mannich-Reaktionen, die in der direkten Variante von zu vielen Nebenreaktionen begleitet sind. Andererseits sind die Aminomethylierungen von vorgeformten Enolat-Äquivalenten häufig Tieftemperaturreaktionen und damit im industriellen Maßstab aufwendig und teuer. Weitere Nachteile stereoselektiver Varianten mit vorgeformten Enolat-Äquivalenten sind der Einsatz industriell problematischer Lewissäure-Katalysatoren, schlechte Löslichkeiten von Reaktionskomponenten bei der tiefen Temperatur und darauf beruhend die Notwendigkeit des Einsatzes großer Lösungsmittelmengen (schlechte Raum/Zeit-Ausbeuten) oder Verwendung problematischer oder teurer Lösungsmittel. Iminium-Salze sind in der Mannich-Reaktion deutlich reaktiver (elektrophiler) als Imine. Dies ist mit Vor- und Nachteilen verbunden, die den oben für vorgeformte Enolat-Äquivalente beschriebenen ähnlich sind.

Asymmetrische Mannich-Reaktionen sind beispielsweise beschrieben in M. Arend et al. (Angew. Chem. Int. Ed. Engl. 1998, 37, 1044 - 1070), worin auf Seite 1067 festgestellt wird: "Der Zugang zu enantiomerenreinen Mannich-Basen steckt trotz vielversprechender Überlegungen und einiger wichtiger Erfolge noch in den Anfängen. [...] Bedenkt man die breite Vielfalt der in-situ und ohne Racemisierung möglichen Derivatisierungen der kinetischen Produkte (z.B. zu Aminoalkoholen, Diaminen und Aminen), dann wird der berechtigte und auch realistische Wunsch nach leistungsfähigen und möglichst allgemein anwendbaren Verfahren zur Kontrolle der absoluten Konfiguration der Produkte verständlich. Katalytische Verfahren, die in weiten anderen Bereichen der Stereochemie bereits als etabliert gelten können, sind hier nahezu unerforscht".

Die Verwendung von stöchiometrischen Mengen von chiralen Auxiliaren in einer asymmetrischen Mannich-Reaktion wird beispielsweise beschrieben von H. Ishitani et al. (J. Am. Chem. Soc. 2000, 122, 8180-8186). Diese Methode besitzt keine industrielle Relevanz, da das chirale Auxiliar kovalent mit dem vorgeformten Imin (bzw. seltener mit dem vorgeformten Enolat-Äquivalent) verknüpft wird, um die Mannich-Reaktion als diastereoselektive Addition zu führen. Aufbau, Anknüpfen und, nach erfolgter Mannich-Reaktion, Abspaltung des chiralen Auxiliars erfordern mehrere zusätzliche Synthesestufen. Die Mannich-Additionen waren zudem häufig Tieftemperatur-Reaktionen, die chiralen Auxiliare schlecht zugänglich bzw. nur in einer Absolutkonfiguration verfügbar.

Katalytische asymmetrische Mannich-Varianten wurden zusammengefasst von S.E. Denmark & O.J.-C. Nicaise ("Catalytic Enantioselective Mannich-Type Reactions" in Comprehensive Asymmetric Catalysis, E.N. Jacobsen, A. Pfaltz, H. Yamamoto, Eds.; Springer-Verlag: New York, 1999; Vol. 2, Chapter 26.2.9; Seite 954-958). Die katalytischen Varianten sind größtenteils indirekte Mannich-Reaktionen, was ihre industrielle Attraktivität einschränkt. Außerdem müssen komplizierte chirale Übergangsmetallkatalysatoren eingesetzt werden.

Direkte asymmetrische Dreikomponenten Mannich-Reaktionen mit unmodifizierten Ketonen lassen sich durch heteropolymetallische chirale Katalysatoren auf Lanthanid-Basis induzieren, ergeben allerdings wie in S. Yamasaki et al. (Tetrahedron Lett. 1999, 40, 307-310) beschrieben nur mäßige chemische Ausbeuten (≤ 16%) und Enantiomeren-Überschüsse (≤ 64% ee).

Die erste direkte katalytische asymmetrische Dreikomponenten-Mannich-Reaktion, die industriellen Ansprüchen nahe kommt, wurde erst kürzlich bekannt (B. List, J. Am. Chem. Soc. 2000, 122, 9336-9337; vgl. H. Gröger & J. Wilken, Angew. Chem. Int. Ed. Engl. 2001, 40, 529 - 532). Darin werden unmodifizierte Ketone mit Aryl- oder Alkyl-Aldehyden und bestimmten Anilin-Derivaten unter Katalyse mit 35 mol% (L)-Prolin in Dimethylsulfoxid oder Chloroform bei Raumtemperatur zu optisch aktiven Mannich-Basen umgesetzt. Die chemischen Ausbeuten waren mäßig bis gut (35-90%), die optischen Reinheiten mittel bis sehr gut (73-96% ee).

Mannich-Basen und ihre Derivate haben zahlreiche industrielle Anwendungen, die in M. Arend et al. (Angew. Chem. 1998, 110, 1096-1122) auf Seite 1045 zusammengestellt sind. Das wichtigste Einsatzgebiet, insbesondere von chiralen Mannich-Basen, ist die Herstellung von Wirkstoffen für Arzneimittel, wie z.B. dem Neuroleptikum Moban. Hierzu wird in Arend et al. auf Seite 1047 festgestellt: "Für die enantioselektive Synthese von β-Aminoketonen und -aldehyden ist die klassische Mannich-Reaktion nicht geeignet. Die meisten medizinischen Wirkstoffe, die sich von Mannich-Basen ableiten, werden daher bislang als Racemate eingesetzt. Enantiomerenreine Mannich-Basen und deren Derivate verwendet man nur dann zur Herstellung von Medikamenten, wenn sie durch Racematspaltung zugänglich sind. Dieser Nachteil fällt umso stärker ins Gewicht, wenn man sich vergegenwärtigt, daß die Bedeutung stereochemisch einheitlicher Wirkstoffe ständig zunimmt (Vermeidung von Isomerenballast und von unerwünschten Nebenwirkungen)."

Racemische β-Aminoketone, die sich durch ein Gemisch einer Verbindung der Formel (I) und deren Enantiomer beschreiben lassen, 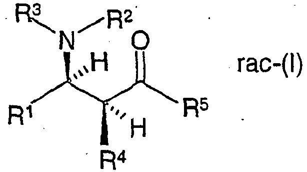 in denen die Substituenten R¹ = Phenyl, R² = H, R³ = Phenyl, R⁴ = Methyl und R⁵ = Phenyl sind, sind beschrieben in T. Akiyama et al, Synlett 1999, 1045-1048;
in denen R¹ = p-Tolyl, R² = H, R³ = p-Methoxycarbonylphenyl, R⁴ = Methyl und R⁵ = Phenyl sind, sind beschrieben in N. Shida et al, Tetrahedron Lett. 1995, 36, 5023-5026;
in denen R¹ = Phenyl, R² = H, R³ = p-Chlorphenyl, R⁴ = Methyl und R⁵ = Phenyl sind, sind beschrieben in CA120: 257988.
in denen R¹ = tert.-Butyl oder Phenyl, R² = R³ = R⁴ = Methyl und R⁵ = Phenyl sind, sind beschrieben in E.G. Nolen et al, Tetrahedron Lett. 1991, 32, 73-74.

Chirale 1,3-Aminoalkohole besitzen, wie z.B. das Analgetikum Tramadol, Bedeutung als Arzneimittelwirkstoffe, sowie als chirale Hilfsstoffe für asymmetrische Synthesen, beispielsweise dokumentiert in S. Cicchi et al. ("Synthesis of new enantiopure β-amino alcohols: their use as catalysts in the alkylation of benzaldehyde by diethylzinc", Tetrahedron: Asymmetry 1997, 8, 293-301).

Die begrenzt diastereoselektive Reduktion von Mannich-Basen mit LiAlH₄ wurde bereits 1985 von J. Barluenga et al. ("Diastereoselective synthesis of β-amino alcohols with three chiral centers by reduction of β-amino ketones and derivatives" J. Org. Chem. 1985, 50, 4052-4056) beschrieben.
Chirale 1,3-Aminoalkohole der Formel (II), 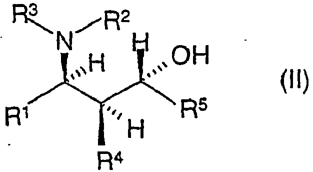 d.h. mit (SR,RS,SR)-Konfiguration, konnten bisher nicht mit industriell brauchbaren Diastereoselektivitäten aus Mannich-Basen der Formel (I) hergestellt werden.

Die Zuordnung der (R)- oder (S) - Konfiguration beruht auf den Prioritätsregeln von Cahn, Ingold und Prelog. Diese Priorisierung kann sich umkehren, wenn man einen oder mehrere Substituenten modifiziert. Die Bezeichnung (SR,RS,SR) besagt, daß bei dieser Verbindung das mittlere Stereozentrum (das R⁴ als Substituent trägt) die (R)-Konfiguration hat, wenn die beiden äußeren Stereozentren (S)-Konfiguration haben (dies ist die in Formel II gezeichnete Konfiguration) oder aber, daß das mittlere Stereozentrum (S)-Konfiguration hat, wenn die beiden äußeren Stereozentren (R)-Konfiguration haben (dies ist das Spiegelbild der in Formel (II) gezeichneten Konfiguration). Die Konfiguration des Stereoisomers hängt von der Wahl des chiralen Anions Y*⁻ *vide infra* ab. Die oben genannte Konfigurationsbezeichnung (SR,RS,SR) bezieht sich auf das Modellprodukt wie in den Beispielen konkretisiert, kann sich bei anderen Verbindungen oder Substituenten aber umkehren. Eindeutig wird die Stereochemie der Verbindung der Formel (II) durch die Strukturformel wiedergegeben.

Eine mehrstufige enzymatische Methode zur Produktion von chiralen 1,3-Aminoalkoholen, ausgehend von racemischen Butan-1,4-diolen, ist beschrieben im US-Patent US 5,916,786.

Bei der Carbonyl-Reduktion von α-chiralen β-Aminoketonen mit LiAlH₄ (Lithiumaluminiumhydrid) oder mit Wasserstoff in Gegenwart von Platin-Katalysatoren entsteht bevorzugt der 1,3-Aminoalkohol dia-(II), dessen Hydroxykonfiguration entgegengesetzt (diastereomer) gegenüber Formel (II) ist, wenn der Aminosubstituent tertiär ist, 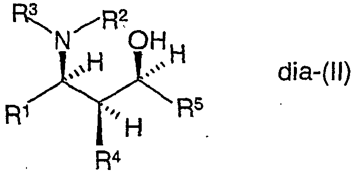 und es entsteht ein etwa äquimolares Gemisch der Diastereomere (II) und dia-(II), wenn der Aminosubstituent sekundär ist (M.-J. Brienne et al., Bull. Soc. Chim. France 1969, 2395; A. Andrisano & L. Angiolini Tetrahedron 1970, 26, 5247).

Die Patentanmeldung EP 1117645 beschreibt optisch aktive 1,3-Aminoalkohole der Formel (II), wobei R¹ = o-Aminophenyl, R² = H, R³ = R⁴ = 2-Pyridyl und R⁵ = Phenyl oder 3,5-Dimethylisoxazol-4-yl sind, die über eine vorherige klassische Racematspaltung hergestellt worden waren.

WO 00/20392 und WO 00/20393 beschreiben substituierte 1,3-Diaryl-2-pyridin-2-yl-3-(pyridin-2-ylamino)-propanol-Derivate sowie deren Herstellung, wobei in einem ersten Schritt durch Umsetzung eines vorgeformten Imins mit einem enolisierbaren Keton eine Mannich-Verbindung hergestellt wird, anschließend die Mannich-Verbindung reduziert wird und durch Trennung der so erhaltenen Isomere das gewünschte Racemat des entsprechenden 1,3-Aminoalkohol-Enantiomere isoliert wird.

Überraschenderweise wurde gefunden, daß Verbindungen der Formel (III) oder deren Diastereomer (III A),

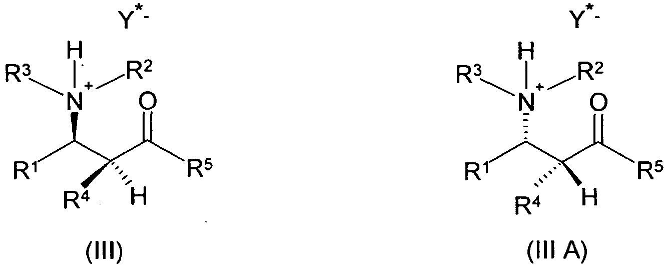

Salze der β-Aminoketone der Formel (I) *vide infra,* deren Kation hohe Enantiomeren- überschüsse und sehr hohe Diastereomerenreinheit (syn / anti - Verhältnis) aufweist, in einfacher Weise durch direkte Vierkomponenten-Kupplung, basierend auf einer dynamischen Racematspaltung, in hoher Ausbeute hergestellt werden können.

Das Kation von (III A) ist enantiomer zum Kation von (III). Da das Anion Y^{*-} aber homochiral ist, stellt die Verbindung (III A) ein Diastereomer zur Verbindung (III) dar.

Gegenstand der vorliegende Erfindung ist daher ein Verfahren zur Herstellung einer Verbindung der Formel (III) oder deren Diastereomer (IIIA), 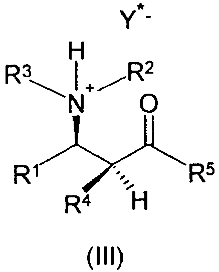
wobei R¹
   1. Wasserstoff,
   2. tert.-Butyl-Gruppe oder
   3. ein carbocyclischer oder heterocyclischer Arylrest R⁶ ist, wobei der Arylrest R⁶ ein carbocyclischer Arylrest mit 5-14 C-Atomen oder ein heterocyclischer Arylrest mit 5-14 C-Atomen ist, wobei 1 bis 4 C-Atome durch N, O oder S ersetzt sind,
      wobei R⁶ unsubstituiert ist oder 1 bis 5 Substituenten trägt, die unabhängig voneinander (C₁-C₇)Alkyl, (C₃-C₇)Cycloalkyl, Alkanoyl (-CO-(C₁-C₇)Alkyl), Aroyl (-CO-(C₅-C₁₄)Aryl), Fluoro, Chloro, Bromo, lodo, Hydroxy, (C₁-C₇)Alkoxy, (C₃-C₇)Cycloalkoxy, (C₅-C₁₄)Aryloxy, (C₁-C₇)Alkanoyloxy, (C₅-C₁₄)Aroyloxy, -O-CO-NHR, -O-CO-NRR', -O-CO-OR, -O-CO-SR, -O-CS-NHR, -O-CS-NRR', -O-CS-OR, -O-CS-SR, -O-SO₂-(C₁-C₇)Alkyl, -O-SO₂-(C₅-C₁₄)Aryl), Nitro, -NH-CO-R, -NR'-CO-R, -NH-CO-OR, -NR'-CO-OR, -NH-CO-NHR, -NR'-CO-NHR, -NR'-CO-NRR", Di(C₁-C₇)alkylamino, Di(C₅-C₁₄)arylamino, N-(C₁-C₇)Alkyl-N-(C₅-C₁₄)aryl-amino, (C₁-C₇)Alkylthio, (C₅-C₁₄)Arylthio, (C₁-C₇)Alkylsulfonyl, (C₅-C₁₄)Arylsulfonyl, (C₅-C₁₄)Arylsulfoxidyl, oder ein unsubstituierter Arylrest R⁶ bedeuten,
      wobei R, R' und R" jeweils unabhängig voneinander (C₁-C₇)Alkyl, (C₃-C₇)Cycloalkyl oder (C₅-C₁₄)Aryl bedeuten,
   vorzugsweise ein Arylrest mit 6-10 C-Atomen, besonders bevorzugt ein carbocyclischer Arylrest mit 6-10 C-Atomen,
   ferner bevorzugt ein Rest aus der Gruppe Phenyl, Naphthyl, Anthracenyl, Phenanthrenyl, Pyridyl, Chinolinyl, Isochinolinyl, Benzochinolinyl, Pyridazinyl, Pyrimidinyl, Pyrazinyl, Triazinyl, Tetrazinyl, Benzopyridazinyl, Benzopyrimidinyl, Benzopyrazinyl (Chinoxalinyl), Benzotriazinyl, Pyridopyridinyl, Pyridochinolinyl (Phenanthrolinyl), Benzochinoxalinyl (Phenazinyl), Pyrrolyl, Benzopyrrolyl (Indolyl), Benzoindolyl, Pyrazolyl, Benzopyrazolyl, Imidazolyl, Benzimidazolyl, Triazolyl, Benzotriazolyl, Tetrazolyl, Imidazopyrimidinyl (9H-Purinyl), Furanyl, Benzofuranyl, Dibenzofuranyl, Thiophen, Benzothiophen, Dibenzothiophen, Isoxazolyl, Benzisoxazolyl, Oxazolyl, Benzoxazolyl, Oxadiazolyl, Benzoxadiazolyl, Thiazolyl, Benzothiazolyl, Isothiazolyl, Benzisothiazolyl, Thiadiazolyl oder Benzthiadiazolyl,
   besonders bevorzugt ein Rest R⁷, wobei R⁷ definiert ist als Rest aus der Gruppe Phenyl, Naphthyl, Pyridyl, Chinolinyl, Isochinolinyl, Benzochinolinyl, wobei R⁷ unsubstituiert oder mit bis zu 5 Substituenten versehen ist, die unabhängig voneinander bedeuten: (C₁-C₇)Alkyl, (C₃-C₇)Cycloalkyl, Fluoro, Chloro, Bromo, (C₁-C₇)Alkoxy, (C₃-C₇)Cycloalkoxy, (C₅-C₁₄)Aryloxy, (C₁-C₇)Alkanoyloxy, (C₅-C₁₄)Aroyloxy, -O-CO-NHR, -O-CO-NRR', -O-CO-OR, Nitro, Phenyl, Naphthyl, Pyridyl, Chinolinyl, Isochinolinyl oder Benzochinolinyl,
   speziell bevorzugt ein carbocyclischer oder heterocyclischer Arylrest R⁸, wobei R⁸ definiert ist als ein Rest aus der Gruppe Phenyl, Naphthyl, Pyridyl oder Chinolinyl, wobei R⁸ unsubstituiert oder mit bis zu 5 Substituenten versehen ist, die unabhängig voneinander bedeuten: Nitro, Fluoro, Chloro oder Bromo,
R², R³ und R⁴ unabhängig voneinander
   1. Wasserstoff,
   2. (C₁-C₇)Alkyl,
      wobei (C₁-C₇)Alkyl unsubstituiert oder substituiert ist mit einem Arylrest R⁶,
   3. (C₃-C₇)Cycloalkyl oder
   4. ein Arylrest R⁶ bedeutet,
   vorzugsweise unabhängig voneinander Wasserstoff oder ein Arylrest R⁷,
   besonders bevorzugt unabhängig voneinander Wasserstoff oder ein Arylrest R⁸,
R⁵ ein Arylrest R⁶ ist,
   bevorzugt ein Arylrest R⁷,
   besonders bevorzugt ein Arylrest R⁸,
und wobei das Anion Y^{*-} die konjugierte Base einer optisch aktiven, organischen Brönstedt-Säure (Protonen-Säure) ist,
vorzugsweise eine optisch aktive natürlich vorkommende oder industriell hergestellte Carbonsäure, beispielsweise (R)-(-)-Mandelsäure, (S)-(+)-Mandelsäure, D-(-)-Weinsäure, L-(+)-Weinsäure, (+)-Di-O,O'-pivaloyl-D-weinsäure [(+)-DPWS], (-)-Di-O,O'-pivaloyl-L-weinsäure, [(-)-DPWS], (+)-O,O'-Dibenzoyl-D-weinsäure, (-)-O,O'-Dibenzoyl-L-weinsäure, (-)-Di-O,O'-benzoyl-L-weinsäure-mono(dimethylamid), (+)-O,O'-Dianisoyl-D-weinsäure [(+)-DAWS], (-)-O,O'-Dianisoyl-L-weinsäure [(-)-DAWS], (+)-Di-O,O'-p-Toluyl-D-weinsäure, (-)-Di-O,O'-p-Toluyl-L-weinsäure, D-(+)-Äpfelsäure, L-(-)-Äpfelsäure, L-(+)-Milchsäure, D-(-)-Milchsäure, (S)-(-)-2-(Phenylaminocarbonyloxy)-propionsäure, (R)-(+)-2-(Phenylaminocarbonyloxy)-propionsäure, D-(+)-Gluconsäure, (-)-2,3,4,6-Di-O-isopropyliden-2-keto-L-gulonsäure, (D)-(-)-Chinasäure, (-)-3,4,5-Trihydroxy-1-cyclohexen-1-carbonsäure [Shikimisäure], (S)-(+)-(2,2-Dimethyl-5-oxodioxolan-4-yl)-essigsäure, (+)-Camphersäure, (-)-Camphersäure, (1R)-(+)-Camphansäure, (1 S)-(-)-Camphansäure, (R)-(-)-O-Acetylmandelsäure, (S)-(+)-O-Acetylmandelsäure, (R)-2-Phenoxy-propionsäure, (S)-2-Phenoxy-propionsäure, (S)-(+)-α-Methoxyphenylessigsäure, (R)-(-)-α-Methoxyphenylessigsäure, (R)-(+)-α-Methoxy-α-trifluormethylphenylessigsäure, (S)-(-)-α-Methoxy-α-trifluormethyl-phenylessigsäure, (S)-(+)-2-Phenyl-propionsäure, (R)-(-)-2-Phenylpropionsäure, (R)-(+)-2-Chlor-propionsäure, (S)-(-)-2-Chlor-propionsäure, (R)-(+)-N-(α-Methylbenzyl)phthalsäuremonoamid, , (S)-(-)-N-(α-Methylbenzyl) phthalsäuremonoamid, (R)-(-)-5-Oxotetrahydrofuran-2-carbonsäure, (S)-(+)-5-Oxotetrahydrofuran-2-carbonsäure, D-(+)-3-Phenylmilchsäure, L-(-)-3-Phenylmilchsäure, L-(+)-α-Hydroxyisovaleriansäure, D-(-)-α-Hydroxyisovaleriansäure, (+)-Menthyloxyessigsäure, (-)-Menthyloxyessigsäure, (+)-mono-(1S)-Menthylphthalat, (-)-mono-(1R)-Menthylphthalat, (+)-trans-5-Norbornen-2,3-dicarbonsäure, (-)-trans-5-Norbornen-2,3-dicarbonsäure, (R)-(+)-Methylbernsteinsäure, (S)-(-)-Methylbernsteinsäure, (R)-(+)-6-Hydroxy-2,5,7,8-tetramethylchroman-2-carbonsäure [(R)-(+)-Trolox^{®}], (S)-(-)-6-Hydroxy-2,5,7,8-tetramethylchroman-2-carbonsäure [(S)-(-)-Trolox^{®}], (S)-(+)-2-(4-Isobutylphenyl)-propionsäure [(S)-Ibuprofen], (R)-(-)-2-(4-Isobutylphenyl)-propionsäure [(R)-Ibuprofen], (+)-2-(6-Methoxy-2-naphthyl)-propionsäure [(+)-Naproxen], (-)-2-(6-Methoxy-2-naphthyl)-propionsäure [(-)-Naproxen], sowie die verfügbaren natürlichen oder unnatürlichen α- oder β-Aminosäuren und deren gut zugängliche Derivate, insbesondere N-acylierte Derivate,
oder eine optisch aktive Sulfonsäuren, beispielsweise (1 S)-(+)-Campher-1 0-sulfonsäure, (1R)-(-)-Campher-10-sulfonsäure, (-)-3-Brom-campher-8-sulfonsäure, (+)-3-Brom-campher-10-sulfonsäure,
oder ein optisch aktives Phosphorsäure-, Phosphinsäure- oder Phosphonsäure-Derivat, beispielsweise (R)-(-)-1,1'-Binaphthalin-2,2'-diyl-hydrogen-phosphat, (S)-(+)-1,1'-Binaphthalin-2,2'-diyl-hydrogenphosphat, (+)-Phosphinothricin, (-)-Phosphinothricin],
oder ein optisch aktives Phenol, beispielsweise (R)-(+)- oder (S)-(-)-Binaphthol,
dadurch gekennzeichnet, daß
die Verbindungen der Formeln (IV), (V), (VI) und (VII) 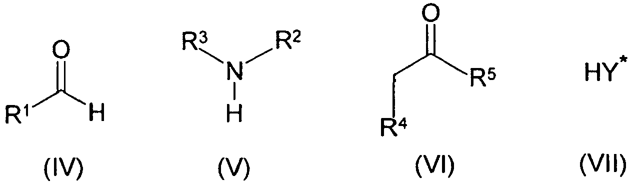 wobei die Reste R¹, R², R³, R⁴ und R⁵ in den Verbindungen der Formeln (IV), (V), (VI) und (VII) wie in der Verbindung der Formel (I) definiert sind,
in einem oder mehreren geeigneten Lösungsmitteln oder ohne Lösungsmittel zur Verbindung der Formel (III) umgesetzt werden,
wobei entweder die Verbindungen der Formeln (IV), (V), (VI) und (VII) in einer direkten Mannich-Reaktion gleichzeitig umgesetzt werden,
oder zunächst die Verbindungen der Formeln (IV) und (V) zu einem Imin der Formel (X) oder zu einem Aminal der Formel (XI) reagieren, das optional isoliert werden kann, 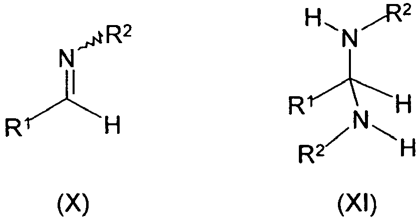 und die Verbindung der Formel (X) oder (XI) anschliessend unter Zusatz der Verbindungen der Formel (VI) und (VII) zu einer Verbindung der Formel (III) umgesetzt werden.

Die oben beschriebene Reaktion zu einer Verbindung der Formel (III) wird im folgenden Verfahrensschritt 1 genannt.

Alkyl- und Alkoxy-Reste können verzweigt oder unverzweigt sein.

(C₁-C₇)Alkyl-Reste sind beispielsweise Methyl, Ethyl, Propyl, iso-Propyl, n-Butyl, isoButyl, tert-Butyl, Pentyl, iso-Pentyl, Neopentyl, Hexyl, Heptyl.

(C₃-C₇)Cycloalkyl sind beispielsweise Cyclopropyl, Cyclobutyl, Cyclopentyl, Cyclohexyl, Cycloheptyl, 2-Methylcyclopentyl, 3-Methylcyclohexyl.

Während des gesamten Anmeldungstextes ist bei Angabe einer Stereoformel darunter entweder die durch die Stereoformel ausgedrückte Absolutkonfiguration oder deren Enantiomer zu verstehen, wobei die Verbindungen stets in einer Enantiomerenreinheit von größer gleich 90% ee, vorzugsweise größer gleich 95% ee, besonders bevorzugt größer gleich 98% ee vorliegen. Insbesondere gilt dies für Verbindungen der Formeln (I), (II) und (III).

Unter einer "klassischen Racematspaltung" verstehen wir während des gesamten Anmeldungstextes die Trennung von Bild und Spiegelbild eines racemischen Stoffes durch Einsatz eines (weitgehend) enantiomerenreinen Hilfsstoffes unter Bildung von diastereomeren Salzen, die aufgrund unterschiedlicher physikalischer Eigenschaften, z.B. unterschiedlicher Löslichkeiten, voneinander getrennt werden, wobei es unter den Bedingungen der Racematspaltung nicht zu einer (signifikanten) Umwandlung von Bild zu Spiegelbild kommt. Die maximal erzielbare Ausbeute des enantiomerenreinen Stoffes mittels einer klassischen Racematspaltung beträgt 50%. Sie unterscheidet sich grundlegend von der "dynamischen Racematspaltung", bei der sich Bild und Spiegelbild unter den Bedingungen der Racematspaltung ineinander umwandeln und darum Ausbeuten des enantiomerenreinen Stoffes bis zu 100% erzielbar sind. Dynamische Racematspaltungen können im Prinzip kinetisch kontrolliert oder thermodynamisch kontrolliert sein. Eine Gruppe von Reaktionen innerhalb der thermodynamisch kontrollierten dynamischen Racematspaltungen sind die Kristallisations-induzierten dynamischen Racematspaltungen. Die in der vorliegenden Erfindung beschriebenen Beispiele gehören zu dieser Gruppe von Reaktionen.

In einer bevorzugten Ausführungsform werden die vier Komponenten der Formel (IV), (V), (VI), (VII) und optional ein geeignetes Lösungsmittel in einen Reaktor gegeben und gerührt. Die Zugabereihenfolge ist unkritisch. Im großen Maßstab ist es, insbesondere wenn es sich bei (IV) - (VII) um Feststoffe handelt, am praktikabelsten, diese Edukte im Reaktor vorzulegen und das Solvent dann, falls notwendig unter Kühlung, zulaufen zu lassen. Anschließend wird das Reaktionsgemisch auf die gewünschte Reaktionstemperatur erwärmt. In der normalen Durchführungsform liegt anfangs eine Lösung vor. Insbesondere wenn eine oder mehrere der vier Komponenten schwerlöslich sind, kann der Verfahrensschritt aber auch so durchgeführt werden, daß die schwerlöslichen Edukte erst mit fortschreitender Reaktion in Lösung gehen. Aufgrund der nach einiger Zeit einsetzenden Kristallisation der Salze (III) und (III A) kann in letzterem Fall während des gesamten Reaktionsverlaufs eine Suspension vorliegen.

Entnimmt man, ausgehend von einer klaren Lösung der Edukte (IV) - (VII) dem Reaktionsgemisch eine Probe, unmittelbar nachdem die Kristallisation der Salze (III) / (III A) eingesetzt hat und filtriert diese Probe, so stellt man bei der Analyse fest, daß im Niederschlag das Salz (III) geringfügig bis mäßig, aber signifikant, gegenüber dem diastereomeren Salz (III A) überwiegt. Im Filtrat liegen die Salze (III) und (III A) hingegen im Verhältnis 1:1 vor. Im weiteren Reaktionsverlauf nimmt die Niederschlagsmenge kontinuierlich zu und das Verhältnis von (III) zu (III A) steigt kontinuierlich an, während es im Filtrat bei 1:1 verharrt. Schließlich geht die Reaktion in einen stationären Zustand über, wobei weder die Niederschlagsmenge, noch das Verhältnis von (III) zu (III A) weiter ansteigt. Die Niederschlagsmenge lag in der Regel bei 85-95% derTheorie und der Enantiomerenüberschuß der Mannich-Base (I) im (III) / (III A)-Niederschlag bei 90-99% ee.

Die Bestimmung des Enantiomeren-Verhältnisses kann wegen der Retro-Mannich-Tendenz von (III) und (III A) in der Regel nicht durch direkte HPLC- oder DC-Analyse erfolgen. Eine Bestimmung per NMR ist zwar prinzipiell möglich, aber wegen Signalüberlagerungen ungenau. Am besten erfolgt die Bestimmung nach Derivatisierung der Probe mit optisch reinem (+)- oder (-)-Camphansäurechlorid (VIII A) oder achiralem Pivaloylchlorid (VIII B) per HPLC:

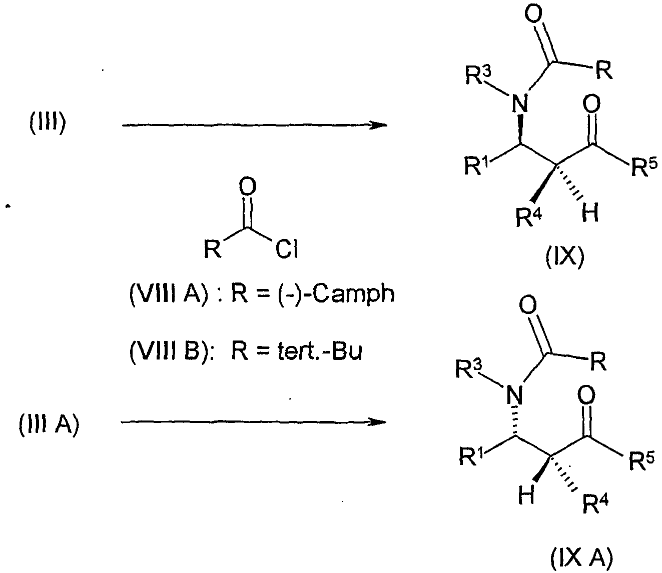

Die N-acylierten Derivate (IX) und (IX A) sind stabil und können keine Retro-Mannich-Reaktion mehr unterlaufen. Die Verwendung von (-)-Camphansäurechlorid hat den Vorteil, daß die Derivate (IX) und (IX A) Diastereomere sind und sich deshalb auf konventionellen HPLC-Säulen mit achiraler stationärer Phase voneinander trennen lassen. Die Methode hat aber den Nachteil, daß es während der Derivatisierung zu einer (meistens geringfügigen) Verfälschung der Stereoisomerenverhältnisse (unerwünschte kinetische Racematspaltung) kommen kann, da die Reaktionsgeschwindigkeiten von (III) und (III A) mit diesem Säurechlorid nicht identisch sind. Mit dem achiralen Pivaloylchlorid (VIII B) müssen (III) und (III A) mit gleicher Geschwindigkeit reagieren, so daß eine Verfälschung der Stereoisomerenverhältnisse hier ausgeschlossen ist. Die Derivatisierungsprodukte (IX) und (IX A) sind in diesem Fall aber Enantiomere, so daß für ihre Trennung eine HPLC-Säule mit chiraler stationärer Phase benötigt wird. Aus den Analysen einer großen Probenzahl ergibt sich, daß die mit (-)-Camphanoylchlorid bestimmten Enantiomeren-Überschüsse gegenüber den zuverlässigeren Bestimmungen mit Pivaloylchlorid um bis zu 4% zu schlechteren ee-Werten verfälscht sind.

Beispielhaft für die zeitliche Zunahme des Anteils des Produktes (III) zu Lasten von (III A) im Niederschlag einer Vierkomponenten-Kupplungsreaktion wurde eine Reaktion untersucht, wobei R¹ = o-Nitrophenyl, R² = 2-Pyridyl, R³ = H, R⁴ = 2-Pyridyl, R⁵ = Phenyl und HY^{*} = (+)-Di-O,O'-pivaloyl-D-weinsäure bedeutet, und wobei das Solvent = Ethanol, und die Reaktionstemperatur = 20-25°C war.

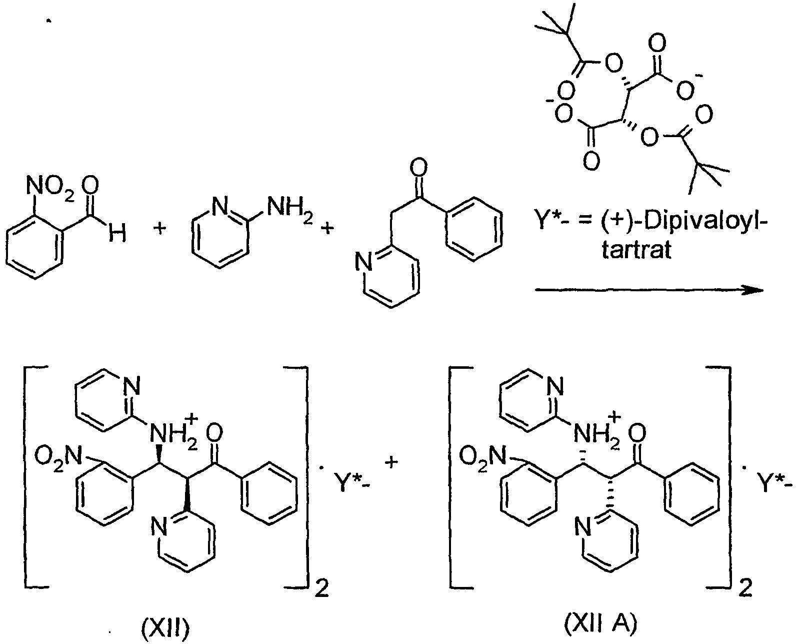

**Tabelle 1: Zeitlicher Verlauf der Bildung einer beispielhaften Verbindung der Formel (III) im Verhältnis zur deren Enantiomer**

| t [h] | Gehalt an (XII) [%] | Gehalt an (XII A) [%] |
|---|---|---|
| 21 | 62,68 | 37,32 |
| 46 | 67,27 | 32,73 |
| 62,5 | 69,67 | 30,33 |
| 130 | 78,40 | 21,60 |
| 154,5 | 83,68 | 16,32 |
| 177 | 86,74 | 13,26 |
| 202 | 89,99 | 10,01 |
| 225 | 94,89 | 5,11 |
| 297 | 96,91 | 3,09 |
| 322 | 97,67 | 2,33 |

Im Niederschlag (XII) / (XII A) kommen jeweils zwei Kationen auf jedes (+)-DPWS-Anion. Das Reaktionsgemisch wurde bei diesem Versuch mit einem Teflon-überschichteten Magnetrührfisch in einem Rundkolben gerührt. Die erste, nach 21 Stunden entnommene Probe, enthielt (III) und (III A) im Verhältnis 62.7 : 37.3. Nach 322 Stunden betrug das Verhältnis 97.7 : 2.3. Dies entspricht einem Enantiomeren-Überschuß der zugrundeliegenden freien Base von 95.4% ee. Der Anstieg des (XII) / (XII A) - Verhältnisses im Niederschlag der Vierkomponenten-Kupplung erfolgt umso rascher, je höher die Reaktionstemperatur ist und zeigt eine deutliche Abhängigkeit vom Lösungsmittel und von der Natur der chiralen Brönstedt-Säure (VII).

Für optimale Resultate wird der erfindungsgemäße Verfahrensschritt 1 bevorzugt unter Einsatz eines Rührers durchgeführt, der besonders effiziente Durchmischung und Zerkleinerung von Feststoffpartikeln in der Reaktionssuspension gewährleistet.

Der erfindungsgemäße Verfahrensschritt 1 kann in Wasser, mit oder ohne Zusatz organischer Lösungsmittel und / oder Löslichkeitsvermittler durchgeführt werden, oder, wenn eines oder mehrere der Edukte (IV) - (VII) bei der Reaktionstemperatur flüssig ist, auch in Abwesenheit von Lösungsmitteln ("neat").

Ein geeignetes Lösungsmittel ist Wasser oder ein organisches Lösungsmittel, oder ein Gemisch von Wasser mit einem organischem Lösungsmittel, optional enthaltend ein löslichkeitsverbesserndes Additiv, z.B. einen Phasentransfer-Katalysator, wobei organische Lösungsmittel in 100%iger Reinheit oder in technischer Qualität vorliegen können und beispielsweise ein C₁-C₈-Alkohol, verzweigt oder unverzweigt, vorzugsweise Methanol, Ethanol, n-Propanol, Isopropanol oder n-Butanol, oder ein ketonisches Lösungsmittel, vorzugsweise Aceton oder Methylethylketon (MEK), oder ein Ester, vorzugsweise Ethylacetat oder n-Butylacetat, oder ein Ether, vorzugsweise Tetrahydrofuran, Methyl-tert.-butylether, Diisopropylether, 1,2-Dimethoxyethan, Diethylenglycoldimethylether (Diglyme), oder ein Kohlenwasserstoff, aliphatisch oder aromatisch, vorzugsweise Toluol, oder ein superkritisches Medium, vorzugsweise superkritisches Kohlendioxid oder ein halogenierter Kohlenwasserstoff, vorzugsweise Dichlormethan, oder ein aprotisch-polares Lösungsmittel, vorzugsweise DMF, DMSO oder NMP sein kann,
und in der Reaktion vorhandenes Wasser optional durch beispielsweise aceotrope Destillation oder den Zusatz wasserbindender Additive wie z.B. Magnesiumsulfat oder aktiviertes Molekularsieb entfernt wird.

Die Reaktion wird bei -15°C bis +140°C, bevorzugt bei +10°C bis +100°C, besonders bevorzugt bei +30°C bis +70°C durchgeführt.

Der Verfahrensschritt 1 kann bei Normaldruck, im Vakuum (*vide supra,* z.B. zwecks Abdestillieren eines Azeotrops) oder unter Druck, letzteres zwecks Reaktionsbeschleunigung, in einer Inertgas-Atmosphäre oder an der Luft durchgeführt werden.

Der erfindungsgemäße Verfahrensschritt 1 wird unter Einsatz von 0.80 - 2.00 Mol-Äquivalenten der Edukte (IV) und (V), sowie 0.80 - 4.00 Mol-Äquivalenten der chiralen Säure (VII), jeweils bezogen auf Edukt (VI), durchgeführt. Bevorzugt wird das erfindungsgemäße Verfahren unter Einsatz von 0.95 - 1.30 Mol-Äquivalenten der Edukte (IV) und (V), sowie 1.00 - 2.00 Mol-Äquivalenten der chiralen Säure (VII), jeweils bezogen auf 1.00 Mol-Äquivalente des Edukts (VI) durchgeführt. Besonders bevorzugt wird das erfindungsgemäße Verfahren unter Einsatz von 1.00 - 1.25 Mol-Äquivalenten der Edukte (IV) und (V), sowie 1.05 - 1.50 Mol-Äquivalenten der chiralen Säure (VII), jeweils bezogen auf 1.00 Mol-Äquivalenten des Edukts (VI), durchgeführt.

Tabelle 2 zeigt die Ergebnisse von Vierkomponentenkupplungen zu einer Verbindung der Formel (III), wobei (+)-Dipivaloyl-D-Weinsäure [(+)-DPWS] als chirale Säure (VII) eingesetzt wurde, 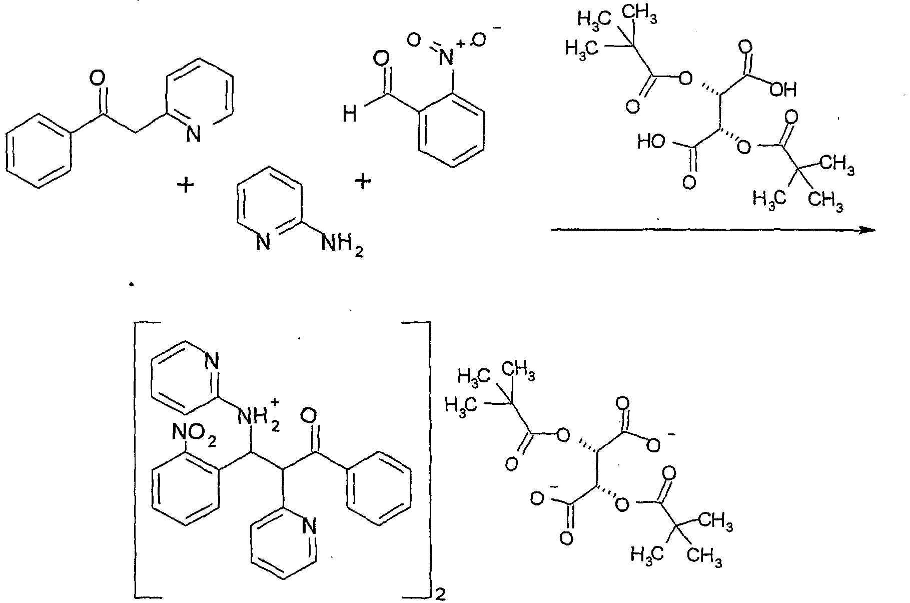 und wobei labortypische Glasreaktionsgefäße (bis 0.5 Mol in Mehrhalsrundkolben, über 0.5 Mol in zylindrischen, am Boden abgerundeten Doppelmantelreaktoren) mit motorgetriebenen mechanischen Rührern (bis 0.5 Mol Glas-KPG-Rührer mit Teflon-Paddeln; über 0.5 Mol Turbinenrührer aus Stahl) eingesetzt wurden.

| Tabelle 2 | | | | | | | | | |
|---|---|---|---|---|---|---|---|---|---|
| Nr. | (IV) (R¹ = 2-Nitro-Ph) mmol Mol-Äquiv | (V) (R² = 2-Py, R³ = H) mmol Mol-Äquiv | (VI) (R⁴ = 2-Py, R⁵ = Ph) mmol Mol-Äquiv | (VII) (+)-Dipivaloyl-D-Weinsäure mmol Mol-Äquiv | Solvent [mL] | Reaktions-bedingungen | (III) isolierte Ausbeute % d. Th. | (III) % ee (HPLC) Camph.-Derivat | (III) % ee (HPLC) Piv.-Derivat |
| 1 | 386.33 | 402.43 | 321.94 | 321.94 | MeOH 756, dann | MeOH 30°C / 55 h dann | 93.6 | 17 h 30°C MeOH 80.5 | - |
| | 1.2 | 1.25 | 1.0 | 1.0 | | | | 30 h 30°C MeOH 83.8 | |
| | | | | | | | | 51 h 30°C MeOH 86.1 | |
| | | | | | EtOH | EtOH 40°C | | 10 h 40°C EtOH 89.5 | |
| | | | | | 756 | / 60 h | | 58 h 40°C EtOH 92.2 | |
| | | | | | | | | isol. (60 h) EtOH 91.8 | |
| 2 | 36 | 37.5 | 30 | 30 | Ethanol | RT/8 Tage; 4-Toluolsulfonsäure Monohydrat (p-TosOH), 0.3 mmol, 0.01 meq | 85.3 | 42 h : 26.7 | - |
| | 1.2 | 1.25 | 1.0 | 1.0 | 75 | | | 91 h : 42.8 | |
| | | | | plus 0.3 mmol, 0.01 meq 4-Toluolsulfonsäure Monohydrat (p-TosOH) | | | | isol. (190 h) 74.0 | |
| 3 | 36 | 37.5 | 30 | 30 | Ethanol | RT / 8 Tage | 83 | 42 h: 30.7 | - |
| | 1.2 | 1.25 | 1.0 | 1.0 | 75 | | | 164 h : 65.1 | |
| | | | | | | | | isol. (190 h) 74.2 | |
| 4 | 152.13 | 158.63 | 126.75 | 126.75 | Ethanol | RT / 11 | 98.2 | 1 Tag: 27.8 | - |
| | 1.2 | 1.25 | 1.0 | 1.0 | 318 | Tage | | 7 Tage: 71.9 | |
| | | | | | | | | isoliert (11 T.) 95.1 | |
| 5 | 28.2 | 29.4 | 23.5 | 23.5 | Ethanol | RT / 14 | 83.6 | 1 Tag: 25.4 | - |
| | 1.2 | 1.25 | 1.0 | 1.0 | 59 | Tage | | 6 Tage: 56.8 | |
| | | | | | | | | 10 Tage: 89.8 | |
| | | | | | | | | 13 Tage: 93.8 | |
| | | | | | | | | isol. (14 T.) 95.3 | |
| 6 | 30.5293 | 31.759 | 25.654 | 25.375 | Ethanol | 40°C / 1 | 99.5 | 4.16 h : 55.7 | - |
| | 1.19 | 1.24 | 1.0 | 1.0 | 70 | Tag | | 20 h : 93.0 | |
| | | | | | | | | isoliert 95.9 | |
| 7 | 34.059 | 35.642 | 28.239 | 28.279 | Ethanol | 40°C / 1 | 94.4 | 5.7 h : 68.9 | 96.8 |
| | 1.206 | 1.262 | 1.0 | 1.0 | 65 | Tag | | 22 h : 95.3 | |
| | | | | | | | | 25 h : 95.5 | |
| | | | | | | | | isoliert 96.6 | |
| 8 | 75.469 | 78.672 | 62.867 | 62.90 | Aceton | 40°C / 17 h | | 1.5 h: 95.2 | 94.6 |
| | 1.20 | 1.25 | 1.0 | 1.0 | 170 | Probe des Produkts bei 40°C in Aceton verrührt | | 17 h : 93.0 | |
| | | | | | | | | isol. 92.5 | |
| | | | | | | | 90.9 | 1 h : 96.4 | 96.4 |

Entsprechend den oben beschriebenen Reaktionen wurden Vierkomponentenkupplungen zu Verbindungen der Formel (III) mittels S-(+)-Mandelsäure durchgeführt.

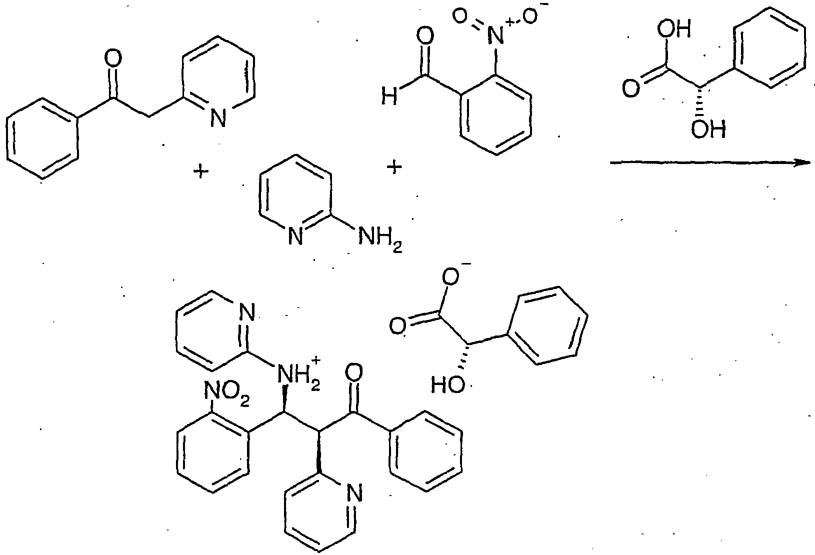

Tabelle 3 zeigt die Ergebnisse von Vierkomponentenkupplungen, wobei R¹ = o-Nitrophenyl, R² = 2-Pyridyl, R³ = H, R⁴ = 2-Pyridyl, und R⁵ = Phenyl ist, und in denen S-(+)-Mandelsäure als Brönstedt-Säure (VII) verwendet wurde:

| Tabelle 3 | | | | | | | | | | |
|---|---|---|---|---|---|---|---|---|---|---|
| Nr. | (IV): R¹ = 2-Nitro-Ph; mmol Mol-Äquiv | (V): R² =2-Py, R³ = H; mmol Mol-Äquiv. | (VI): R⁴ =2-Py, R⁵ = Ph; mmol Mol-Äquiv. | (VII) = (S)-(+)-Mandelsäure mmol Mol-Äquiv. | Solvent [mL] | Reaktionsbedingungen | (III) isolierte Ausbeute % d. Th. | (III) % ee (HPLC) Camph.-Derivat | (III) % ee (HPLC) Piv.-Derivat | (III) syn / anti - Verhältnis (¹H-NMR) |
| 1 | 250.0 | 262.0 | 250.0 | 275.0 | MeOH | 40°C/2h, dann | 83.3 | 95.0 | 97.3 | >99.0 : <1,0 |
| | 1.00 | 1,05 | 1.00 | 1,10 | 400 | 60°C/1h | | | | |
| 2 | 250.0 | 262.0 | 250.0 | 275.0 | MeOH | 60°C / 16h | 78 | 1 h : 92.6 | 97.1 | n.b. |
| | 1.00 | 1.05 | 1,00 | 1.10 | 400 | | | isoliert: 95.6 | | |
| 3 | 91.25 | 91.30 | 76.05 | 152.09 | MeOH | 60°C / 23 h | 67.4 | 95.7 | 95.6 | >99.0 : <1.0 |
| | 1.20 | 1,20 | 1,00 | 2,00 | 195 | | | | | |
| 4 | 770.7 | 800.0 | 643.9 | 1287.8 | EtOH | 40°C / 53 h | 95.3 | 94.4 | n.b. | 99.0 : 1.0 |
| | 1.18 | 1.24 | 1,00 | 2,00 | abs. 1512 | | | | | |
| 5 | 755.5 | 792 | 643,9 | 1314,5 | EtOH (MEK) | 40°C / 44 h | 93,8 | 3,5 h : 6,7 | 95,2 | 98,4 : 1,6 |
| | 1,17 | 1,23 | 1,00 | 2,04 | | | | 21 h : 79.0 | | |
| | | | | | 1510 | | | 25 h: 89,0 | | |
| | | | | | | | | 44 h : 94,8 | | |
| | | | | | | | | isoliert : 95,6 | | |
| 6 | 30,44 | 30,39 | 25,35 | 50,68 | EtOH (MEK) | 60°C / 4 h | 90,9 | 2 h : 94,0 | 95,2 | 99,1 : 0,9 |
| | 1,20 | 1,20 | 1,00 | 2,00 | | | | isoliert : 94,6 | | |
| | | | | | 65 | | | | | |
| 7 | 591,4 | 591,4 | 492,8 | 985,6 | EtOH (MEK) | 60°C / 4,5 h | 92,4 | 2 h : 91,5 | 97,5 | 98,6: 1,4 |
| | 1,20 | 1,20 | 1,00 | 2,00 | | | | 3,5 h : 93,0 | | |
| | | | | | 1200 | | | isoliert : 94,4 | | |
| 8 | 1200 | 1250 | 1000 | 2000 | EtOH (MEK) | 60°C / 6 h | 92,6 | 2 h: 90,7 | 94,6 | 99,0 : 1,0 |
| | 1,20 | 1,25 | 1,00 | 2,00 | | | | 4 h : 92,4 | | |
| | | | | | 2430 | | | 5 h : 92,9 | | |
| | | | | | | | | isoliert : 95,8 | | |
| 9 | 840,0 | 840,0 | 700,0 | 1400,0 | EtOH (MEK) | 60°C / 20 | 90,2 | 2 h : 80,7 | n.b. | 99,4:0,6 |
| | 1,20 | 1,20 | 1,00 | 2,00 | | | | 19 h : 94,2 | | |
| | | | | | 852 | | | isoliert : 94,1 | | |
| 10 . | 30,0 | 31,25 | 25,00 | 37,5 | EtOH (MEK) | 60°C / 7 h | 87,2 | 1,5 h : 56,4 | 94 | 98,9 : 1,1 |
| | 1,20 | 1,25 | 1,00 | 1,50 | | | | 3,5 h : 92,8 | | |
| | | | | | 60 | | | 5,3 h : 92,9 | | |
| | | | | | | | | 93,2 | | |
| 11 | 152,13 | 152,15 | 126,75 | 190,14 | EtOH (MEK) | 60°C / 7 h | 92,2 | 2 h : 61,3 | 94,7 | 98,7 : 1,3 |
| | 1,20 | 1,20 | 1,00 | 1,50 | | | | 4 h : 91,4 | | |
| | | | | | 162,5 | | | 6 h : 92,6 | | |
| | | | | | | | | isoliert : 92,8 | | |
| 12 | 26,61 | 27,24 | 25,35 | 27,88 | EtOH (MEK) | 60°C / 5 h | 77,2 | 2 h : 88,6 | 94 | n.b. |
| | 1,05 | 1,075 | 1,00 | 1,10 | | Stehen 15 h | | 4 h: 94,6 | | |
| | | | | | 65 | bei RT | | Stehen RT:86,2 | | |
| | | | | | | 60°C / 1.5 h | | 6,5 h Rkt. : 95,3 | | |
| | | | | | | | | isoliert : 92,3 | | |
| 13 | 1065 | 1090 | 1014 | 1115 | EtOH (MEK) | 60°C / 7 h | 92,7 | 2 h : 70,9 | 92,9 | 98,5 : 1,5 |
| | 1,05 | 1,075 | 1,00 | 1,10 | | | | 4 h : 93,7 | | |
| | | | | | 1300 | | | 6 h : 94,4 | | |
| | | | | | | | | 7 h Rkt. : 94,2 | | |
| | | | | | | | | isoliert : 93,1 | | |
| 14 | 250,0 | 262,0 | 250,0 | 275,0 | EtOH (MEK) | 60°C / 16 h | 86,1 | 92,2 | 95,4 | >99,0 : <1,0 |
| | 1,00 | 1,05 | 1,00 | 1,10 | | | | | | |
| | | | | | 400 | | | | | |
| 15 | 125,0 | 131,0 | 125,0 | 131,0 | EtOH (MEK) | 60°C / 16 h | 88,1 | 95,2 | 95,4 | >99,0 : <1,0 |
| | 1,00 | 1,05 | 1,00 | 1,05 | | | | | | |
| | | | | | 250 | | | | | |
| 16 | 30,44 | 31,66 | 25,35 | 26,62 | EtOH (MEK) | 60°C / 5 h | 79 | 4 h : 95,1 | | 98,5 : 1,5 |
| | 1,20 | 1,25 | 1,00 | 1,05 | | Stehen 16 h bei RT; | | Stehen RT : 87,9 | 93,5 | |
| | | | | | 65 | | | | | |
| | | | | | | 60°C / 2h | | 7 h Rkt. : 96,4 | | |
| | | | | | | | | isoliert : 93,0 | | |
| 17 | 250,0 | 262,0 | 250,0 | 275,0 | i-PrOH | 60°C / 16 h | 90,7 | 92,8 | 96,3 | n.b. |
| | 1,00 | 1,05 | 1,00 | 1,10 | 400 | | | | | |
| 18 | 250,0 | 262,0 | 250,0 | 275,0 | n-BuOH | 60°C / 16 h | 86,2 | 92,2 | 95,6 | n.b. |
| | 1,00 | 1,05 | 1,00 | 1,10 | 400 | | | | | |
| 19 | 125,0 | 131,0 | 125,0 | 131,0 | n-BuOH | 60°C / 16 h | 87 | 93,4 | 93,3 | n.b. |
| | 1,00 | 1,05 | 1,00 | 1,05 | 250 | | | | | |
| 20 | 591,4 | 591,4 | 492,8 | 985,6 | Aceton | 40°C / 24 h | 88,2 | 95,7 | 97 | 98,6 : 1,4 |
| | 1,20 | 1,20 | 1,00 | 2,00 | 1200 | | | | | |
| 21 | 24,31 | 25,40 | 20,53 | 40,75 | Aceton | 40°C / 27 h | 85,3 | 97,2 | n.b. | 99,0 : 1,0 |
| | 1,18 | 1,24 | 1,00 | 1,98 | 56 | | | | | |
| 22 | 125,0 | 131,0 | 125,0 | 131,0 | MeCO2n -Bu | 60°C / 16 h | 84,6 | 92,4 | 95,1 | n.b. |
| | 1,00 | 1,05 | 1,00 | 1,05 | | | | | | |
| 23 | 125,0 | 131,0 | 125,0 | 131,0 | MeCO₂n -Bu | 60°C / 16 h | 93,4 | 95,4 | 98 | >99,0 : <1,0 |
| | 1,00 | 1,05 | 1,00 | 1,05 | | (Schiffsche Base erzeugt), 40°C→60°C / 20% Überschuß Mandelsäure | | | | |

Entsprechend den Reaktionsbedingungen, die in den Tabellen 4 und 5 zusammengefaßt sind, wurden in den zehn parallel betriebenen Reaktoren eines Surveyor-Laborautomaten der Firma Argonaut Vierkomponenten-Kupplungen mit (+)-DPWS, (S)-(+)-Mandelsäure oder (-)-Äpfelsäure in verschiedenen Lösungsmitteln untersucht. In diesen Reaktoren erfolgt die Durchmischung mit schnellen Schwingkolben ("piston activated magnetic agitation"). Die Durchmischung ist wesentlich effizienter als die von Magnetrührfischen oder von KPG-Paddelrührern und geringfügig effizienter als die von Turbinenrührern.

Tabelle 4 zeigt die Ergebnisse der Vierkomponentenkupplungen mit (+)-DPWS, (S)-(+)-Mandelsäure oder (-)-Äpfelsäure:

| Tabelle 4 | | | | | | | | | |
|---|---|---|---|---|---|---|---|---|---|
| Batch | Säure (VII) | Lösungsmittel | T [°C] | t [h] | Gehalt (IX) [%] | Gehalt (IX A) [%] | Molgewicht (I) [g/mol] | Verhältnis (I) / (VII) | Ausbeute in Gewicht [g], % derTheorie |
| 1 | 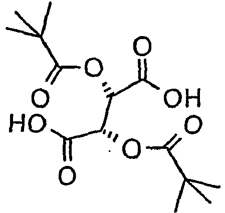 | EtOH | 35 | 3 | 65,21 | 34,79 | 1167,25 | 2 : 1 . | 2,35 |
| | | | | 6 | 67,19 | 32,81 | | | |
| | | | | 9 | 67,31 | 32,69 | | | 80,5% |
| | | | | 15 | 73,22 | 26,78 | | | |
| 10 | | EtOH | 50 | 3 | 87,90 | 12,1 | 1167,25 | 1,75: 1 (NMR) | 2,12 |
| | | | | 6 | 91,16 | 8,84 | | | 72,7% |
| | | | | 9 | 95,97 | 4,03 | | | |
| | | | | 15 | 98,36 | 1,64 | | | |
| 4 | | MeOH | 30 | 3 | 90,12 | 9,88 | 1167,25 | 1,82 : 1 (NMR) | 2,19 |
| | | | | 6 | 93,85 | 6,15 | | | 75,1% |
| | | | | 9 | 96,94 | 3,06 | | | |
| | | | | 15 | 98,50 | 1,5 | | | |
| 7 | | n-BuOH | 50 | 3 | 84,83 | 15,17 | 1167,25 | 1,91 : 1 (NMR) | 2,04 |
| | | | | 6 | 85,76 | 14,24 | | | 69,9% |
| | | | | 9 | 87,84 | 12,16 | | | |
| | | | | 15 | 91,40 | 8,6 | | | |
| 2 | | EtOH | 40 | 3 | 54,98 | 45,02 | 576,61 | 1 : 1 | 2,16 |
| | | | | 6 | 61,12 | 38,88 | | | |
| | | | | 9 | 67,16 | 32,84 | | | 74,9% |
| | | | | 15 | 71,94 | 28,06 | | | |

| Batch | Säure (VII) | Lösungsmittel | T [°C] | t [h] | Gehalt (IX) [%] | Gehalt (IX A) [%] | Molgewicht (I) [g/mol] | Verhältnis (I) / (VII) | Ausbeute in Gewicht [g], % der Theorie |
|---|---|---|---|---|---|---|---|---|---|
| 5 | 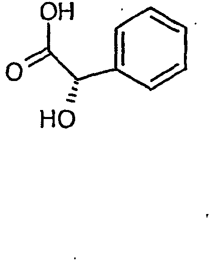 | MeOH | 30 | 3 | 67,37 | 32,63 | 576,61 | 1 : 1 (NMR) | 2,14 |
| | | | | 6 | 71,31 | 28,69 | | | 74,2% |
| | | | | 9 | 82,67 | 17,33 | | | |
| | | | | 15 | 97,72 | 2,28 | | | |
| 8 | | n-BuOH | 50 | 3 | 60,01 | 39,99 | 576,61 | 1 : 1 (NMR) | 2,27 |
| | | | | 6 | 66,15 | 33,85 | | | 78,7% |
| | | | | 9 | 79,32 | 20,68 | | | |
| | | | | 15 | 97,13 | 2,87 | | | |
| 3 | 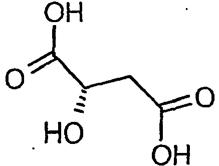 | EtOH | 50 | 3 | - | - | 938,01 | 2 : 1 | 1,21 |
| | | | | 6 | 49,05 | 50,95 | | | |
| | | | | 9 | 47,08 | 52,92 | | | 51,6% |
| | | | | 15 | 48,87 | 51,13 | | | |
| 6 | | MeOH | 50 | 3 | - | - | 938,01 | 2: 1 . | 0,58 |
| | | | | 6 | - | - | | | |
| | | | | 9 | - | - | | | 24,7% |
| | | | | 15 | 49,08 | 50,92 | | | |
| 9 | | n-BuOH | 50 | 3 | | - | 938,01 | 2 : 1 (NMR) | 1,67 |
| | | | | 6 | - | - | | | 71,2%. |
| | | | | 9 | - | - | | | |
| | | | | 15 | 91,52 | 8,48 | | | |

Tabelle 5 zeigt Vierkomponenten-Kupplungen mit (S)-(+)-Mandelsäure in verschiedenen Lösungsmitteln im Surveyor-Laborautomaten:

| Tabelle 5 | | | | | |
|---|---|---|---|---|---|
| mol. eq. Mandelsäure | Masse [g] | Ausbeute % d. Th. | Lösungsmittel | ee (HPLC) [%] | Verhältnis (I) / (VII) [¹H-NMR] |
| 2,00 | 2,51 | 86,0 | EtOH,MEK | 95,2 | 1 : 1 |
| 2,00 | 2,52 | 86,3 | EtOH, Toluol | 94,4 | 1 : 1 |
| 2,00 | 2,39 | 81,8 | EtOH, abs. | 94,8 | 1 : 1 |
| 2,00 | 2,52 | 86,3 | n-BuOH | 96 | 1 : 1 |
| 2,00 | 2,75 | 94,2 | i-PrOH | 94 | 1 : 1 |
| 2,00 | 2,24 | 76,7 | MeOH | 98,6 | 1 : 1 |
| 2,00 | 1,49 | 51,0 | MEK | 97,4 | 1 : 1 |
| 2,00 | 1,92 | 65,8 | Aceton | 97,8 | 1 : 1 |
| 2,00 | 2,55 | 87,3 | n-BuOAc | 96,0 | 1 : 1 |
| 2,00 | 2,40 | 82,2 | MeOH | 98,6 | 1 : 1 |
| 1,1 | 2,35 | 80,6 | MEOH | 95,0 | 1 : 1 |
| 1,2 | 2,44 | 83,5 | MeOH | 94,8 | 1 : 1 |
| 1,5 | 2,49 | 85,2 | MeOH | 95,2 | 1 : 1 |
| 2,0 | 2,57 | 88,0 | MeOH | 93,2 | 1 : 1 |
| 1,1 | 2,40 | 82,3 | EtOH,MEK | 91,2 | 1 : 1 |
| 1,2 | 2,48 | 85,0 | EtOH,MEK | 91,8 | 1 : 1 |
| 1,1 | 2,59 | 88,8 | i-PrOH | 92,0 | 1 : 1 |
| 1,2 | 2,69 | 92,2 | i-PrOH | 93,4 | 1 : 1 |
| 1,5 | 2,71 | 92,7 | i-PrOH | 93,0 | 1 : 1 |
| 2,0 | 2,40 | 82,2 | i-PrOH | 94,3 | 1 : 1 |
| 1,1 | 2,29 | 78,4 | n-BuOAc | 90,8 | n.b. |
| 1,2 | 2,53 | 86,6 | n-BuOAc | 94,0 | n.b. |
| 1,5 | 2,45 | 83,9 | n-BuOAc | 94,4 | n.b. |
| 2,0 | 2,57 | 88,0 | n-BuOAc | 96,0 | n.b. |
| 1,5 | 2,58 | 88,4 | EtOH,MEK | 93,6 | n.b. |
| 2,0 | 2,48 | 84,9 | ETOH,MEK | 93,0 | n.b. |
| 1,1 | 2,52 | 86,3 | n-BuOH | 92,4 | n.b. |
| 1,2 | 2,52 | 86,3 | n-BuOH | 95,4 | n.b. |
| 1,5 | 2,63 | 90,1 | n-BuOH | 96,4 | n.b. |
| 2,0 | 2,40 | 82,2 | n-BuOH | 95,0 | n.b. |
| 1,5 | 13,08 | 89,5 | MeOH | 91,2 | n.b. |
| 1,5 | 9,67 | 66,2 | Aceton | 94,6 | n.b. |

Die Isolierung des Produkts (III) erfolgte, wenn nicht anders in den Tabellen vermerkt, durch Abkühlung der Suspension auf Raumtemperatur, gefolgt von Filtration und Nachwaschen des Feststoffs mit wenig kaltem Solvent.

In Methanol bei 60°C verlief die kombinierte Vierkomponentenkupplung / dynamische Racematspaltung sehr schnell. Bereits nach einer Stunde hatte die dem Niederschlag (III) / (III A) zugrundeliegende freie Mannich-Base (I) einen Enantiomerenüberschuß von 92.6% ee (Tabelle 3, Zeile 2) erreicht und nach maximal 3 Stunden war die Reaktion bei 97.3% ee abgeschlossen (Tabelle 3, Zeile 1). Im Surveyor Laborautomaten wurden wegen der effizienteren Durchmischung bis zu 98.6% ee erzielt (Tabelle 5).

Aufgrund der nicht unerheblichen Löslichkeit von (III) bei Raumtemperatur in Methanol lagen die Ausbeuten um mindestens 10% unter denen in Ethanol. Selbst bei nur 30°C war die Reaktion in Methanol in 15 Stunden abgeschlossen (Tabelle 4). In Ethanol erforderte die Reaktion bei 40°C 44 - 53 Stunden (Tabelle 3, Zeilen 4 und 5). Ausbeuten (bis 95.3% der Theorie) und Enantiomeren-Überschuß (ca. 95% ee) waren hoch. Bei 60°C war die Reaktion in Ethanol bereits nach ca. 4 Stunden abgeschlossen, wenn zwei Äquivalente Mandelsäure eingesetzt wurden. Ausbeuten (bis 92.6 % der Theorie) und Enantiomeren-Überschuß (bis 97.5% ee) blieben hoch (Tabelle 3, Zeilen 6-8). Wurde die Reaktion bei sehr hoher Konzentration durchgeführt, so sank die Reaktionsgeschwindigkeit etwas ab, Ausbeute und ee sanken marginal (Tabelle 3, Zeile 9). Mit 1.5 Äquiv. Mandelsäure erforderte die Reaktion bei 60°C in Ethanol ca. 7 Stunden und führte zu nur geringfügig niedrigeren Ausbeuten und ee-Werten (Tabelle 3, Zeilen 10 und 11). Mit 1.10 Äquiv. Mandelsäure (Tabelle 3, Zeile 12 bis 14) bzw. mit nur 1.05 Äquiv. Mandelsäure (Tabelle 3, Zeile 15 bis 16) wurde wiederholt das Phänomen beobachtet, daß ein bei 60°C in Ethanol bereits erzielter ee sich beim Abkühlen der Suspension auf RT (vor dem Absaugen des Produkts) deutlich verschlechterte. Beim Stehen über Nacht kann diese ee-Abnahme 8% betragen (Zeile 16). Erfolgte das Abkühlen der Suspension und Absaugen von (III) aber rasch, ließen sich auch bei Einsatz von nur 1.05 Äquiv. Mandelsäure 88% Ausbeute und 95.4% ee erzielen (Zeile 15). Bei Reaktionen mit 2.0 Äquiv. Mandelsäure erfolgten derartige ee-Verschlechterungen beim Abkühlen nicht. Ein Aliquot der ausreagierten Reaktionssuspension (60°C, Ethanol) wurde entnommen und 72 Stunden bei Raumtemperatur gerührt. Der Enantiomeren-Überschuß und das syn / anti-Verhältnis waren danach unverändert.
Die Reaktion kann mit ähnlichem Erfolg in längerkettigen verzweigten oder unverzweigten Alkoholen, wie z.B. Isopropanol (Tabelle 3, Zeile 17, Tabelle 5) oder n-Butanol (Tabelle 3, Zeile 18 und 19; Tabelle 4 und 5) durchgeführt werden. Sie gelingt auch in ketonischen Lösungsmitteln, wie z.B. Aceton (Tabelle 3, Zeile 20 und 21; Tabelle 5) oder Methylethylketon (MEK, Tabelle 5), in Estern wie z.B. Ethylacetat oder n-Butylacetat (Tabelle 3, Zeile 22 und 23; Tabelle 5) und in halogenierten Kohlenwasserstoffen wie z.B. Dichlormethan.

In Ethern, wie z.B. Tetrahydrofuran, Methyl-tert.butylether, Diisopropylether, 1,2-Dimethoxyethan, Diethylenglycoldimethylether (Diglyme), in Kohlenwasserstoffen wie z.B. Toluol, sowie in superkritischen Medien wie z.B. superkritischem Kohlendioxid, ist die Reaktion prinzipiell durchführbar. Der Zusatz löslichkeitsverbessernder Additive, wie z.B. Phasentransfer-Katalysatoren oder Co-Solventien, kann vorteilhaft sein. In aprotisch-polaren Lösungsmitteln wie z.B. Dimethylformamid (DMF), Dimethylacetamid (DMA), Dimethylsulfoxid (DMSO) oder N-Methylpyrrolidinon (NMP) ist die Reaktion durchführbar. Die isolierten Ausbeuten sind in diesen Lösungsmitteln dann konkurrenzfähig, wenn die Löslichkeit von (III) in Ihnen nicht zu hoch ist.

Die Reaktion toleriert einen Feuchtigkeitsgehalt. Ein Vergleich von Tabelle 3, Zeile 4 mit Zeile 5 und von Tabelle 5, Zeilen 1-3 zeigt, daß absolutes Ethanol keine Vorteile gegenüber technischem, MEK- oder Toluol-vergälltem Ethanol bietet. An einigen Beispielen wurde beobachtet, daß bei Verwendung von Lösungsmitteln, die niedrig siedende Azeotrope mit Wasser bilden (wie z.B. Ethanol), das kontinuierliche azeotrope Abdestillieren des bei der Mannich-Reaktion entstehenden Reaktionswassers bei Normaldruck oder im Vakuum zu signifikanten bis mäßigen Reaktionsbeschleunigungen führte. Dies kann zu Optimierungen der Raum-ZeitAusbeute und, wegen der kürzeren thermischen Belastung, gelegentlich zur Verbesserung der chemischen Reinheit und isolierten Ausbeute des Produkts genutzt werden.

Ähnliche Ergebnisse lassen sich auch durch Zusatz wasserbindender Additive, wie z.B. getrocknetem Magnesiumsulfat oder aktiviertem Molekularsieb, erzielen. Der Ausschluß von Wasser und / oder die Entfernung des entstehenden Reaktionswassers sind aber weder für den praktisch quantitativen Verlauf der Vierkomponenten-Mannich.Kupplung, noch für den Ablauf der dynamischen Racematspaltung essentiell. Tabellen 1 - 5 belegen, daß man bei Inkaufnahme der notwendigen Reaktionszeiten die Produkte (III) auch dann in sehr hoher Ausbeute, chemischer Reinheit und mit hohem Enantiomerenüberschuß isolieren kann, wenn man ungetrocknete Apparaturen und ungetrocknete Lösungsmittel verwendet und das entstehende Reaktionswasser nicht entfernt.

Entsprechend den Tabellen 2 - 5 sind die relativen molaren Mengen der vier Edukte (IV) - (VI) innerhalb beträchtlicher Intervalle variabel, ohne daß es zu negativen Effekten auf Ausbeute, chemische Reinheit oder Enantiomerenüberschuß des Produkts (III) kommt. Unter Bezug auf jeweils 1.00 Äquivalente der CH-aciden Komponente (VI) wurden die Einsatzmengen der übrigen Edukte in den konkreten Beispielen (Tabellen 1 - 5) innerhalb der folgenden Intervalle variiert: Aldehyd (IV): 1.00 - 1.20 Äqiuivalente; Amin (V): 1.05 - 1.25 Äquivalente; chirale Säure (VII): 1.05 - 2.00 Äquivalente.

Der wesentlichste Faktor für die Effizienz der dynamischen Racematspaltung im Verfahrensschritt 1 ist eine gute Auswahl der chiralen Säure HY* der Formel (VII). Auf allen Gebieten der Stereochemie herrscht mittlerweile Konsens, daß es ein optimales chirales Auxiliar per se oder einen optimalen chiralen Liganden per se nicht gibt und nicht geben kann. Das Ausmaß der Asymmetrie von Reaktionen hängt vielmehr von spezifischen Edukt / Auxiliar - bzw. Produkt / Auxiliar - Wechselwirkungen ("chirale Erkennung") ab. Welche chirale Säure (VII) innerhalb des erfindungsgemäßen Verfahrens ein optimales Resultat liefert, hängt somit von der spezifischen Natur der Substituenten R¹ bis R⁵ ab und muß für jede Kombination der Edukte (IV) bis (VI) jeweils unabhängig, in der Regel experimentell, ermittelt werden. Dies kann auf folgendem Wege erreicht werden:
a) Man stellt die racemische freie Mannich-Base rac.-(I) her. Dies kann besonders einfach auf einem der beiden folgenden alternativen Wege geschehen:
   a1) Man führt die Vierkomponenten-Mannich-Kupplung analog Verfahrensschritt 1 durch, wobei man jedoch die Edukte (IV), (V) und (VI) mit nur katalytischen Mengen einer achiralen Säure in einem Lösungsmittel einsetzt, in dem die Mannich-Base rac.-(I) nur mäßige Löslichkeit besitzt. In vielen Fällen hat sich der Einsatz von ca. 1 Mol-% p-Toluolsulfonsäure Hydrat im Lösungsmittel Ethanol bewährt. Die freie Mannich-Base rac.-(I) kristallisiert dann aus dem Reaktionsgemisch in teilweise sehr hoher Ausbeute aus und kann durch Filtration isoliert werden. Beispiel 3, beschreibt eine entsprechende Prozedur.
   a2) Man führt die Vierkomponenten-Mannich-Kupplung analog Verfahrensschritt 1 durch, wobei man jedoch die Edukte (IV), (V) und (VI) mit stöchiometrischen oder überstöchiometrischen Mengen einer achiralen Säure in einem der oben genannten, für Verfahrensschritt 1 geeigneten Lösungsmittel umsetzt. In diesem Fall erhält man ein Salz analog Formel (III), in dem das Kation racemisch ist und das Anion Y⁻ achiral ist. Dieses Salz rac.-(III) setzt man dann analog Verfahrensschritt 2 zur freien racemischen Mannich-Base rac.-(I) um.
b) Man ermittelt ein Lösungsmittel, in dem rac.-(l) mittel- bis mäßiglöslich ist (bevorzugte Löslichkeit ca. 1 - 5 Gew.-%) und in dem seine Retro-Mannich-Reaktion so langsam wie möglich verläuft. Zur Selektion dieses Lösungsmittels stehen verschiedene alternative physikalische oder chemische Methoden zur Verfügung:
   b1) Man löst rac.-(I) in entsprechenden perdeuterierten Lösungsmitteln und führt ein Monitoring der jeweiligen Retro-Mannich-Geschwindigkeiten durch, indem man die Lösungen in kurzen Zeitabständen wiederholt per ¹H- oder ¹³C-NMR vermißt;
   b2) Man löst rac.-(I) in Lösungsmitteln und erhält ein Echtzeit-Monitoring der Retro-Mannich-Reaktion mit Hilfe einer React-IR-Sonde, oder indem man die Lösung in einer Küvette in einem konventionellen Zweistrahl-IR-Gerät in regelmäßigen Zeitabständen vermißt, wobei sich im Referenzstrahl jeweils eine gleiche Küvette, gefüllt mit dem reinen Lösungsmittel, befindet.
   b3) Man löst bzw. suspendiert rac.-(I) in aprotischen Lösungsmitteln, die kompatibel mit einer Amidierungsreaktion mittels Säurechloriden sind. Man setzt die erhaltenen Lösungen oder Suspensionen unmittelbar nach ihrer Herstellung mit Pivaloylchlorid (VIII B) zum racemischen Pivaloylderivat (IX) / (IX A) um. Die erzielte Ausbeute und Reinheit des Amids (IX) / (IX A) ist umso höher, je langsamer die Retro-Mannich-Reaktion im jeweiligen Lösungsmittel erfolgte. Beispiel 4 beschreibt eine entsprechende Prozedur.

In den bisher untersuchten Beispielen wurde festgestellt, daß die Retro-Mannich-Tendenz der Salze strukturanaloger Mannich-Basen mit Brönstedt-Säuren (Formel III) unter identischen Bedingungen (gleiches Lösungsmittel, gleiche Temperatur, gleiche Brönstedt-Säure) durch elektronendrückende Substituenten in der Aldehyd-Komponente der Formel (IV) gefördert wurde. Elektronenziehende Substituenten in der Aldehyd-Komponente der Formel (IV) senkten die Retro-Mannich-Tendenz. Das 1H-NMR-Monitoring der syn/anti-Isomerisierung eines syn-Mannich-Salzes der Formel (III) via Retro-Mannich-Reaktion bei 300 K in DMSO-d6-Lösung kann Beispiel 28 entnommen werden. Wie man Beispiel 27 entnimmt, lassen sich bei guter Wahl der Reaktionsparameter in der Vierkomponenten-Kupplung Mannich-Salze in ausgezeichneter Ausbeute selbst dann mit sehr hoher Diastereomeren- und Enantiomerenreinheit der zügrundeliegenden Mannich-Base erhalten, wenn die Aldehyd-Komponente elektronendrückende Substituenten enthält und die Retro-Mannich-Tendenz hoch ist.

In den oben beschriebenen Beispielen wurde festgestellt, daß die Retro-Mannich-Reaktion von freien Mannich-Basen rac.-(I) häufig in Aceton sehr langsam verläuft.
c) Man führt mit der Lösung oder Suspension von rac.-(I) in dem gemäß b) ermittelten Lösungsmittel, z.B. Aceton, ein Screening aller verfügbaren optisch aktiven Brönstedtsäuren HY^{*} (VII) bezüglich Effizienz einer klassischen Racematspaltung durch. Dazu setzt man die frisch bereitete Suspension von rac.-(I), wenn die Substituenten R¹ bis R⁵ keine basischen Zentren enthalten, mit 1.0 Mol-Äquivalenten der Säure (VII) um, wenn (VII) eine einbasige Säure ist, bzw. mit 0.5 Mol-Äquivalenten der Säure (VII), wenn VII eine zweibasige Säure ist. Enthalten die Substituenten R¹ bis R⁵ basische Zentren, so setzt man entsprechend mehr Mol-Äquivalente der Säure (VII) ein. Man rührt ca. 20 Std. bei Raumtemperatur, isoliert das ausgefallene Salz (III) durch Filtration und bestimmt das in der zugrundeliegenden freien Base (I) vorliegende Enantiomerenverhältnis durch Derivatisierung zu (IX) / (IX A), gefolgt von HPLC-Analyse *(vide supra*). Man wählt diejenigen chiralen Brönstedtsäuren (VII) aus, die bei diesem Screening die höchsten (IX) : (IX A) - Verhältnisse liefern, vorzugsweise (IX) : (IX A) ≥ 95 : ≤ 5. Beispiel 6 beschreibt eine repräsentative Prozedur für einen Versuch im Rahmen eines derartigen Screenings.
d) Unter den gemäß c) selektierten optisch aktiven Brönstedtsäuren (VII) kann weiter selektiert werden, um möglichst weitgehend die folgenden Kriterien für besonders bevorzugte Säuren (VII) zu erfüllen:
   - Y^{*-} ist konfigurationsstabil unter den Reaktionsbedingungen;
   - sie führt zu einem maximalen Lösüchkeitsunterschied zwischen ihren beiden diastereomeren Salzen (III) und (III A)
   - sie bewirkt eine möglichst niedrige Löslichkeit des gewünschten Diastereomeren der Formel (III) und eine möglichst hohe Löslichkeit des ungewünschten Diastereomeren der Formel (III A)
   - das Racemat des Salzes der Formel (III) (1:1-Gemisch von Salz (III) und dessen Spiegelbild) kristallisiert als KonglomeratEin Konglomerat besteht aus einem Gemisch zweier spiegelbildlicher Kristallstrukturen, von denen eine Kristallstruktur der Kristallstruktur des optisch aktiven Salzes (III) entspricht. Im Konglomerat sind nicht nur die enantiomeren Moleküle, sondern auch die beiden Kristallstrukturen als supramolekulare Gebilde zueinander spiegelbildlich. Die beiden Kristallstrukturen im Konglomerat unterscheiden sich nicht nur in der Chiralität der Moleküle. Auch die Kristallpackung, d.h. die dreidimensinal periodische Anordnung / Stapelung der Moleküle ist in den beiden Kristallstrukturen spiegelbildlich.
   - sie katalysiert die Vierkomponenten-Mannich-Reaktion, die zur Bildung von (III) und (III A) führt,
   - sie katalysiert die Refiro-Mannich-Reaktion des besser löslichen diastereomeren Salzes (III A), d.h. die Rückspaltung des Salzes (III A) in das enolierbare Keton (VI) und das Iminium-Salz R¹CH=N⁺R²R³ Y^{*-} bzw. dessen Spaltprodukte, den Aldehyd (IV) und das Salz des Amins (V) mit HY^{*}.

Falls die freie Mannichbase der Formel (I) als Konglomerat kristallisiert, dann gehört zum Umfang der vorliegenden Erfindung auch eine spezielle Durchführungsform, bei der die Dreikomponentenkupplung und die dynamische Racematspaltung in Abwesenheit einer chiralen Hilfssäure HY* durchgeführt werden können. Dabei wird die Lösung der drei Komponenten (IV), (V) und (VI), ggf. in Gegenwart katalytischer Mengen (ca. 1-10 Mol%) einer achiralen Säure, z.B. p-Toluolsulfonsäure, mit Kristallen der optisch reinen freien Mannichbase angeimpft. Aufgrund des Konglomerat-Effekts ("Preferential Crystallization"), kann dann nur dieser Antipode der freien Mannichbase aus der Reaktionslösung auskristallisieren und wird aus dem in Lösung bleibenden Spiegelbild ständig nachgebildet. Ist diese Nachbildung rasch gegenüber der Kristallisationsgeschwindigkeit des gewünschten Antipoden, wird die Grenzkonzentration des falschen Antipoden, bei dem auch dessen Kristallisation einsetzen würde, im Reaktionsverlauf nie erreicht. Darum besteht am Ende der Reaktion der Niederschlag ausschließlich aus dem gewünschten Antipoden und die chemische Ausbeute kann nahe an 100% herankommen. Diese Asymmetrische Transformation der 2. Art ohne Notwendigkeit eines chiralen Auxiliars ist unter dem Begriff "total spontaneous resolution" bekannt (E.H. Eliel, S.H. Wilen "Stereochemistry of Organic Compounds", John Wiley, New York, 1994, Seite 316 ; Y. Okada et al, J. Chem. Soc., Chem. Commun. 1983, 784 - 785).

In einer weiteren Variante des erfindungsgemäßen Verfahrensschritts 1 wird aus den Edukten (IV) und (V) zunächst das Imin (X) vorgeformt und erst dann das CH-acide Keton (VI) zugesetzt, was in Gegenwart einer geeigneten optisch aktiven Säure (VII) zur Bildung des Mannich-Salzes (III) unter dynamischer Racematspaltung führt. Natürlich ist es möglich, aus dem Aldehyd (IV) und dem Amin (V), in bekannter Weise, katalysiert durch eine Säure, die achiral sein kann, z.B. ca. 1 Mol-% p-Toluolsulfonsäurehydrat, das Imin (X) zu bilden und es zu isolieren. Eine derartige Prozedur ist im Beispiel 9 beschrieben. Das Imin (X) kann dann anschließend mit dem Keton (VI) und der optisch aktiven Säure (VII) zum Mannich-Salz (III) umgesetzt werden.

Einige der Nachteile der indirekten Mannich-Reaktion werden vermieden, wenn man eine Lösung des Imins (X) vorbildet, indem man den Aldehyd (IV) und eine mindestens äquimolare Menge des Amins (V) in einem der oben genannten geeigneten Lösungsmittel, besonders bevorzugt n-Butylacetat, erwärmt und das dabei entstehende Reaktionswasser, bevorzugt im Vakuum, azeotrop abdestilliert. Besonders bevorzugt wird dieser Reaktionsschritt in einer Apparatur / einem Reaktor durchgeführt, die die Funktion eines Wasserabscheiders hat, d.h. nach Kondensation des azeotropen Dampfes und daraufhin erfolgende Phasentrennung fließt das spezifisch leichtere organische Lösungsmittel automatisch in den Reaktor zurück, während das Wasser im Abscheider zurückgehalten wird. Nachdem sich die theoretische Menge Wasser abgeschieden hat, werden der Reaktionslösung 0.80 - 2.00 Äquivalente des CH-aciden Ketons (VI) und 0.80 - 4.00 Äquivalente der chiralen Säure (VII) (jeweils bezogen auf den Aldehyd (IV)), bevorzugt 0.95 - 1.30 Äquivalente (VI) und 1.00 - 2.00 Äquivalente (VII), besonders bevorzugt 1.00 -1.25 Äquivalente (VI) und 1.05 - 1.25 Äquivalente (VII), zugesetzt und ggf. weiter erwärmt, bis die Enantiomerenreinheit in dem nach kurzer Zeit auftretenden Niederschlag (III) / (III A) aufgrund der ablaufenden dynamischen Racematspaltung ihren Maximalwert erreicht hat.

Wie man Tabelle 3 (Nr. 22, 23) entnimmt, wurde für R¹ = o-Nitrophenyl, R² = 2-Pyridyl, R³ = H, R⁴ = 2-Pyridyl, R⁵ = Phenyl, HY^{*} = (S)-(+)-Mandelsäure im Solvent n-Butylacetat bei 60°C mit der normalen Vierkomponentenkupplung das Mannich-Salz III in einer isolierten Ausbeute von 84.6% der Theorie und mit 95.1 % ee erhalten. Wurde die n-Butylacetatlösung des Imins (X) hingegen in der beschriebenen Weise vorgebildet, dann wurde das Mannich-Salz (III) in einer Ausbeute von 93.1 % der Theorie mit 96.7 % ee isoliert, wenn nach Zusatz von (VI) und (VII) unmittelbar auf 60°C geheizt wurde. Eine besonders hohe Ausbeute von 93.4% der Theorie und 98 % ee wurde erzielt, wenn nach dem Zusatz von (VI) und (VII) zunächst nur auf 40°C geheizt wurde (beginnende Niederschlagsbildung) und erst nach 4 Std. die Temperatur auf 60°C erhöht wurde. Bei der normalen Vierkomponentenkupplung erfolgen Bildung und Abreaktion des Imins (X) hingegen parallel. Eine Untersuchung im Reaktionskalorimeter RC1 (Firma Mettler) unter Echtzeit-Monitoring des Reaktionsverlaufs per React-IR-Sonde zeigte beim vorliegenden Beispiel, daß es in keiner Phase der Vierkomponentenkupplung zu einer Akkumulation von mehr als 40% der theoretischen Menge des Imins (X) im Reaktionsgemisch kommt. Darüberhinaus ist die Zeitdauer der thermischen Belastung signifikanter Mengen des Imins (X) dort wesentlich kürzer.

In einer weiteren Durchführungsvariante des erfindungsgemäßen Verfahrensschritts 1 kann auch das Aminal der Formel (XI) vorgebildet werden (Beispiel 10) und dann, entweder nach zwischenzeitlicher Isolierung oder in der ursprünglichen Reaktionslösung, mit dem Keton (VI) und der Säure (VII), gegebenenfalls unter Zusatz eines zusätzlichen Äquivalents des Aldehyds (IV), zum Mannich-Salz (III) umgesetzt werden. Auch in dieser Durchführungsvariante isoliert man (III) mit optischen Ausbeuten, die denen der Vierkomponenten-Kupplung (Tabellen 2 bis 5) nahe kommen.

Bei allen hier genannten Durchführungsvarianten des erfindungsgemäßen Verfahrensschrittes 1 beruht die hohe optische Aktivität des Mannich-Salzes (III) auf dem Ablauf einer dynamischen Racematspaltung. Der erfindungsgemäße Verfahrensschritt unterscheidet sich somit grundlegend von der von B. List beschriebenen Vierkomponenten-Mannich-Reaktion (J. Am. Chem. Soc. 2000, 122, 9336-9337). Bei letzterer handelt es sich um eine katalytische asymmetrische Mannich-Reaktion, d.h. der Additionsschritt eines Enamins, entstanden aus der Kondensationsreaktion des CH-aciden Ketons (VI) mit dem Katalysator (L)-Prolin, an das Imin (X), das aus der Kondensationsreaktion des Aldehyds (IV) mit dem Amin (V) entstanden ist, unter direkter Bildung der freien Mannich-Base (I) erfolgt asymmetrisch. Bei der List-Reaktion werden darum nur ca. 35 Mol-% (L)-Prolin eingesetzt. Bereits das in Lösung befindliche Reaktionsprodukt ist optisch aktiv und nach gegenwärtigem Kenntnisstand verändert sich die optische Reinheit des Produkts während des Reaktionsverlaufs nicht grundlegend. Im Gegensatz dazu wird der erfindungsgemäße Verfahrensschritt 1 nicht mit "katalytischen" Mengen der chiralen Säuren (VII) durchgeführt: setzt man weniger als 0.8 Mol-Äquivalente einer einbasigen Säure (VII) oder weniger als 0.4 Mol-Äquivalente einer zweibasigen Säure (VII) ein, fallen die isolierten Ausbeuten an Mannich-Salz (III) zwangsläufig auf unter 70% der Theorie und sind dann industriell nicht mehr akzeptabel. Da die Addition des Ketons (VI) an das *in situ* im Reaktionsgemisch gebildete Imin (X) von den chiralen Säuren (VII) nicht signifikant asymmetrisch induziert wird, liegt in Lösung das Verhältnis der Mannich-Salze (III) : (III A) bei etwa 1:1. Weiterhin steigt die optische Reinheit im auskristallisierten Mannich-Salz (III) während des gesamten Reaktionsverlaufs kontinuierlich an.

Die chiralen Säuren (VII) des erfindungsgemäßen Verfahrensschrittes 1 können auf einfache Weise nahezu quantitativ und mit unveränderter optischer Reinheit zurückgewonnen und im nächsten Batch erneut eingesetzt werden. Durch vielfachen Wiedereinsatz des chiralen Auxiliars (VII) beim wiederholten batchweisen Durchlaufen des Verfahrensschritts 1 kann man brutto die Mannich-Salze (III) mit wesentlich weniger als 0.35 Mol-% (VII) herstellen. Weiterhin geht aus B. List (J. Am. Chem. Soc. 2000, 122, 9336-9337) hervor, daß die Reaktion nur mit Prolin gelingt und bereits bei sehr engen Analoga des Prolins versagt.

Aufgrund seines andersartigen Mechanismus gelingt der erfindungsgemäße Verfahrensschritt 1 hingegen mit einer breiten Palette strukturell zum Teil sehr divergenter Säuren (VII). Aus Tabellen 2 bis 5 ergibt sich beispielsweise, daß die gleiche Mannich-Base in hoher optischer Reinheit unter Verwendung von (S)-(+)-Mandelsäure, (+)-Dipivaloylweinsäure oder (L)-(-)-Äpfelsäure hergestellt werden konnte. Von industriellem Interesse ist dabei auch, daß (S)-(+)-Mandelsäure und (L)-(-)-Äpfelsäure einen mit (L)-Prolin vergleichbaren Preis haben, aber die enantiomeren Verbindungen (R)-(-)-Mandelsäure und (D)-(+)-Äpfelsäure wesentlich billiger als (D)-Prolin sind. Große Vorteile unseres Verfahrensschritts 1 gegenüber der List-Reaktion sind die sehr breite Palette anwendbarer Lösungsmittel, die Isolierung des optisch aktiven Mannich-Salzes (III) ohne Aufarbeitung (durch simple Filtration), sowie die hohen isolierten chemischen Ausbeuten (85-95% der Theorie). Diese Eigenschaften werden sämtlich durch die Beispiele in den Tabellen 2 bis 5 belegt.

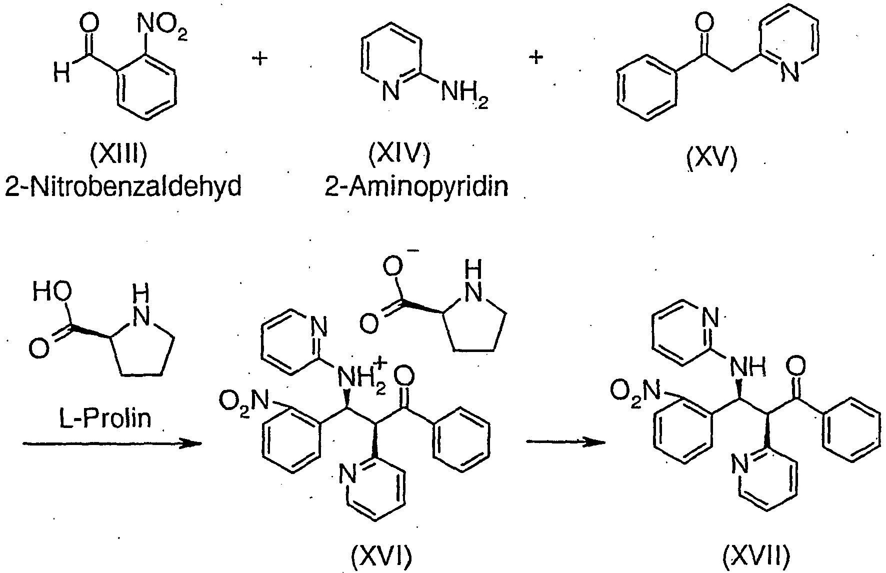

In einem Versuch einer asymmetrischen Mannich-Reaktion zu einer Verbindung der Formel (III), in der R¹ = o-Nitrophenyl, R² = 2-Pyridyl, R³ = H, R⁴ = 2-Pyridyl und R⁵ = Phenyl sind, und wobei L-Prolin als Überträger der chiralen Information benutzt wurde, wurden in die 8 Glasreaktoren eines Surveyor Laborreaktors jeweils 493 mg (1.00 Äquiv.) des Ketons der Formel (XV), 294 mg (1.25 Äquiv.) 2-Aminopyridin (XIV) und 453 mg (1.20 Äquiv.) 2-Nitrobenzaldehyd (XIII) eingewogen. In die Reaktoren 1-5 und 7 wurden außerdem je 101 mg (0.35 Äquiv.) L-Prolin, in die Reaktoren 6 und 8 je 576 mg (2.00 Äquiv.) L-Prolin eingewogen. Dann wurden jeweils 10 ml der in der Tab. angegeben Lösungsmittel zugefügt. Die Reaktoren 1-6 wurden bei Raumtemp. (22°C) gerührt, die Reaktoren 7-8 bei 40°C IT. Nach den angegebenen Reaktionszeiten wurden entnommene Proben mit Camphanoylchlorid (VIII A) derivatisiert und die entstandenen isomeren Amide (XVII), (XVII A), anti-Isomer von (XVII), anti-Isomer von (XVII A) per HPLC quantifiziert.

Tabelle 6 zeigt die Ergebnisse einer versuchten asymmetrischen Mannich-Reaktion mit L-Prolin.

**Tabelle 6**

| Verhältnis [%, HPLC] | | | | t [h] | T [°C] | Nr. | Lösungsmittel | mol-Äquiv. L-Prolin |
|---|---|---|---|---|---|---|---|---|
| (XVII) | ent -(XVII) | trans- (XVII) | ent-trans- (XVII) | | | | | |
| - | - | - | - | 19 | 22 | 1 | Aceton | 0,35 |
| - | - | - | - | 40,5 | | | | |
| - | - | - | - | 53 | | | | |
| - | - | - | - | 131 | | | | |
| 9,7 | 47,8 | 2 | 1,2 | 19 | 22 | 2 | Methanol | 0,35 |
| 51,1 | 47,6 | 0,6 | 0,7 | 40,5 | | | | |
| 51,9 | 47,4 | 0,3 | 0,5 | 53 | | | | |
| 56,0 | 43,7 | 0,1 | 0,2 | 155 | | | | |
| - | - | - | - | 19 | 22 | 3 | DMSO | 0,35 |
| - | - | - | - | 40,5 | | | | |
| - | - | - | - | 53 | | | | |
| - | - | - | - | 131 | | | | |
| - | - | - | - | 19 | 22 | 4 | Dichlormethan | 0,35 |
| 19,1 | 19,1 | 24,3 | 37,6 | 40,5 | | | | |
| 23,3 | 21,6 | 24,1 | 31,0 | 53 | | | | |
| 24,9 | 22,6 | 23,1 | 29,4 | 131 | | | | |
| 50,5 | 49,5 | - | - | 19 | 22 | 5 | Ethanol | 0,35 |
| 48,9 | 47,1 | 1,7 | 2,3 | 40,5 | | | | |
| 49,5 | 47,9 | 1,1 | 1,6 | 53 | | | | |
| 55,3 | 43,8 | 0,4 | 0,5 | 155 | | | | |
| 48,9 | 45,9 | 2,4 | 3,4 | 19 | 22 | 6 | Ethanol | 2,00 |
| 48,4 | 47,4 | 1,6 | 2,5 | 40,5 | | | | |
| 49,5 | 46,7 | 1,6 | 2,2 | 53 | | | | |
| 54,1 | 45,4 | 0,3 | 0,1 | 155 | | | | |
| 50,4 | 48,0 | 0,7 | 0,8 | 18 | 40 | 7 | Ethanol | 0,35 |
| 54,9 | 45,1 | - | - | 131 | | | | |
| | | | | | | | | |
| | | | | | | | | |
| 49,8 | 48,7 | 0,6 | 0,8 | 18 | 40 | 8 | Ethanol | 2,00 |
| 52,6 | 44,7 | 2,4 | 0,3 | 131 | | | | |
| | | | | | | | | |
| | | | | | | | | |

Unter den in J. Am. Chem. Soc. 2000, 122, 9336-9337 explizit beschriebenen Bedingungen und unter naheliegenden Varianten dieser Bedingungen werden keine präparativ brauchbaren Ergebnisse erzielt. Unter den bevorzugten Bedingungen (35 mol% (L)-Prolin im Lösungsmittel Aceton bzw. DMSO bei Raumtemperatur) war nach Reaktionszeiten von 19 Stunden bis 131 Stunden die Mannich-Base bzw. dessen Enantiomer nicht in signifikanten Mengen gebildet worden (Tabelle 6, Nr. 1 bzw. 3). In den von List nicht genannten Lösungsmitteln Methanol bzw. Ethanol führt der Einsatz von 35 mol% (L)-Prolin bei Raumtemperatur zur Bildung der nahezu racemischen Mannich-Base innerhalb von 19 Stunden (Tabelle 6, Nr. 2 bzw. 5). Erst beim fortgesetzten Rühren des Reaktionsgemischs über 155 Stunden erlangte die gebildete Mannich-Base einen geringen, aber signifikanten Enantiomerenüberschuß (ca. 12% ee) unter gleichzeitigem Verschwinden der ursprünglich enthaltenen geringen Mengen an trans-Isomer (Tabelle 6, Nr.2 bzw. 5). Eine Erhöhung der Reaktionstemperatur (Ethanol, 40°C) steigert den nach 131 Stunden erzielten Enantiomerenüberschuß der Mannich-Base nicht (Tabelle 6, Nr. 7). Selbst mit 200 mol% (L)-Prolin wird in Ethanol sowohl bei Raumtemperatur als auch bei 40°C nur eine geringe optische Reinheit der erhaltenen Mannich-Base erzielt (8-9% ee, Tabelle 6, Nr. 6 bzw. 8). Mit 35 mol% (L)-Prolin im Lösungsmittel Dichlormethan bei Raumtemperatur liegt bis zu einer Reaktionszeit von 40 Stunden ungefähr doppelt soviel trans-Isomer als das gewünschte cis-Isomer der Mannich-Base vor. Erst nach 131 Std. haben sich die Mengen an trans- und cis-Isomer angeglichen. Signifikante Enantiomerenüberschüsse erreichen beide Diastereomere während des gesamten Zeitraums nicht (Tabelle 6, Nr. 4).

Weiterhin wurden Bedingungen gefunden, unter denen ein β-Aminoketon der Formel (I) aus der Verbindung der Formel (III) ohne signifikanten Verlust der stereochemischen Reinheit freigesetzt werden kann.

Gegenstand der vorliegenden Erfindung ist daher ferner ein Verfahren zur Herstellung eines optisch aktiven β-Aminoketons (Mannich-Base) der Formel (I) oder dessen Spiegelbild, 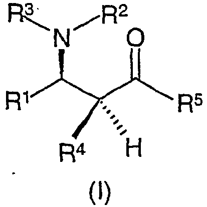 dadurch gekennzeichnet, daß
in Verfahrensschritt 1 die Verbindung der Formel (III) wie oben beschrieben hergestellt wird und in Verfahrensschritt 2 die Verbindung (III) unter Zusatz einer geeigneten Base in einem geeigneten Lösungsmittel umgesetzt wird, wobei die Reste R¹, R², R³, R⁴ und R⁵ und Y*⁻ wie oben definiert sind.

Geeignete Basen sind organische Amine, vorzugsweise (C₁-C₁₀)Trialkylamine, vorzugsweise (C₁-C₃)Trialkylamine, beispielsweise Triethylamin, Diisopropylethylamin, ferner Alkali- oder Erdalkali-hydrogencarbonate, -carbonate oder -hydroxide.

Geeignete Lösungsmittel sind Wasser oder organische Lösungsmittel, oder ein Gemisch von Wasser mit einem organischem Lösungsmittel, optional ein löslichkeitsverbesserndes Additiv, z.B. einen Phasentransfer-Katalysator enthaltend, wobei organische Lösungsmittel in 100%iger Reinheit oder in technischer Qualität vorliegen können und beispielsweise ein C₁-C₈-Alkohol, verzweigt oder unverzweigt, wie z.B. Methanol, Ethanol, n-Propanol, Isopropanol oder n-Butanol oder ein ketonisches Lösungsmittel, wie z.B. Aceton oder Methylethylketon (MEK), oder ein Ester, wie z.B. Ethylacetat oder n-Butylacetat, oder ein Ether, wie z.B. Tetrahydrofuran, Methyl-tert.-butylether, Diisopropylether, 1,2-Dimethoxyethan, Diethylenglycoldimethylether (Diglyme), oder ein Kohlenwasserstoff, aliphatisch oder aromatisch, wie z.B. Toluol, oder ein superkritisches Medium, wie z.B. superkritisches Kohlendioxid oder ein halogenierter Kohlenwasserstoff, wie z.B. Dichlormethan, oder ein aprotisch-polares Lösungsmittel wie z.B. DMF, DMSO oder NMP sein kann,

Die Freisetzung von (I) aus (III) kann im Temperaturbereich vom Schmelzpunkt bis zum Siedepunkt des Lösungsmittels (bzw. Lösungsmittelgemischs) durchgeführt werden, beispielsweise von -30 bis 100 °C, vorzugsweise bei 0 - 40°C, besonders bevorzugt bei 0 bis 25°C.

Die Freisetzung der Mannich-Base (I) aus dem optisch aktiven Mannich-Salz (III) unter vollständigem Konfigurationserhalt ist ein nicht-trivialer Verfahrensschritt, da er unter Bedingungen durchgeführt werden muß, unter denen es
1. zu keiner Deprotonierung der C-H aciden α-Position zur Ketofunktion in (III) oder (I) kommt, da dies zur Bildung des unerwünschten anti-Diastereomers von (III) bzw. (I) führen würde, und
2. zu keiner Retro-Mannich-Spaltung von (III) oder (I) kommt, da dies zu Ausbeuteverlust, Bildung chemischer Verunreinigungen, Bildung des unerwünschten anti-Diastereomers sowie partiellem Verlust der optischen Reinheit der Mannich-Base (I) führen würde.

Die Freisetzung kann im Prinzip in solchen organischen Lösungsmitteln, vorzugsweise in Aceton, durchgeführt werden, in denen die Retro-Mannich-Spaltung sehr langsam verläuft *(vide supra*), unter Einsatz von Basen, vorzugsweise Triethylamin, Diisopropylethylamin, Alkali- oder Erdalkali-hydrogencarbonate oder -carbonate), die zwar die N-H acide Ammoniumgruppe, nicht aber die C-H acide α-Position von (III) bzw. (I) deprotonieren können.

Die Freisetzung kann ferner im wäßrigem Milieu durchgeführt werden, wobei als Basen beispielsweise Alkali- oder Erdalkalihydrogencarbonate, -carbonate oder - hydroxide benutzt werden, vorzugsweise unter pH-Stat-Bedingungen bei pH ca. 8-9. Bevorzugt werden Natriumhydrogencarbonat oder Natriumhydroxid unter pH-Stat-Bedingungen bei pH ca. 8-9, besonders bevorzugt wird Natriumhydroxid.

Da die Löslichkeit sowohl der Mannich-Salze (III) als auch der freien Mannich-Basen (I) in schwach basischem Wasser meistens sehr gering ist, führt die Freisetzungsreaktion zur Umwandlung einer Suspension des Salzes (III) in eine Suspension freier Mannich-Base (I). Nach Reaktionsende kann das Produkt (I) darum durch simples Abschleudern oder Filtrieren isoliert werden. Aufgrund der geringen Löslichkeit befindet sich stets nur ein sehr kleiner Teil des vorhandenen Edukts (III) in Lösung und auch dieser nur für kurze Zeit, da die gebildete freie Base (I) sofort wieder ausfällt. Aus diesem Grunde spielt die Retro-Mannich-Reaktion in wäßrigem Milieu praktisch keine Rolle. Die isolierte Ausbeute an freier Base (I) betrug in den untersuchten Fällen 95-100% der Theorie, der Gehalt an anti-Diastereomer war unter optimierten Bedingungen mit ca. 0.7 - 1.5% innerhalb der Meßgenauigkeit unverändert gegenüber dem des eingesetzten Mannich-Salzes (III), der Enantiomerenüberschuß von (I) nahm bei optimierter Durchführung um kleiner gleich 2%, vorzugsweise 1 % ee gegenüber dem Salz (III) ab (Tabelle 7).

Bei Salzen der Formel (III), die in reinem Wasser zu wenig löslich sind, um von Basen wie NaOH oder NaHCO₃ oder Na₂CO₃ mit brauchbarer Geschwindigkeit zu (I) deprotoniert zu werden, können in Mengen von <25 Vol%, bevorzugt 1-10 Vol%, besonders bevorzugt 5-10 Vol%, ein oder mehrere organische, mit Wasser mischbare Lösungsmittel zugesetzt werden (z.B. Methanol, Ethanol, Isopropanol, n-Propanol, Aceton, Tetrahydrofuran). Bevorzugt setzt man als Co-Solvenz 1-10 Vol% des Lösungsmittels zu, in dem die vorausgegangene Vierkomponenten-Kupplung (Verfahrensschritt 1) durchgeführt wurde, soweit dieses Lösungsmittel mit Wasser mischbar ist. Besonders bevorzugt ist die Verwendung von Methanol, Ethanol, n-Propanol oder Isopropanol sowohl als Lösungsmittel für die Vierkomponenten-Kupplung, als auch als Co-Solvenz für die Freisetzung von (I) im wäßrigen Milieu. Ganz besonders bevorzugt wird das Mannich-Salz (III) alkoholfeucht, wie in der Schleuderung erhalten, ohne vorherige Trocknung, für die Freisetzung im wäßrigen Milieu eingesetzt. Ob und in welchem Umfang ein organisches Co-Solvenz der wäßrigen Suspension von (III) zugesetzt werden muß, hängt von der Löslichkeit und wäßrigen Benetzbarkeit von (III), somit von der Natur seiner Substituenten R¹ bis R⁵ und seines Anions Y*⁻ ab. Der Co-Solvenz-Zusatz wird bevorzugt soweit minimiert, wie dies mit einer akzeptablen Freisetzungsgeschwindigkeit unter pH-Stat-Bedingungen vereinbar ist. Unnötig hoher Co-Solvenz-Zusatz zum wäßrigen Milieu kann die isolierte Ausbeute an freier Mannich-Base (I) senken oder eine aufwendigere Isolierung von (I) erforderlich machen (Abdestillieren des Co-Solvenz aus der Reaktionssuspension vor dem Abschleudern des Feststoffs zwecks vollständiger Ausfällung von (I) in der Suspension). Außerdem kann ein unnötig hoher Co-Solvenz-Zusatz die Retro-Mannich-Reaktion während der Freisetzung unter pH-Stat-Bedingungen fördern und damit Ausbeute, chemische Reinheit, Diastereomeren-Reinheit und Enantiomerenreinheit des Produkts (I) verschlechtern.

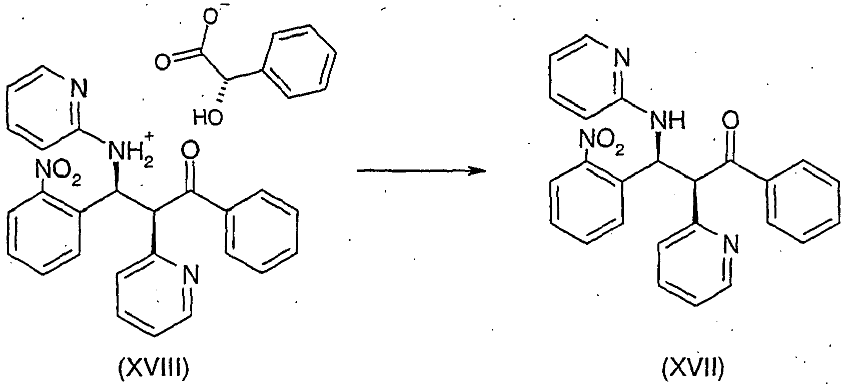

Die Freisetzung einer Mannich-Base der Formel (I), wird im folgenden beispielshaft an der Reaktion einer Verbindung der Formel (I) erläutert, in der R¹ = o-Nitrophenyl, R² = 2-Pyridyl, R³ = H, R⁴ = 2-Pyridyl und R⁵ = Phenyl ist (Verbindung der Formel (XVII)), wobei (XVII) unter verschiedenen Bedingungen aus dem entsprechenden Mandelat-Salz der Formel (XVIII) freigesetzt wurde.

Tabelle 7 zeigt die Ergebnisse der Umsetzung der Verbindung (XVIII) zu Verbindung (XVII):

| Tabelle 7 | | | | | | | | | | | | |
|---|---|---|---|---|---|---|---|---|---|---|---|---|
| Nr | Edukt (XVIII) mmol | Edukt (XVIII) trans-Gehalt (NMR) ee (Piv-Derivat.) ee (Camph-Derivat.) | Base (mmol) Äquiv. | Lösemittel [mL] | Lösemittel-zusatz [mL] | Versuchsbeschreibung | Grad der Freisetzung (NMR) [%] | Produkt (XVII) isolierte Ausbeute [% der Theorie] | Produkt (XVII) ee [%] (Camph.-Derivat) | Produkt (XVII) ee [%] (Piv-Derivat) | trans-Isomeren-Gehalt (NMR) [%] | H2O-gehalt (Karl-Fischer -Titrat.) [%] |
| 1 | 1,7 | 1,4 % trans ee (Camph) : 96,2% ee (Piv) : nicht bestimmt | NaOH 2N (1,9) 1,1 | H2O 5 | keiner | Mandelat-Salz (XVIII) in H2O susp., bei Rt 2N NaOH auf einmal zugeg., 14 h rühren -> beige-gelbe Susp., abgesaugt, mit H2O gewaschen., getrocknet | 100 | 96,5 | 96,0 | 98,2 | ca. 10 | |
| 2 | 35 | 1,4 % trans ee (Camph) : 96,2% ee (Piv) : n.b. | NaOH 2N (38) 1,1 | H2O 100 | keiner | Mandelat-Salz (XVIII) in H2O susp., bei Rt 2N NaOH auf einmal zugeg., 14 h rühren -> beige-gelbe Susp., abgesaugt, mit H2O gewaschen., getrocknet | 100 | 98,8 | 91,4 | 94,8 | 9,1 | 0,4 |
| 3 | 1,7 | 1,4 % trans ee (Camph) : 96,2% ee (Piv) : n.b. | NaOH 2N (1,64) 0,95 | H2O 5 | keiner | Mandelat-Salz (XVIII) in H2O susp., bei 0°C 2N NaOH auf einmal zugeg., 14 h rühren u. Erwärm. auf Rt -> beige-gelbe Susp., abgesaugt, mit H2O gewaschen, getrocknet | 100 | 99,4 | 79,5 | | 5,3 | |
| 4 | 1,7 | 1,4 % trans ee (Camph) : 96,2% ee (Piv) : n.b. | NaOH 2N (1,73) 1,0 | H2O 5 | keiner | Mandelat-Salz (XVIII) in H2O susp., bei 0°C 2N NaOH auf einmal zugeg., 14 h rühren u. Erwärm. auf Rt -> beige-gelbe Susp., abgesaugt, mit H2O gewaschen, getrocknet | 100 | 100 | 91,8 | | 6,2 | |
| 5 | 1,7 | 1,4 % trans ee (Camph): 96,2% ee (Piv) : n.b. | NaOH 2N (1,81) 1,1 | H2O 5 | keiner | Mandelat-Salz (XVIII) in H2O susp., bei 0°C 2N NaOH auf einmal zugeg., 14 h rühren u. Erwärm. auf Rt -> beige-gelbe Susp., abgesaugt, mit H2O gewaschen, getrocknet | 100 | 96,5 | 93,0 | | 7,0 | |
| 6 | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 1 h bei Rt rühren -> beige-gelbe Susp., abgesaugt, H2O gew., getr. | 20 | 96,9 unter Berücksichtigung des Restgeh. (XVIII) | 89,0 | | 3,7 | 0,2 |
| 7 | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 1 h bei Rt rühren -> beige-gelbe Susp., abgesaugt, mit H2O / EtOH qew., qetr. | 19 | 94,3 unter Berücksichtigung des Restgeh. (XVIII) | 96,2 | | 1,3 | 0,3 |
| 8 | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 3 h bei Rt rühren -> beige-gelbe Susp., pH 11, abgesaugt, mit H2O gewaschen, getrocknet | 78 | 97,5 unter Berücksichtigung des Restgeh. (XVIII) | 82,2 | | 9,1 | 0,7 |
| 9 | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 5.5 h bei Rt rühren -> beige-gelbe Susp., pH 7.5, abgesaugt, mit H2O gewaschen, getrocknet | 100 | 94,0 | 91,8 | 95,5 | 8,9 | 0,7 |
| 10 | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 21.5 h bei Rt rühren -> beige-gelbe Susp., pH 7.5, abgesaugt, mit H2O gewaschen, getrocknet | 100 | 95,6 | 93,5 | 96,6 | 8,9 | 1,5 |
| | 8,7 | 1,4 % trans ee (Camph) : 96,2% ee (Piv) : n.b. | NaOH 2N (9,54) 1,1 | H2O 25 | keiner | Mandelat-Salz (XVIII) in H2O susp., bei Rt 2N NaOH auf einmal zugeg., 21.5 h bei Rt rühren -> beige-gelbe Susp., pH 7.5, abgesaugt, mit H2O / EtOH gewaschen, getrocknet | 100 | 95,4 | 92,3 | 96,2 | 8,2 | 0,9 |
| 12 | 8,7 | 1,4 % trans ee (Camph) : 96,2% ee (Piv) : n.b. | NaHCO3 (17,34) 2,0 | H2O 25 | keiner | Mandelat-Salz (XVIII) in H2O susp., bei 0°C NaHCO3 auf einmal zugeg., 14 h bei 0°C rühren -> gelbe Susp., pH 8,5 , abgesaugt, mit H2O gewaschen, getrocknet | 2,4 | 100,2 unter Berück-sichtigung des Restgeh. (XVIII) | | 97,4 | 1,5 | |
| 13 | 8,7 | 1,4 % trans ee (Camph) : 96,2% ee (Piv) : n.b. | NaOH 2N (9,1) 1,05 | H2O 25 | keiner | Mandelat-Salz (XVIII) in H2O susp., bei 0°C 2N NaOH in 5 h zudosiert, 19 h bei 0°C rühren -> beige-gelbe Susp., pH 11, abgesaugt, mit H2O gewaschen, getrocknet | 13 | 98,6 unter sichtigung Restgeh. | Berückdes (XVIII) | | 2,5 | |
| 14 - | 8,7 | 1,4 % trans ee (Camph) : 96,2% ee (Piv) : n.b. | NaHCO3 (17,34) 2,0 | H2O 25 | Aceton 1,25 | Mandelat-Salz (XVIII) in H2O susp.,bei 0°C NaHCO3 dann Aceton zugeg., 23 h bei 0°C rühren -> gelbe Susp., pH 9,5 , abgesaugt, mit H2O gewaschen, getrocknet | 100 | 95,6 | 96,4 | 97,0 | Probe 4 h 1,4 1,9 | 0,5 |
| 15 | 8,7 | 1,4 % trans ee (Camph) : 96,2% ee (Piv) : n.b. | NaOH 2N (9,1) 1,0 | H2O 25 | keiner | Mandelat-Salz (XVIII) in H2O susp., bei 0°C 2N NaOH in 5 h zudosiert, 15 h bei 0°C rühren -> beige-gelbe Susp., pH 12, abgesaugt, mit H2O gewaschen, getrocknet | 10,7 | 98,4 unter Berücksichtigung des Restgeh. (XVIII) | | | 1,4 | 0,3 |
| 16 | 104 | 1,4 % trans ee (Camph) : 96,2% ee (Piv) : n.b. | NaHCO3 (208,1) 2,0 | H2O 300 | Aceton 15 | Mandelat-Salz (XVIII) in H2O susp., bei 0°C NaHCO3 dann Aceton zugeg., 19 h bei 0°C rühren -> gelbe Susp., pH 8,5 , abgesaugt, mit H2O gewaschen, getrocknet | Probe nach 1 h 26,5 3 h 39,8 20 h 90,4 | 98,5 | | 94,0 | Probe 1 h 1,5 3 h 1,7 20h 2,0 | 0,5 |
| 17 | 8,7 | 1,6 % trans ee (Camph) = 96,3% ee (Piv) : 96,3% | NaHCO3 (17,34) 2,0 | H2O 25 | Aceton 2,5 | Mandelat-Salz (XVIII) in H2O susp., bei 0°C NaHCO3 dann Aceton zugeg., 20 h bei 0°C rühren -> gelbe Susp., pH 8,5 , abgesaugt, mit H2O gewaschen, getrocknet | 97,5 | 98,6 | | 95,9 | 1,4 | 0,1 |
| 18 | 8,7 | 1,6 % trans ee (Camph) : 96,3% ee (Piv) : 96,3% | NaHCO3 (17,34) 2,0 | H2O 25 | Aceton 2,5 | Mandelat-Salz (XVIII) in H2O susp., bei Rt NaHCO3 dann Aceton zugeg., 19 h bei Rt rühren -> gelbe Susp., pH 8,5 , abgesaugt, mit H2O gewaschen, getrocknet | 100 | 98,6 | | 96,0 | 1,4 | 0,2 |
| 19 | 8,7 | 1,6 % trans ee (CAmph) : 96,3% ee (Piv) : 96,3% | NaHCO3 (17,34) 2,0 | H2O 25 | Ethanol 2,5 | Mandelat-Salz (XVIII) in H2O susp., bei Rt NaHCO3 dann EtOH zugeg., 21 h bei Rt rühren -> gelbe Susp., pH 9,5 , abgesaugt, mit H2O gewaschen, getrocknet | 100 | 105 | | 97,0 | 0,7 | 0,3 |
| 20 | 43 | 1,6 % trans ee (Camph) : 96,3% ee (Piv) : 96,3% | NaOH 2N (21,48) 0,99 | H2O 125 | Ethanol 12,5 | Mandelat-Salz (XVIII) in H2O / EtOH susp., bei Rt 2N NaOH bei pH-stat 8,5 zudosiert, 4 h bei Rt rühren -> gelbe Susp., pH 9,5 , abgesaugt, mit H2O gewaschen, getrocknet | 100 | 98,4 | | 96,7 | 1,0 | 0,6 |
| 21 | 2274 | ? % trans ee (Camph) : 93,4% ee (Piv) = n.b. | NaOH 2N (2.280) 1,002 | H2O 5686 | Ethanol 569 | Mandelat-Salz (XVIII) in H2O / EtOH susp., bei Rt 2N NaOH bei pH-stat 8,5 zudosiert, 21 h bei Rt rühren -> gelbe Susp., pH 8,7 , abgesaugt, mit H2O gewaschen, getrocknet | 100 | 99,5 | | 95,6 | 1,2 | 0,6 |
| 22 | 520 | 1,6 % trans ee (Camph) : 96,3% ee (Piv) : 96,3% | NaHCO3 (1040,4) 2,0 | H2O 1500 | Aceton 150 | Mandelat-Salz (XVIII) in H2O susp., bei 0°C NaHCO3, dann 5 % Aceton zugegeben, 18 h bei 0°C rühren, 5 % Aceton zugegeben, 20 h bei 0°C rühren, 5 h Rt. -> gelbe Susp., abges., H2O gew., getr. | Probe nach 18 h 50 23 h 94,75 25 h 99,13 100 | 101,4 | | 95,2 | < 1 | 0,5 |
| 23 | 397 | ? % trans ee (Camph) : 95,7% ee (Piv) : 95,6% | NaHCO3 (793,24) 2,0 | H2O 1143 | Aceton 114 | Mandelat-Salz (XVIII) in H2O susp., bei 10°C NaHCO3 und Aceton zugeg., 19 h bei 10°C rühren -> gelbe Susp., abgesaugt, mit H2O gewaschen, getrocknet | Probe nach 4,5 h 84,6 7,5 h 90,9 ca.22 h 100 100 | 99,9 | 96,8 | 96,2 | < 1 | 0,3 |
| 24 | 17 | 0,9 % trans ee (Camph) : 94,6% ee (Piv) : 95,2% | NaHCO3 (34,75) 2,0 | H2O 50 | Aceton 5 | Mandelat-Salz (XVIII) in H2O susp., bei Rt NaHCO3 und Aceton zugeg., 2 h bei 40°C rühren -> gelbe Susp., abgesaugt, mit H2O gewaschen, getrocknet | 100 | 96,7 | 94,8 | 94,8 | 1,2 | |
| 25 | 33 | 1,4 % trans ee (Camph) : 95,7% ee (Piv) = 95,6% | NaOH 2N (31,25) 0,95 | H2O 95 | Ethanol 9,5 | Mandelat-Salz (XVIII) in H2O susp., bei 40°C EtOH zugeg., dann 2N NaOH bei pH-stat 8,5 zudosiert (80 min bei 40°C) -> gelbe Susp., pH 8,7 , abges., mit H2O gew., getr. | Probe nach 0,5 h 68,5 1 h 96,6 1,33 h 100 | 99,6 | 94,9 | 94,1 | 1,4 | |
| 26 | 445 | ? % trans ee (Camph) : 94,3% ee (Piv) : 97,5% | NaOH 2N (443,83) 0,997 | H2O 1280 | Ethanol 128 | Mandelat-Salz (XVIII) in H2O susp., bei Rt EtOH zugeg., dann 2N NaOH bei pH-stat 8,5 zudosiert (3.5 h bei Rt, 4.5 h bei 40°C), 9.5 h bei Rt rühren -> gelbe Susp., pH 9.2, abges., mit H2O gew., getr. | Probe nach 1 h 39,3 2,5 h 70,7 6 h 91,9 8 h 96,8 10 h 100 | 99,3 | 92,8 | 92,5 | 1,5 | 0,43% |

In den Umsetzungen, die in Tabelle 7 beschrieben sind, wurde einer Suspension von (XVIII) in reinem Wasser 0.95 -1.10 Äquiv. 2N Natronlauge auf einmal bei 0°C oder bei Raumtemperatur zugesetzt, dann erfolgte die Freisetzung von (XVII) quantitativ , war aber von der Bildung von 5 bis 10% des anti-Diastereomers von (XVII) begleitet (Nr. 1 - 5 und 8 - 11). In Abhängigkeit von den konkreten Reaktionsbedingungen war die Abnahme des Enantiomerenüberschusses von (XVII) entweder nur minimal. (Nr. 1), geringfügig (Nr. 2, 4, 5, 9-11) oder deutlich (Nr. 3 und 8). Unmittelbar nach der Zugabe der gesamten Natronlaugemenge in einer Portion war die Hydroxidionen-Konzentration also so hoch, daß es in erheblichem Umfang nicht nur zur gewünschten Deprotonierung der Ammoniumfunktion des Mannich-Salzes (XVIII) kam, sondern auch zur unerwünschten Deprotonierung seiner C-H aciden α-Position zur Carbonylgruppe.

Da die Reprotonierung des entstehenden Enolat-lons von (XVII) nicht stereospezifisch, sondern in ähnlichem Umfang von beiden Seiten der Enolat-Ebene erfolgt, entsteht neben (XVII) auch sein anti-Isomer. Wurde nach der Natronlauge-Zugabe die Rührzeit auf 1 Stunde bei Raumtemperatur beschränkt, dann bildete sich das anti-Isomer nur zu 1,3 - 3,7% (Nr. 6 und 7), aber der Grad der Freisetzung betrug in dieser Zeit nur ca. 20% und bei einem der Versuche fiel der Enantiomerenüberschuß des Salzes (XVIII) (96.2% ee) auch um 7% auf nur noch 89.0% in der freien Base (XVII) ab (Nr. 6).

Wenn der wäßrigen Suspension des Mandelats (XVIII) anstatt Natronlauge 2 Äquivalente Natriumhydrogencarbonat bei 0°C zugesetzt wurden, trat innerhalb 14 Stunden nur 2,4% Freisetzung ein (Nr. 12), das abgesaugte Produkt als (XVIII) / (XVII) -Gemisch enthielt aber keine gesteigerte Menge anti-Isomer. Ebenso trat nur 11-13% Freisetzung ein, wenn 1 Äquivalent 2N Natronlauge sehr langsam über 5 Stunden bei 0°C zur rein wäßrigen Suspension von (XVIII) zudosiert wurde (Nr. 13 und 15).

Der Zusatz von 5 bzw. 10 Vol% Aceton zur Freisetzung mit 2 Äquivalenten NaHCO₃ bewirkte aber quantitative Bildung der freien Base (XVII) unter vollständigem Erhalt der Enantiomerenreinheit und ohne signifikanten Anstieg des anti-Isomers, sowohl bei 0°C (Nr. 14, 16, 17, 22) als auch bei 10°C (Nr. 23), bei Raumtemperatur (Nr. 18), oder bei 40°C (Nr. 24). Nochmals marginal bessere Ergebnisse wurden mit Natriumhydrogencarbonat in Wasser / Ethanol (10:1) bei Raumtemperatur erzielt (Nr. 19).

Gleichwertige Ergebnisse wurden beim Zudosieren von 0.95 - 1.00 Äquivalenten 2N Natronlauge bei pH 8.5 (Einsatz einer Autobürette unter pH-Stat-Bedingungen) zur Suspension von (XVIII) in Wasser / Ethanol (10:1) erzielt (Nr. 20, 21, 25, 26). Erhaltung der Enantiomeren- und Diastereomeren-Reinheit schienen bei Raumtemperatur (Nr. 20 und 21) etwas besser als bei 40°C (Nr. 25 und 26) zu sein.

Der erfindungsgemäße Verfahrensschritt 2 bietet die Möglichkeit, die während der Vierkomponentenkupptung eingesetzte optisch aktive Säure HY^{*} der Formel (VII) weitgehend und in unveränderter Enantiomerenreinheit aus der schwach basischen, wäßrigen Mutterlauge der Freisetzungsreaktion zurück zu gewinnen. Die hierfür bevorzugte Methode hängt von der Löslichkeit, sowie der chemischen und optischen Stabilität der chiralen Säure in saurem wäßrigem Milieu ab. Bei Säuren (VII), die in Wasser bei ca. pH 3 sehr unlöslich sind, ist es meistens ausreichend, die Mutterlauge anzusäuern und den ausgefallenen Feststoff (VII) abzuschleudern oder abzufiltrieren. Wurde eine α-Aminosäure als chirale Säure (VII) verwendet, dann reicht es in der Regel, die wäßrige Mutterlauge der Freisetzung auf den isoelektrischen Punkt der α-Aminosäure anzusäuern und den Feststoff anschliessend abzuschleudern oder abzufiltrieren. Liegt, wie z.B. im Falle der Weinsäure, der Äpfelsäure oder der Mandelsäure eine nicht unerhebliche Wasserlöslichkeit der chiralen Säure (VII) vor, oder besteht die Gefahr einer partiellen Racemisierung unter zu stark aciden Bedingungen, dann ist die bevorzugte Rückgewinnungsmethode häufig die Extraktion aus der schwach angesäuerten wäßrigen Mutterlauge. Beispielsweise gelingt die Rückgewinnung von (S)-(+)-Mandelsäure in 88% Ausbeute, >99.5% chem. Reinheit, und 100% ee per Ethylacetat-Extraktion.

Bei sehr hoher Wasserlöslichkeit, Mineralsäure-Empfindlichkeit oder einem hohen Preis des chiralen Auxiliars kommen auch andere Rückgewinnungsmethoden, z.B. Gefriertrocknung der neutralisierten wäßrigen Mutterlauge der Freisetzungsreaktion, in Betracht.

Desweiteren wurde eine einfache Reduktionsmethode gefunden, mit der β-Aminoketone der Formel (I) oder ihre Salze der Formel (III) mit sehr hoher Diastereoselektivität zu 1,3-Aminoalkoholen reduziert werden, wobei die Reduktion ohne Verlust der in den Verbindungen der Formel (I) bzw. (III) bereits enthaltenen stereochemischen Reinheit verläuft und keine chiralen Hilfsstoffe eingesetzt werden müssen.

Gegenstand der vorliegenden Erfindung ist ferner ein Verfahren zur Herstellung eines optisch aktiven 1,3-Aminoalkoholes der Formel (II) oder dessen Spiegelbild, 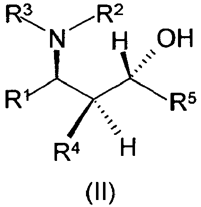 dadurch gekennzeichnet, dass in Verfahrensschritt 1 die Verbindung der Formel (III) wie oben beschrieben hergestellt wird, in Verfahrensschritt 2 die Verbindung (III) wie oben beschrieben zu einer Verbindung der Formel (I) umgesetzt wird,
und entweder in einem an Verfahrensschritt 2 anschließenden Verfahrensschritt 3 die Verbindung der Formel (I) mit einem geeigneten Reduktionsmittel zu einer Verbindung der Formel (II) reduziert wird, und anschließend optional die Verbindung der Formel (II) mit an sich bekannten Methoden aufgearbeitet wird, oder
alternativ zu den Verfahrensschritten 2 und 3 in einem Verfahrensschritt 4 die Verbindung der Formel (III) mit einem geeigneten Reduktionsmittel zu einer Verbindung der Formel (II) reduziert wird, und anschließend optional die Verbindung der Formel (II) mit an sich bekannten Methoden aufgearbeitet wird,
wobei die Reste R¹, R², R³, R⁴ und R⁵ und Y*⁻wie oben definiert sind.

Geeignete Reduktionsmittel sind Boran- oder Borhydrid-Reagenzien, optional in Gegenwart eines chiralen Katalysators.

Im Rahmen des erfindungsgemäßen Verfahrensschritts 3 wird eine deutliche Diastereoselektion bei der Reduktion der Ketogruppe von optisch aktiven α-Aminoketonen (I) zugunsten von 1,3-Aminoalkoholen der Formel (II) beim Einsatz von Boran- oder Borhydrid-Reagenzien erzielt.

Die diastereoselektive Reduktion von (I) zu (II) kann mit achiralen Reduktionsmitteln (Prinzip der simplen Diastereoselektion) oder in Gegenwart optisch aktiver Katalysatoren erzielt werden, wobei in letzterem Fall die Enantioselektivität des katalytisch aktiven Reagenzes der simplen Diastereoselektion überlagert ist und meist dominiert. Bei der Reduktion in Gegenwart optisch aktiver Katalysatoren werden hohe Diastereomeren-Überschüsse erzielt, wenn die Enantioselektivität des chiralen. Katalysators der simplen Diastereoselektivität der Reduktion gleichgerichtet ist ("matched case"). Niedrigere Diastereomeren-Überschüsse werden erhalten, wenn der Katalysator die entgegengesetzte Absolutkonfiguration hat und seine Enantioselektivität darum der simplen Diastereoselektivität entgegengerichtet ist ("mismatched case").

Achirale Reduktionsmittel (Prinzip der simplen Diastereoselektion) sind beispielsweise:
1. ein Boran-Sulfid-Komplex, z.B. Boran-Dimethylsulfid- oder Boran-1,4-Thioxan-Komplex;
2. ein Boran-Etherat, z.B. Boran-Tetrahydrofuran-Komplex;
3. Catecholboran;
4. ein Boran-Sulfid-Komplex oder ein Boran-Etherat oder Catecholboran in Gegenwart einer Lewis-Säure, z.B. Titanchlorid-triisopropylat (iPrO)₃TiCl;
5. ein Boran-Amin-Komplex, z.B. Boran-Ammoniak-, Boran-tert.Butylamin-, Boran-N,N-Diethylanilin-, Boran-N-Ethyldiisopropylamin-, Boran-N-Ethylmorpholin-, Boran-N-Methylmorpholin, Boran-Morpholin-, Boran-Piperidin-, Boran-Pyridin-, Boran-Triethylamin-, Boran-Trimethylamin-Komplex;
6. ein Boran-Amin-Komplex in Gegenwart einer Lewissäure, z.B. Titanchlorid-triisopropylat (iPrO)₃TiCl;
7. ein Boran-Phosphin-Komplex, z.B. Boran-Tributylphosphin oder Boran-Triphenylphosphin-Komplex;
8. eine Kombination eines Borhydrids, bevorzugt Natriumborhydrid oder Tetraalkylammoniumborhydrid, mit einem Reagenz, das zur in situ -Erzeugung von Boran führt. Solche Kombinationen sind z.B. Natriumborhydrid / Iod, Natriumborhydrid / Bortrifluorid-Diethyletherat, Natriumborhydrid / Chlortrimethylsilan; Tetraalkylammoniumborhydrid / Alkylhalogenid (z.B. Methyliodid) in Dichlormethan oder das Zweiphasengemisch eines Alkylbromids (z.B. n-Butylbromid) und einer gesättigten wäßrigen Lösung von Natriumborhydrid und katalytischen Mengen (ca. 10 mol%) eines quaternären Onium-Salzes als Phasentransfer-Katalysator (B. Jiang, Y. Feng, J. Zheng Tetrahedron Lett. 2000, 41, 10281)
9. ein Borhydrid eines ein- oder zweiwertigen Metallkations, z.B. Natriumborhydrid, Lithiumborhydrid oder Zinkborhydrid, bzw. ein Tetraalkylammonimborhydrid, mit oder ohne Gegenwart eines Cer(III)-Salzes, z.B. CeCl₃, als Additiv;
10. Diboran (B₂H₆).

Folgende Reduktionen in Gegenwart eines oder mehrerer optisch aktiver Katalysatoren können beispielsweise eingesetzt sind:
1. ein Borhydrid eines ein- oder zweiwertigen Metallkations, bevorzugt Natriumborhydrid, in Gegenwart katalytischer Mengen eines optisch aktiven Aldiminato-Kobalt(II)-Komplexes, z.B. (1S,2S)-N,N'-Bis[3-oxo-2-(2,4,6-trimethylbenzoyl) butylidene]-1,2-diphenylethylendiaminato Kobalt(II) (S)-MPAC, mit oder ohne Gegenwart von Tetrahydrofurfurylalkohol als Co-Ligand. Diese Reagenzien-Kombination wurde von (T. Makaiyama et al., Synlett 1996, 1076) beschrieben. Sie führt zu einer katalytischen enantioselektiven Borhydrid-Reduktion von Carbonylgruppen. Bei der hier vorliegenden neuartigen Anwendung zur Reduktion von Mannich-Basen (I) kann die natürliche Diastereoselektivität von Natriumborhydrid durch die gleichgerichtete Enantioselektivität des Reagenzes verstärkt werden.
2. ein Borhydrid eines ein- oder zweiwertigen Metallkations, bevorzugt Natriumborhydrid, katalysiert durch einen Rhodium-Komplexes, der durch Koordination von zwei Molekülen optisch reinem 1,3-Aminoalkohol (II) pro Molekül [(µ⁵)-Pentamethyl-cyclopentadienyl]-rhodiumdichlorid-Dimer entsteht. Es ist hier möglich und vorteilhaft, die Substituenten R¹ bis R⁵ im chiralen Liganden (II) so zu wählen, daß sie identisch mit denen des entstehenden Reduktionsprodukts (II) sind, die Natriumborhydrid-Reduktion also autokatalytisch erfolgt. Solche Katalysatoren sind den CATHy™ - Katalysatoren der Firma AVECIA (WO 98/42643) ähnlich, unterscheiden sich aber in den folgenden Punkten:
   - CATHy™ - Katalysatoren werden aus dem Cyclopentadienyl-rhodiumchlorid-Dimer und chiralen 1,2-Aminoalkoholen, wie z.B. cis-1-Amino-2-indanol, hergestellt. In der vorliegenden Anmeldung werden chirale 1,3-Aminoalkohole verwendet.
   - CATHy™ - Katalysatoren wurden zu enantioselektiven Transfer-Hydrierungen eingesetzt, bei denen sekundäre Alkohole, bevorzugt Isopropanol, oder Triethylamin / Ameisensäure-Gemische als Wasserstoffdonatoren fungieren. In der vorliegenden Anmeldung fungiert hingegen ein Boranat, bevorzugt Natriumborhydrid, als Reduktionsmittel.
   - CATHy™ - Katalysatoren wurden zu enantioselektiven Transferhydrierungen von verschiedenen prochiralen Ketonen eingesetzt, jedoch noch nicht für die Reduktion der Keto-Gruppe in racemischen oder optisch aktiven Mannich-Basen (wie z.B. (I)) bzw. deren Salzen (wie z.B. (III)).

Bevorzugt sind als geeignete Reduktionsmittel ein Boran-Sulfid-Komplex, ein Boran-Etherat, Natriumborhydrid oder ein Natriumborhydrid-Komplex enthaltend einen in situ Katalysator, der durch Koordination von [(µ⁵)-Pentamethylcyclopentadienyl]-rhodiumdichlorid-Dimer an den optisch aktiven 1,3-Aminoalkohol (II) erzeugt wird.

Besonders bevorzugt ist als geeignetes Reduktionsmittel ein Boran-Dimethylsulfid- oder Boran-Tetrahydrofuran-Komplex.

Aufgrund seiner Titerstabilität bei Lagerung bei Raumtemperatur sowie der industriellen Verfügbarkeit in hoher Konzentration (94-95%ige Flüssigkeit) ist Boran-Dimethylsulfid-Komplex ganz besonders bevorzugt.

Die Reaktion wird unter Einsatz von 0.3 - 10.0 Mol-Äquivalenten eines der genannten Reduktionsmittel durchgeführt, bevorzugt mit 0.5 - 4.0 Mol-Äquivalente, besonders bevorzugt mit 1.0 - 2.5 Mol-Äquivalenten.

Verfahrensschritt 3 und 4 erfolgt beispielsweise in einem aromatischen Kohlenwasserstoff (z.B. Toluol, Cumol, Xylol, Tetralin, Pyridin), einem gesättigten Kohlenwasserstoff (z.B. Cyclohexan, Heptan, Pentan), einem Ether (z.B. Anisol, Tetrahydrofuran, tert.-Butylmethylether, Diisopropylether, 1,2-Dimethoxyethan, 1,4-Dioxan), einem chlorierten Kohlenwasserstoff (z.B. Dichlormethan, Chloroform, Chlorbenzol), einem Amid (z.B. N-Methylpyrrolidon, N,N-Dimethylacetamid), einem Ester (z.B. Isobutylacetat, Butylacetat, Isopropylacetat, Propylacetat, Ethylacetat) oder einem Sulfoxid bzw. Sulfon (z.B. Dimethylsulfoxid oder Sulfolan) als Lösungsmittel. Die drei letztgenannten Klassen von Lösungsmitteln sind gegenüber dem Boran nicht inert. Bevorzugt wird die Reaktion in Toluol, Cumol, Tetrahydrofuran oder Anisol durchgeführt. Besonders bevorzugt ist Toluol, Cumol, oder THF. Die Reduktionsreaktion wird im Temperaturbereich von -70°C bis zum Siedepunkt des verwendeten Lösungsmittels, vorzugsweise 120°C durchgeführt, bevorzugt bei -10°C bis +40°C, besonders bevorzugt bei 0°C bis +25°C.

Es bestehen die Optionen
a) die Lösung des Boran-Komplexes zu der Suspension bzw. Lösung der Mannich-Base (I) zu geben ("normale Addition"), oder
b) die Suspension bzw. Lösung der Mannich-Base (I) zu der vorgelegten Lösung des Boran-Komplexes zu geben ("inverse Addition").

Die Reaktionsdauer der Reduktion ist vom konkreten Edukt (Natur der Substituenten R¹ bis R⁵), von der gewählten Reaktionstemperatur und der Löslichkeit des Edukts im Lösungsmittel abhängig. Sie beträgt ca. 30 Minuten bis 3 Tage, bevorzugt 1 Stunde bis 5 Stunden, besonders bevorzugt 1 - 2 Stunden.

Bei Verwendung der besonders bevorzugten Reduktionsmittel Boran-Dimethylsulfid- bzw. Boran-THF-Komplex ist das Primärprodukt der Reaktion ein Diastereomerengemisch von Oxazaborinanen, welches sich, soweit gewünscht, leicht isolieren läßt und dessen stark dominierender Komponente basierend auf seinem HPLC-Verhalten, seiner per HPLC/MS bestimmten Molmasse (M+H⁺: m/z = 437.3) und seiner unter Einwirkung von Methanol / Methansulfonsäure glatt verlaufenden Umwandlung in den 1,3-Aminoalkohol (II) die Formel (C) zugeordnet wird.

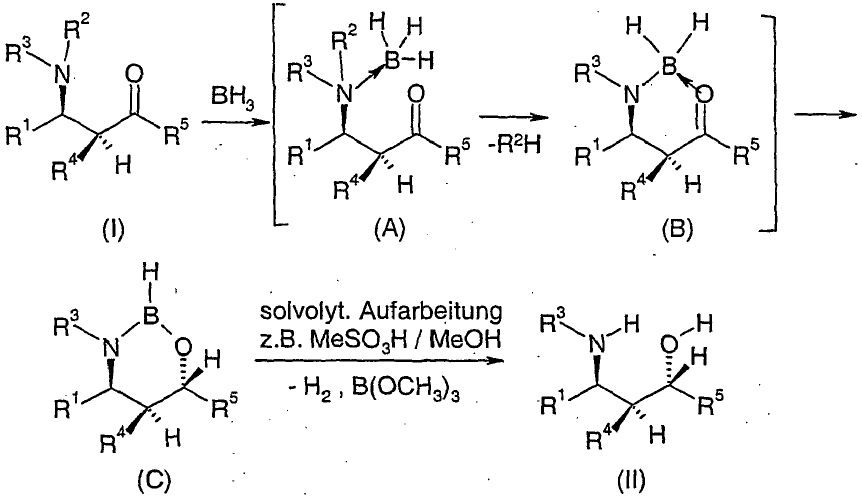

Tabelle 8 faßt die Resultate einer beispielhaften Umsetzung des Aminoketons (XVII) (Verbindung der Formel (I), wobei R¹ = o-Nitrophenyl, R² = 2-Pyridyl, R³ = H, R⁴ = 2-Pyridyl, R⁵ = Phenyl) zum 1,3-Aminoalkohol (XIX) bzw. dessen Diastereomer dia-(XIX) zusammen:

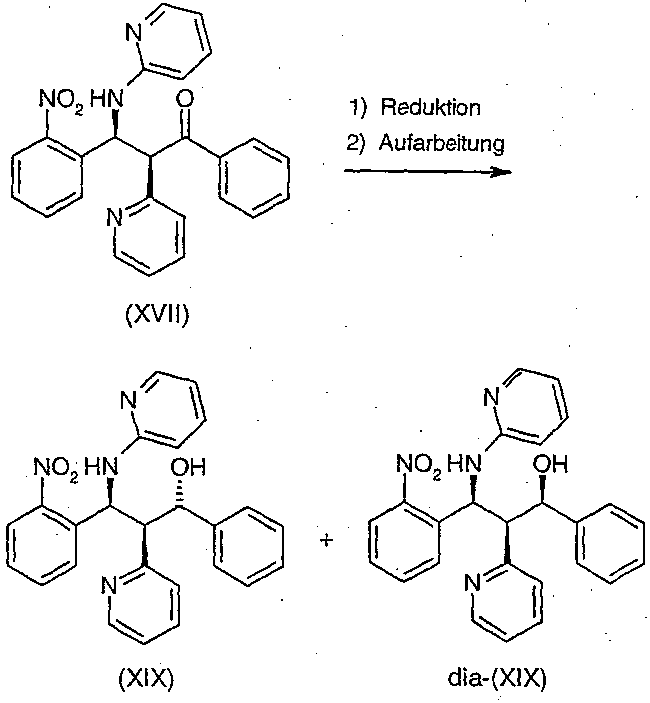

| Tabelle 8 | | | | | | | | |
|---|---|---|---|---|---|---|---|---|
| Nr. | Edukt (XVII) | BH₃-Me₂S bzw. Alternative; Äquivente; Zugabezeit | Lösungsmittel (Volumen) | Rührtemperatur und -zeit | Aufarbeitung | Verhältnis (XIX) / dia- (XIX), (a) | Summe Verunreinigungen, (a) | Ausbeute % isoliertes Reinprodukt (Rohprodukt) |
| 1 | racemisch , 10 mmol Zugabe als Feststoff in 5 Portionen a 848 mg im Abstand von je 1.5 h | NaBH₄ 1.34 vorgelegt | EtOH (25 mL) | RT Zugabe:8h Nachrühren: 15h | (XVII) quant. umgesetzt. Nicht aufgearbeitet | 69: 31 | 8.0% | n.b. |
| 2 | racemisch, 2 mmol (XVII) und CeCl₃ x 7 H₂O (2 mmol) vorgelegt | NaBH₄ 1.1 1 min | EtOH (10 mL) | 0°C bis RT 4 h | (XVII) ca. 50% umgesetzt. Nicht aufgearbeitet | 83 : 17 | ca. 7% | n.b. |
| 3 | racemisch 2 mmol (XVII) | BH₃-*tert.*-Butylamin 3.0 | MeOH (20 mL) | RT 24 h | nicht aufgearbeitet | 61.3 : 38.6 | 9.2% | n.b. |
| 4 | racemisch, 2 mmol (XVII) und (iPrO)₃TiCl (2 mmol) vorgelegt | BH₃-*tert*.-Butylamin 2.2 , 10 s | MeOH (20 mL) | 0°C / 3 h, dann RT/12h | <50% Umsatz von (XVII). Nicht aufgearbeitet | 70:30 | | n.b. |
| 5 | racemisch 5 mmol | BH₃-THF (1.0 M) 3.0 b) 15 min | THF (45 mL) | +1°C 1 h über Nacht auf RT | 2 N HCl (16 Äquiv.) | 87.8 : 12.2 | 7.7% | (103%) |
| 6 | racemisch 5 mmol | 3.0 , (b) (95%) 2 min | Toluol (25 mL) | 0°C bis RT 2 h bei RT | MeSO₃H (3.0 Äquiv.) in MeOH | 96.7: 3.2 | 7.2% | (83.4%) |
| 7 | racemisch 5 mmol | 3.0 , (b) (95%) 2 min | MTB-Ether (25 mL) | 0°C bis RT 2.5 h bei RT | MeSO₃H (3.0 Äquiv.) in MeOH | 94.4 : 5.5 | 11.7% | (105%) |
| 8 | racemisch 15 mmol | 3.0 , (b) (95%) 2 min | Toluol (75 mL) | 0°C bis RT 1.75 h bei RT | 2 N HCl (9 Äquiv.) | geringe Spaltung des Oxazaborinan | | n.b. |
| 9 | racemisch 15 mmol) | 3.0, (b) (95%) 5 min | Toluol (75 mL) | 0°C bis RT 2.5 h bei RT | MeSO₃H (3.0 Äquiv.) in MeOH | 97.0 : 3.0 | 4.7% | (85.8%) |
| 10 | racemisch 15 mmol | 2.0, (b) (95%) 3 min | Toluol (150 mL) | 0°C bis RT 2.5 h bei RT | MeSO₃H (3.0 Äquiv.) in MeOH | 95.9 : 4.0 | 7.1% | (87.5%) |
| 11 | 30 mmol, davon ca. 10% Mandelat (XVIII), 94.0% ee | 3.0, (b) (95%) 3 min | Toluol (150 mL) | 0°C bis RT 2.25 h bei RT | MeSO₃H (3.0 Äquiv.) in MeOH | 97.1 : 2.8 | 2.8% | (95.8%) |
| 12 | 30 mmol, davon ca. 10% Mandelat (XVIII), 94.0% *ee* | 3.0 , (b) (95%) 2 min | Toluol (150 mL) | 0°C bis RT 2.5 h bei RT | MeSO₃H (3.0 Äquiv.) in MeOH | 96.9 : 3.1 | 6.0% | n.b. |
| 13 | 150 mmol , 100% (XVII) 95.2% *ee* 0.50% H₂O | 3.0, (c) (95%) 2 min | Toluol (750 mL) | 0°C bis RT 2 h bei RT | MeSO₃H (3.0 Äquiv.) in MeOH | 96.5 : 3.4 | 4.2% | (106.2%) (d) (84.2%) (e) |
| 14 | 30 mmol , 100% (XVII) 95.2% *ee* 0.50% H₂O | 3.0, (c) (95%) 1 min | Toluol (150 mL) | +1°C 2.25 h | MeSO₃H (3.0 Äquiv.) in MeOH | 96.7 : 3.2 | 5.3% | (97.3%) |
| 15 | 30 mmol) , 100% (XVII) 95.2% *ee* 0.50% H₂O | 2.0 , (c) (95%) 1 min | Toluol (150 mL) | +1°C 4h | MeSO₃H (2.0 Äquiv.) in MeOH | 96.4 : 3.5 | 11.5% | (97.0%) |
| 16 | 30 mmol , 100% (XVII) 95.2% *ee* 0.50% H₂O | 2.0 , (c) (95%) 0.5 - 1 min | Toluol (150 mL) | 0°C bis RT 2.25 h bei RT | MeSO₃H (2.0 Äquiv.) in MeOH | 95.9 : 4.0 | 8.6% | (96.4%) |
| 17 | 30 mmol , 100% (XVII) 95.2% *ee* 0.50% H₂O | 2.5 , (c) (95%) 0.5 - 1 min | Toluol (150 moL) | 0°C bis RT 2.5 h bei RT | MeSO₃H (2.5 Äquiv.) in MeOH | 96.8 : 3.1 | 3.1% | (98.2%) |
| 18 | 30 mmol , 100% (XVII) 95.2% *ee* 0.50% H₂O | 2.5 , (c) (95%) 0.5 - 1 min | Toluol (150 mL) | 0°C bis RT 2.5 h bei RT | MeSO₃H (2.5 Äquiv.) in MeOH | 96.6 : 3.3 | 4.1% | >65% |
| 19 | 388 mmol , 100% (XVII) 96.2% *ee* 0.32% H₂O | 2.5 , (c) (95%) 0.5 - 1 min | Toluol (1270 mL) THF (86 mL) | 0°C bis RT | MeSO₃H (2.5 Äquiv.) in MeOH bei +20°C | 95.8 : 4.1 | 4.9% | 78% (f) >99.8% rein 100% ee |
| 20 | 50 mmol , 100% (XVII) 92.5% ee 0.43% H₂O | 2.2 , (c) (95%) 0.5 min | Toluol | 0°C bis RT | MeSO₃H (2.2 Äquiv.) in MeOH bei +20°C | 93.9 : 6.0 | 4.7% | 82.0% (f) |
| | | | (160 mL) | 2.25 h bei RT | | | | 99.7% rein |
| | | | | | | | | 100% ee |
| | | | | | | | | 1.5% H₂O |
| 21 | 50 mmol, , 100% (XVII) 92.5% ee 0.43% H₂O | 2.2 , (c) (95%) 0.5 min | Toluol | 0°C bis RT | MeSO₃H (2.2 Äquiv.) in MeOH bei +20°C | 94.0 : 5.9 | 6.8% | 81.4% (f) |
| | | | (250 mL) | 2 h bei RT | | | | 99.4% rein |
| | | | | | | | | 97.6% ee |
| | | | | | | | | 2.0% H₂O |
| 22 | 350 mmol , 100% (XVII) 95.6% ee 0.62% H₂O | (c) (94%) 5 min | Toluol | 0°C bis RT | MeSO₃H (2.2 Äquiv.) in MeOH bei +20°C | 93.5: 6.4 | 9.2% | 81.7% (f) |
| | | | (1120 mL) | 2 h bei RT | | | | 99.8% rein |
| | | | | | | | | 100% *ee* |
| | | | | | | | | 1.5% H₂O |
| 23 | 50 mmol , 100% (XVII) 95.6% ee , 0.36% H₂O inverse Zugabe als Feststoff in 7 min | 2.5, (c) (94%) in Toluol vorgelegt | Toluol | 0°C bis RT | MeSO₃H (2.5 Äquiv.) in MeOH bei +20°C | 94.1 : 5.8 | 4.7% | 80.3% (f) |
| | | | (160 mL) | 1 h bei RT | | | | 99.5% rein |
| | | | | | | | | 100% ee |
| | | | | | | | | 3.4% H₂O |
| 24 | 50 mmol , 100% (XVII) 95.6% ee, O.36% H₂O inverse Zugabe als Feststoff in 30 min | 2.5 , (c) (94%) in Toluol vorgelegt | Toluol | 0°C bis RT | meSO₃H (2.5 Äquiv.) in MeOH bei +20°C | 94.3 : 5.6 | 4.8% | 78.9% (f) |
| | | | (160 mL) | 1 h bei RT | | | | 99.4% rein |
| | | | | | | | | 100% *ee* |
| | | | | | | | | 1.2% H₂O |
| 25 | 150 mmol, 100% (XVII) 95.6% ee, 0.36% H₂O inverse Zugabe als Feststoff in 50 min | 2.5, (c) (94%) in Toluol vorgelegt | Toluol | 0°C bis RT | MeSO₃H (2.5 Äquiv.) in MeOH bei +20°C | 93.9 : 6.0 | 7.0% | 80.1%(f) |
| | | | (480 mL) | 1 h bei RT | | | | 99.8% rein |
| | | | | | | | | 100% ee |
| | | | | | | | | 3.4% H₂O |
| 26 | 49 mmol, 100% (XVII) 95.6% *ee,* 0.36% H₂O inverse Zugabe als Feststoff in 20 min | 2.5, (c) (94%) in Toluol vorgelegt | Toluol | 0°C bis RT | MeSO₃H (1.5 Äquiv.) in MeOH bei 20°C bis 40°C | 94.4 : 5.5 | 4.4% | 81.4% (f) |
| | | | (160 mL) | 1.25 h bei RT | | | | 99.8% rein |
| | | | | | | | | 100% ee |
| | | | | | | | | 3.63% H₂O |
| 27 | 50 mmol , 100% (XVII) 95.6% *ee*, 0.36% H₂O inverse Zugabe als Feststoff in 17 min | 2.5, (c) (94%) in Toluol vorgelegt | Toluol (160 mL) | 0°C bis RT | meSO₃H | 94.2 : 5.8 | 7.6% | 74.5% (f) |
| | | | | 1.25 h bei RT | (1.5 Äquiv.) | | | 99.8% rein |
| | | | | | in MeOH bei | | | 100% *ee* |
| | | | | | 20°C bis 40°C | | | 3.5% H₂O |
| 28 | 50 mmol , 100% (XVII) 95.6% ee, 0.36% H₂O | 2.5 , (c) (94%) 25 min 25min | Toluol (160 mL) | 0°C bis RT | MeSO₃H (1.5 Äquiv.) in MeOH bei 20°C bis 40°C | 94.3 : 5.6 | 6.4% | 75.9% (f) |
| | | | | 1.25 h bei RT | | | | 99.9% rein |
| | | | | | | | | 100% ee |
| | | | | | | | | 1.2% H₂O |
| 29 | 50.39 mmol , | 2.5, (c) | Toluol | 0°C bis RT | MeSO₃H | 94.3 : 5.6 | 6.1% | 80.3% (f) |
| | 100% (XVII) | (94%) | (160 mL) | 1.5 h bei RT | (2.0 Äquiv.) | | | 99.8% rein |
| | 95.6% ee , | 25 min | | | in MeOH bei | | | 100% ee |
| | 0.36% H₂O | | | | 20°C bis 40°C | | | 3.6% H₂O |
| 30 | 200 mmol , | 2.5 , (c) | Toluol | 0°C bis RT | (g) | 92.7 : 7.2 | 18.0% | 60.1% (f) |
| | >99% (XVII) | (94%) | (640 mL) | 1 h bei RT | MeSO₃H | | | 99.1 % rein |
| | 93.4% *ee ,* | 25 min | | | (2.0 Äquiv.) | | | 100% *ee* |
| | 0.02% H₂O Durchführung im RC1-Kalorimeter | | | | in MeOH bei 20°C bis 40°C | | | 3.6% H₂O |
| 31 | 198.2 mmol, , | 2.5 , (c) | Toluol | 0°C bis RT | (h) | 93.5 : 6.4 | 11.4% | 74.2% (f) |
| | >99%(XVII) | (94%) | (640 mL) | 1.25 h bei RT | MeSO₃H | | | 99.2% rein |
| | 93.4% *ee* , | 22 min | | | (2.5 Äquiv.) | | | 100% *ee* |
| | 0.02% H₂O | | | | in MeOH bei 20°C bis 40°C | | | 1.3% H₂O |
| 32 | 200.0 mmol , | 2.5 , (c) | Toluol | 0°C bis RT | (h) | 94.1 : 5.9 | 11.0% | 76.8% (f) |
| | >99.5% (XVII) _{.} | (94%) | (640 ml) | 2.5 h bei RT ' | MeSO₃H | | | 99.7% rein |
| | 90.5% ee | 15 min | | | (2.5 Aquiv.) | | | 99.4% ee |
| | | | | | in MeOH bei 15°C bis 22°C | | | 4.3% H₂O |
| 33 . | 150.0 mmol, | 2.6 , (c) . | Toluol | 1 ° bis 20°C | (i) | 94.4 : 5.6 | 11.6% | 83.7% (f) |
| | >99% (XVII) | (94%) | (400 ml) | 4h bei 20°C | MeSO₃H | | | 99.7% rein |
| | 93.4% ee, | 15 min | | | (3.2 Äquiv.) | | | 100% ee |
| | 0.02% H₂O | | | | in MeOH bei 15°C bis 22°C | | | 1.3% H₂O |
| 34 | 15.0 mmol, | Me₃SiCl | THF | Umsetz. von | MeSO₃H | 94.1 : 5.9 | 8.5% unumgesetztes (XVII) und Retro-Mannich-Prod., | 74.8% (f) |
| | >99% (XVII) | 3.00 | (215 ml) | Me₃SiCl mit | (3.4 Äquiv.) | | | 99.6% rein |
| | 93.4% ee, | NaBH₄ | | NaBH₄ 50°/45 | in MeOH | | | 100% ee |
| | 0.02% H₂O | 3.00 | | min; Add. (XVII) | | | | |
| | | | | bei 2°C; innerh 15min auf 20° erwärmt und 2h bei 20° gerührt | | | 5.4% dia-(XIX); Summe 14.9% Verunreinig. | |
| 35 | 150.0 mmol, | 2.6 , (c) | Toluol | Umsetz. von BMS mit kat. | MeSO₃H | 94.3 : 5.7 | 2.4% | 75.9% |
| | >99% (XVII) | (94%), | (400 ml) | | | | | 99.5% rein |
| | 93.4% ee, | 0.1 Äquiv. (XIX) | | (XIX) in Tol. (40 ml) bei 0-20°C / 1h. Zugabe v. | (3.2 Äquiv.) | | | 100% ee |
| | 0.02% H₂O | | | | in MeOH | | | 3.1% H₂O |
| | | | | Susp. (XVII) in Tol. (360ml) bei 2°C/15 min. 3h bei 20° gerührt | | | | |
| 36 | 75.0 mmol, | 2.6 , (c) | THF | Umsetz. von BMS mit kat. | MeSO₃H | 94.3 : 5.7 | 11.8% | 67.7% |
| | >99% (XVII) | (94%) | (180 ml) | | | | | 99.2% rein |
| | 93.4% ee, | 0.1 Äquiv. (X1X) | Toluol | (XIX) in Tol. (20 ml) bei 20°C/1h. | (3.2 Äquiv.) | | | 99.3% ee |
| | 0.02% H₂O | | (20 ml) | | in MeOH | | | 3.9% H₂O |
| | | | | Zugabe von THF (180 ml). Bei 45° Zugabe v. (XVII) in 30 min, dann bei 45°C 12.h gerührt | | | | |

| | | | | | | | | |
|---|---|---|---|---|---|---|---|---|
| Anmerkungen: (a) Bei Rkt. Nr. 5-36: HPLC-Analyse des ausreagierten Reaktionsgemischs nach saurer Solvolyse des intermediären Oxazaborinans. Bei Rkt. Nr. 1-4: HPLC-Analyse des Hydroborierungs-Gemischs. (b) Die eingesetzte Menge Boran-Komplex wurde volumetrisch bestimmt (Zugabe per graduierter. Spritze). (c) Die eingesetzte Menge Boran-Komplex wurde per Einwaage bestimmt. (d) Ausbeute nach Ausfällung aus 2 N NaOH, Filtration und Trocknung. (e) Ausbeute nach einer erneuten Waschung in E-Wässer, Filtration und Trocknung. (f) Nach Ausfällung in 2 N NaOH wurde (XIX) direkt bei 45-50°C in n-Butanol gelöst und mit 2.2 Äquiv. 30%-iger Salzsäure sein Dihydrochlorid gefällt. (g) Die Extraktion von (XIX) mit 2 N HCl wurde durch eine zusätzliche Zugabe von 1.4 Äquiv. MeSO₃H und Extraktion mit Wasser ersetzt. (h) Die Extraktion von (XIX) mit 2N HCl wurde durch eine zusätzliche Zugabe von 0.7 Äquiv. MeSO₃H und Extraktion mit Wasser ersetzt. (i) Aufgrund der sofortigen Verwendung von 3.2 Äquiv. anstatt 2.5 Äquiv. MeSO₃H erübrigte sich die Zugabe von 2 N HCl bzw. zusätzlicher MeSO₃H zur Extraktion von (XIX) aus der Toluol- in die Wasserphase. | | | | | | | | |

Die Resultate zeigen, daß sich gemäß dieser Methode die Carbonylgruppe mit hoher Stereoselektivität (bis >97: <3 ; siehe Nr. 9 und 11) reduzieren läßt und die Retro-Mannich-Reaktion des Edukts (I) unter den Reaktionsbedingungen weitestgehend zurückgedrängt ist, so daß die im Edukt bereits enthaltene stereochemische Information praktisch vollständig erhalten bleibt.

Eine ansich bekannte Aufarbeitungsmethode für Reduktionen mit Boran- oder Borhydrid-Reagenzien ist beispielsweise die solvolytische Spaltung und/oder eine Kristallisation.

Die solvolytische Spaltung des bei der Reduktion von (I) primär gebildeten Oxazaborinans (C) zum 1,3-Aminoalkohol (II), sowie dessen Isolierung aus dem Reaktionsgemisch führt zu einer größtmöglichen Entfernung von Stereoisomeren: dem Enantiomer ent-(II), 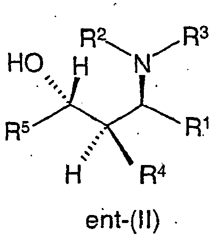 dem Diastereomer dia-(II) 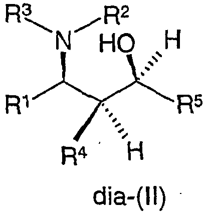 sowie dem Enantiomer des Diastereomers ent-dia-(II)

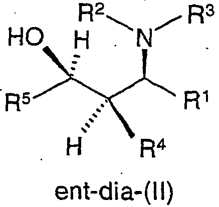

Optional kann in der Aufarbeitung der Reaktionslösung des Produktes der Formel (II) eine in hohen Ausbeuten verlaufende Kristallisation durchgeführt werden, die die in der rohen Reaktionslösung in geringen Mengen enthaltenen Stereoisomeren von (II) vollständig entfernt. Auf diese Weise gelang es, 1,3-Aminoalkohole der Formel (II) in sehr hoher Reinheit (>99.5% chem. Reinheit, ~100% de, >99% ee) in 2-3 Stufen aus meistens kommerziell erhältlichen Ausgangsstoffen herzustellen, wobei hohe Gesamtausbeuten, teilweise von über 70% der Theorie, erzielt wurden.

Die solvolytische Spaltung kann mit einer Reihe unterschiedlicher Prozeduren erreicht werden:
a) Bevorzugt wird die Spaltung mit 1 -4 Äquivalenten einer starken Säure, besonders bevorzugt Methansulfonsäure oder Schwefelsäure, in einem Überschuß eines niedrig-molekularen Alkohols, besonders bevorzugt Methanol, bei 0 - 60°C, besonders bevorzugt bei 15 - 40°C, durchgeführt (Tabelle 8, Nr. 6 - 36). Unter diesen Bedingungen geht das Bor von (C) in einen flüchtigen Borsäuretrialkylester, im besonders bevorzugten Fall in den leichtflüchtigen Borsäüretrimethylester B(OCH₃)₃ über, der mit Methanol ein Azeotrop MeOH B(OMe)₃ vom Siedepunkt 59°C bildet, das ca. 70% B(OMe)₃ im azeotropen Gemisch enthält (M. Couturier et al, Tetrahedron Lett. 2001, 42, 2285). Insbesondere wenn die Boran-Reduktion in besonders bevorzugten Lösungsmitteln wie Toluol oder Cumol durchgeführt wurde, kann man nach vollständiger Solvolyse durch Anlegen von Vakuum das Borsäureester-Solvat und überschüssiges Methanol leicht quantitativ abdestillieren. Der 1,3-Aminoalkohol der allgemeinen Formel (II) liegt in protonierter Form vor und hat darum in der Regel eine gute Wasserlöslichkeit. Setzt man dem toluolischen bzw. cumolischen Destillationsrückstand darum Wasser zu, so wird das Salz von (II) in den meisten Fällen praktisch quantitativ in die wäßrige Phase extrahiert. Mit der toluolischen bzw. cumolischen Phase entfernt man die meisten Reaktionsnebenprodukte, z.B. Retro-Mannich-Produkte und deren Reduktionsprodukte. Stellt man die produkthaltige, saure wäßrige Lösung dann. stark basisch, z.B. mit wäßriger Natronlauge, fällt der freie 1,3-Aminoalkohol (II) aus und kann leicht isoliert werden. Besonders bevorzugt ist aber, (II) durch eine Kristallisation eines seiner Salze zu isolieren, bei der die im Rohprodukt in geringen Mengen enthaltenen Stereoisomeren in der Mutterlauge verbleiben. Das optimale Anion und Lösungsmittel für eine derartige Kristallisation hängen von der Natur der Substituenten R¹ bis R⁵ in (II) ab und müssen deshalb für jeden 1,3-Aminoalkohol der Formel (II) unabhängig ermittelt werden. Für R¹ = o-Nitrophenyl, R² = 2-Pyridyl, R³ = H, R⁴ = 2-Pyridyl, R⁵ = Phenyl erwies sich z.B. die Kristallisation des Dihydrochlorids von (II) aus 1-Butanol als optimal. Man erhielt das Dihydrochlorid mit 99.3 - 100% ee und einer chemischen Reinheit von 99.1 - 99.9% in einer Ausbeute von 74 - 84 % der Theorie bezogen auf eingesetzte Mannich-Base (I) (Tabelle 7, Nr. 19-29, 31-35). Diese Kristallisation kann selbst eine untypisch niedrige Enantiomerenreinheit der eingesetzten Mannich-Base (I) kompensieren. Im Versuch gemäß Tab. 7, Nr. 32 wurde (I) von nur 90.5 % ee eingesetzt. Trotzdem wurde (II)-Dihydrochlorid in 76.8 % Ausbeute mit 99.4 % ee und 99.7 % chemischer Reinheit isoliert.
b) Alternativ kann die Solvolyse von (C) auch mit einem Überschuß einer starken wäßrigen Säure, bevorzugt 2-normaler bis konzentrierter Salzsäure oder wäßriger Methansulfonsäure bei 0 -100°C, bevorzugt bei 0 - 40°C nach vorherigem Abdestillieren des organischen Lösungsmittels der Boran-Reduktion, durchgeführt werden. Diese Aufarbeitung kam in Tabelle 9 (Nr. 4-7, 9-14 und 16-18) und in Tabelle 10 (Nr. 5-8) zur Anwendung. Unter diesen Bedingungen wandelt sich das Bor von (C) in Borsäure B(OH)₃ um, die im sauren wäßrigen Reaktionsgemisch, insbesondere bei Kühlung auf 0-10°C nur wenig löslich ist, weitestgehend auskristallisiert und deshalb leicht entfernt werden kann. Der 1,3-Aminoalkohol liegt hingegen protoniert vor und ist deshalb in der Regel gut wasserlöslich. Stellt man die produkthaltige, saure wäßrige Lösung nach dem Entfernen der Borsäure stark basisch, z.B. mit wäßriger Natronlauge, fällt der freie 1,3-Aminoalkohol (II) aus und kann leicht isoliert werden. Entsprechende typische Prozeduren sind in Beispielen 23 (entsprechend Tab.9, Nr. 18) und 24 (entsprechend Tab.10, Nr. 5) beschrieben. Bevorzugt ist aber, wie bei a), (II) durch eine Kristallisation eines seiner Salze zu isolieren. Man erreicht dies, indem man das Basischstellen der sauren, wäßrigen produkthaltigen Lösung in Gegenwart eines geeigneten organischen, mit Wasser nicht mischbaren, Lösungsmittels, z.B. n-Butanol, durchführt. Der freie 1,3-Aminoalkohol (II) wird dabei praktisch quantitativ in diese organische Phase extrahiert, die man anschließend erwärmt und durch Zugabe einer geeigneten wäßrigen Säure, z.B. konzentrierter Salzsäure, ein Salz von (II) bildet, das beim langsamen Abkühlen der butanolischen Lösung auskristallisiert.
c) Eine weitere alternative Solvolysemethode für (C) ist die Zugabe eines Überschusses der Lösung eines Alkali- oder Erdalkafihydroxids, gefolgt vom Erwärmen auf 30 -100°C, bevorzugt auf 50 - 70°C. Der freie 1,3-Aminoalkohol (II) kann dann mit einem inerten organischen Lösungsmittel extrahiert werden, während das entstandene Alkali- oder Erdalkali-Borat in der wäßrigen Phase verbleibt. Eine entsprechende typische Prozedur ist in Beispiel 25 (entsprechend Tabelle 10, Nr. 3) beschrieben.
d) Eine weitere alternative Solvolysemethode für (C) ist der Zusatz eines organischen Komplexbildners (z.B. Diethylentriamin), der mit dem Bor einen starken Chelatkomplex bildet. Diese Methode wird bevorzugt in folgender Form angewandt: Im Solvolysereaktor wird unter Inertgasatmosphäre Methanol bei 20-60°C, bevorzugt bei 40-50°C, vorgelegt. Das 20-60°C, bevorzugt 40-50°C warme, bevorzugt toluolische, Reduktionsgemisch (im Wesentlichen (C) und überschüssiges Boran enthaltend) wird langsam in das vorgelegte Methanol eindosiert. Nach beendeter Dosierung wird der Komplexbildner, z.B. Diethylentriamin, eindosiert und das Solvolysegemisch gerührt, bis die Solvolyse von (C) unter Bildung von (II) quantitativ ist. Anschließend läßt man zu dem Reaktionsgemisch, bevorzugt bei 60-70°C, Wasser zulaufen. Die organische (toluolische) Phase wird dann, von der wäßrigen Phase getrennt und, bevorzugt bei 60-70°C, mit Wasser gewaschen. Das Bor-Amin-Chelat und überschüssiges Methanol werden mit der Wasserphase entfernt. Aus der toluolischen Phase kann man den Aminoalkohol der Formel (II) nach bekannten Verfahren isolieren. Vorteilhaft kann dabei, in Abhängigkeit von der spezifischen Natur der Substituenten R¹ bis R⁵ sowohl die direkte Kristallisation durch langsames Abkühlen der warmen, konzentrierten toluolischen Lösung sein. Es kann aber auch die Überführung von (II)_{,} wie unter a) beschrieben, in ein anderes polareres Lösungsmittel, z.B. n-Butanol, gefolgt von der Kristallisation eines geeigneten Salzes von (II), z.B. eines Hydrochlorids, vorteilhaft sein.
e) Eine weitere alternative Spaltungsmethode für (C) unter Bildung des 1,3-Aminoalkohols (II) ist die solvolytische Spaltung bei Zusatz von Wasserstoffperoxid-Lösung. Diese Methode ist nur für solche Produkte (II) vorteilhaft, die von Wasserstoffperoxid nicht leicht oxidiert werden. Da außerdem die Reaktion von Boranen und einigen Oxazaborinanen der Formel (II) mit Wasserstoffperoxid extrem exotherm sein kann, sind die Aufarbeitungsmethoden a) und d) häufig gegenüber e) bevorzugt.

Verfahrensschritt 4 wird mit den gleichen Reduktionsmitteln und unter den gleichen Reaktionsbedingungen (Mol-Äquivalente Reduktionsmittel, verwendbare Lösungsmittel, Reaktionstemperatur und -dauer, Zugabemodus) und Aufarbeitungsmethoden durchgeführt, die zuvor für Verfahrensschritt 3 beschrieben wurden.

Für Verfahrensschritt 4 gelten folgende Besonderheiten:
- Mannich-Salze der Formel (III) sind in aller Regel deutlich polarer als die freien Mannich-Basen der Formel (I). Die Löslichkeit der Mannich-Salze (III) in unpolaren Lösungsmitteln (Toluol oder unpolarer) ist darum in den meisten Fällen nicht mehr ausreichend für eine sinnvolle Reaktionsgeschwindigkeit mit dem Reduktionsmittel. Bevorzugte Lösungsmittel für die Reduktion der Mannich-Salze (III) sind darum polarere Lösungsmittel, in denen (III) besser löslich ist, besonders bevorzugt ist Tetrahydrofuran.
- Bei besonders bevorzugten Gegenionen Y*⁻ in den Mannich-Salzen (III) handelt es sich um chirale Carboxylate oder Dicarboxylate. Diese Gegenionen Y*⁻ sind in der Regel nicht völlig inert gegenüber Boranen, Boran-Komplexen oder aktivierten Borhydriden und werden von den eingesetzten Reduktionsmitteln selbst langsam reduziert. Diesem Verbrauch muß durch eine entsprechende Anhebung der Äquivalente Reduktionsmittel Rechnung getragen werden.

Wie für Verfahrensschritt 3 beschrieben entsteht als Reduktionsprimärprodukt ein Oxazaborinan (C), das anschliessend gemäß einer der oben beschriebenen Solvolyse- / Aufarbeitungs-Prozeduren zum gewünschten 1,3-Aminoalkohol der Formel (II) umgewandelt wird.

In Tabelle 9 sind beispielhaft die Ergebnisse von diastereoselektiven Carbonyl-Reduktionen des (S)-(+)-Mandelat-Salzes (XVIII) (Verbindung der Formel (III), wobei R¹ = o-Nitrophenyl, R² = 2-Pyridyl, R³ = H, R⁴ = 2-Pyridyl und R⁵ = Ph sind) zum 1,3-Aminoalkohol (XIX) zusammengestellt:

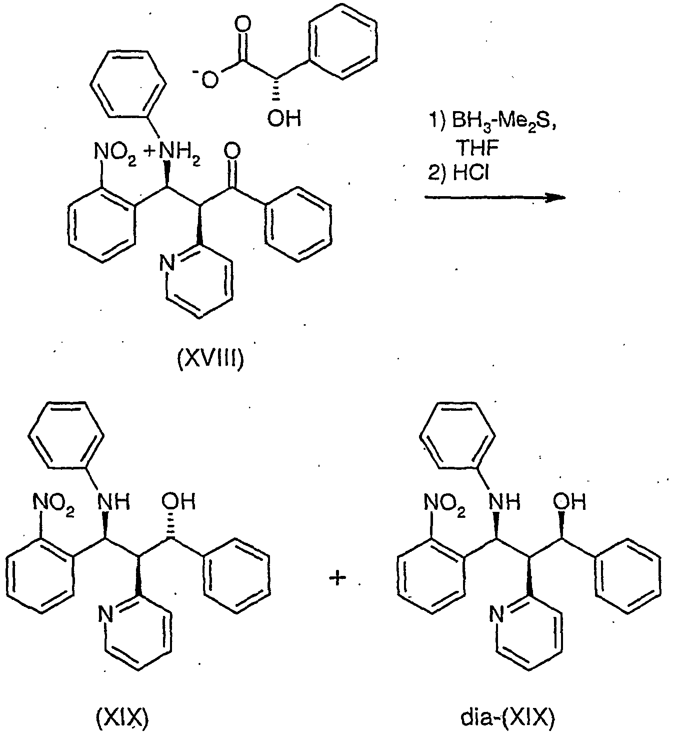

| Tabelle 9: | | | | | | | | |
|---|---|---|---|---|---|---|---|---|
| Nr. | Edukt mmol optische Reinh. | BH₃-Me₂S (Äquiv.) Zugabezeit | Lösungsm. | Rührtemperatur und -zeit | Aufarbeitung | Verhältnis (XIX) / dia-(XIX) (HPLC v. Rkts.gemisch) | Summe der Verunreinigungen (HPLC v. Rkts.qemisch) | Ausbeute % der Theorie (isoliertes Rohprod.) isoliertes Reinprod. |
| 1 | 2 mmol | 5.0 (a) | Toluol | +1°C | 2 N HCl | | Unsaubere Reaktion, Viel nicht umgesetztes Edukt | Ansatz |
| | 95.1 % ee | 5 min | (20 mL) | 4h | (40 Äquiv.) | | | verworfen |
| 2 | 2 mmol) | 4.0 (a) | THF | +1°C | 2 N HCl | 95.4 : 4.5 | 2.9% | n.b. |
| | 95.1 % ee | 15 min | (20 mL) | 1.25 h | (40 Äquiv.) | | | |
| 3 | 2 mmol | 4.0 (a) | THF | 0°C bis RT | 2 N HCl | 95.8 : 4.1 | 1.9% | n.b. |
| | 95.1 % ee | 5 min | (20 mL) | 1 h bei RT | (40 Äquiv.) | | | |
| 4 | 2 mmol | 5.0 (a) | THF | 0°C bis RT | konz. HCl | 96.7 : 3.2 | 2.7% | (>100%) |
| | 95.1 % ee | 0.5 min | (20 mL) | 0.5 h bei | (10 mL) | | | |
| | | | | RT | | | | |
| 5 | 15.4 mmol | 5.0 (a) | THF | +1°C | halbkonz. | 93.7 : 6.2 | 11.3% | (> 100%) |
| | 95.1 % ee | 30 min | (90 mL) | 1 h | HCl | | | |
| | | | | | (60 mL) | | | |
| 6 | 15.7 mmol | 5.0 (a) | THF | 0°C bis RT | halbkonz. | 95.8 : 4.1 | 4.4% | (>100%) |
| | 96.5% ee | 10 min | (95 mL) | 3.5 h bei | HCl | | | |
| | | | | RT | (60 mL) | | | |
| 7 | 13.9 mmol | 3.0 (a) | THF | RT | verd. HCl | 95:5 | >20% | Ansatz verworfen |
| | 96.5% ee | 10 min | (85 mL) | 2.5 h | (70 mL) | | Zersetzungs- | |
| | | bei RT | | | | | | |
| | | (exotherm!) | | | | | produkte des Edukts! | |
| 8 | 13.9 mmol 96.5% ee | 3.0 (a) 15 min | THF (85 mL) | 0°C bis RT 1.25 h bei RT | MeSO₃H (3.0 Äquiv.) in MeOH | 95.5 : 4.4 | 2.6% | (79.8%) |

| Nr. | Edukt mmol optische Reinh. | BH₃-Me₂S (Äquiv.) Zugabezeit | Lösungsm. | Rührtemperatur und -zeit | Aufarbeitung | Verhältnis (XIX) / dia-(XIX) (HPLC v. Rkts.gemisch) | Summe der Verunreinigungen (HPLC v. Rkts.gemisch) | Ausbeute % der Theorie (isoliertes Rohprod.) isoliertes Reinprod. |
|---|---|---|---|---|---|---|---|---|
| 9 | 13.9 mmol | 3.0 (a) | THF | 0°C bis RT | 30%-ige HCl | 95.5 : 4.3 | 5.6% | 83.2% |
| | 96.5% ee | 10 min | (85 mL) | 1.75 h bei | (50 mL) | | | (4% dia-(XIX) und |
| | | | | RT | | | | 5% Nebenprodukt) |
| 10 | 13.9 mmol | 3.0 (a) | THF | 0°C bis RT | 30%-ige HCl | 95.6 : 4.4 | 5.1 % | (97%) |
| | 96.5% ee | 10 min | (85 mL) | 2.5h bei RT | (50 mL) | | | |
| 11 | 13.9 mol | 3.0 (a) | THF | 0°C bis RT | 2 N HCl | 96:4 | 4.1 % | n.b. |
| | 96.5% ee | 7 min | (85 mL) | 2 h bei RT | (63 mL) | | | klebriger Feststoff |
| 12 | 24 mmol | 4.0 (a) | THF | 0°C bis RT | halbkonz. | 96.1 : 3.9 . | 3.8% | (100%) |
| | 96.5% ee | 10 min | (60 mL) | 3 h bei RT | HCl | | | |
| | | | | | (22.5 mL) | | | |
| 13 | 24 mmol | 4.0 (a) | THF | 0°C bis RT | konz. HCl | 96.4 : 3.6 | 3.1 % | (100.8%) |
| | 96.5% ee | 10 min | (60 mL) | 2 h bei RT | (30 mL) | | | |
| 14 | 74.8 mmol | 4.0 (a) | THF | 0°C bis RT | konz. HCl | 96.1 : 3.9 | 6.0% | (> 100%) |
| | 96.5% ee | 10min | (750 mL) | 2 h bei RT | (375 mL) | | | |
| 15 | 11 mmol | 4.0 (a) | THF | 0°C bis RT | MeSO₃H | 96.5 : 3.5 | 4.1 % | (>100%) |
| | 96.5% ee | 10 min | (85 mL) | 2 h bei RT | (4.0 Äquiv.) | | | |
| | | | | | in MeOH | | | |
| 16 | 15 mmol | 3.0 (a) | THF | 0°C bis RT | konz. HCl | 96.2 : 3.8 | 3.8% | n.b. |
| | 96.5% ee | 10 min | (85 mL) | 1.5h bei RT | (55 mL) | | | klebriger Feststoff. |
| 17 | 11 mmol | 4.0 (a) | THF | 0°C bis RT | konz. HCl | 96.3 : 3.7 | 4.7% | (> 100%) |
| | 96.5% ee | 10 min | (85 mL) | 1.5 h bei | (50 mL) | | | |
| | | | | RT | | | | |
| 18 | 52 mmol | 3.0 (a) | THF | 0°C bis RT | konz. HCl | 96.8 : 3.2 | 3.5% | (93.4) ; 78.7% (b) |
| | 96.5% ee | 10 min | (400 mL) | 1.5 h bei | (200 mL), | | | (3.1% dia-(XIX) |
| | | | | RT | Aufschlämmung in (i-Pr.)O | | | 1.8% Nebenprodukte enthaltend) |

| | | | | | | | | |
|---|---|---|---|---|---|---|---|---|
| Anmerkungen: (a) Die eingesetzte Menge Boran-Dimethylsuilfid-Komplex wurde aus dem eingesetzten Volumen des Reagenzes berechnet. (b) Isoliertes Produkt der Formel (XIX) nach Aufschlämmung in Diisopropylether. | | | | | | | | |

Quantitative Umsätze der Mannich-Base - Komponente des Salzes (XVIII) wurden bis herab zu 3.0 Äquivalenten Reduktionsmittel erzielt (Tabelle 9, Nr. 8-11, 16 und 18). Während in Toluol kaum Umsatz zu (XIX) erzielt wurde (Tabelle 9, Nr. 1), erfolgte in THF weitgehender bis vollständiger Umsatz (Tab. 9, Nr. 2-18). Bei der Carbonyl-Reduktion wurden Diastereoselektiven (Verhältnis (XIX) / dia-(XIX)) bis 96.8 : 3.2 erzielt (Tabelle 9, Nr. 4 und 18). Die isolierten Ausbeuten an (XIX) betrugen 78-83% derTheorie, wobei diese Produkte 3-4% des Diastereomeren dia-(XIX) und knapp 2% des Enantiomeren von (XIX) enthielten (Tabelle 9; Nr. 9 und 18), da hier kein Kristallisationsschritt des Dihydrochlorids von (XIX) analog Verfahrensschritt 3, Abschnitt a) erfolgte. Inklusive der Kristallisation des Dihydrochlorids erhielt man den enantiomeren- und diastereomerenreinen Aminoalkohol (XIX) (>99% ee, >99% de, >99% chem. Reinheit) mit einer Ausbeute von 70-75% der Theorie bezogen auf eingesetztes Mandelats (XVIII).

In Tabelle 10 sind beispielhaft die Ergebnisse von diastereoselektiven Carbonyl-Reduktionen des (+)-Dipivaloyl-weinsäure-Salzes (XII) (Verbindung der allgemeinen Formel (III), wobei R¹ = o-Nitrophenyl, R² = 2-Pyridyl, R³ = H, R⁴ = 2-Pyridyl, R⁵ = Ph, HY* = (+)-Dipivaloyl-weinsäure) zum 1,3-Aminoalkohol (XIX) zusammengestellt:

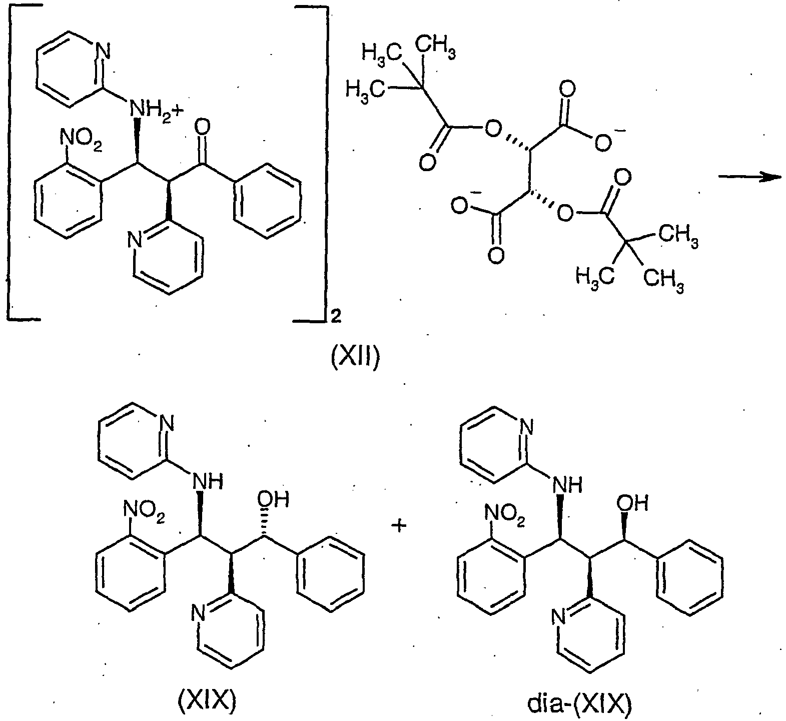

| Tabelle 10 | | | | | | | | | |
|---|---|---|---|---|---|---|---|---|---|
| Nr. | Edukt (XII); darin enthaltene mmol freie Mannich-Base; optische Reinheit | Reduktionsmittel; Reagenzien; (Mol-Äquiv bez. auf freie Mannich-Base) | Lösungsmittel | Temperatur, Zeit | Aufarbeitung | Verhältnis (XIX) / dia-(XIX) (HPLC); (Reaktionsgemisch) isoliertes Produkt | HPLC bzw. HPLC/MS (Reaktionsgemisch) isoliertes Produkt | Auswaage (Gehalt laut HPLC-Assay bezogen auf Standard) | Ausbeute % der Theorie Assay-korrigiert |
| 1 | 15.0 g | NaBH₄ | 200 ml | 30-35 °C | 50 ml Aceton; | | [<5% (XIX); hauptsächl. Nebenprodukt mit Masse 350] | | |
| | (XII) | (4.0 Äquiv) . | Butanol / | 20 h | Ansatz | | | | |
| | 21.58 | | Wasser | | verworfen | | | | |
| | mmol freie Mannich-Base | | 82 : 18 (v/v) | | | | | | |
| | 74.0 % ee | | | | | | | | |
| 2 | 5.0 g (XII) | NaBH₄ | 25 ml | Raumtemp. | 4.5 ml Aceton | (65/35) | 53% (XIX), 38% | 1.48 g | (44.0 %) |
| | 7.92 mmol | (3.0 Äquiv) | Ethanol | 3 h | | 58/42 | dia-(XIX), 5% | | |
| | freie Mannich-Base | BzEt₃N⁺Cl⁻ (0.1 Äquiv) | | | | | Nebenprod. mit Masse 436 und 3% mit Masse | | |
| | 74.0 % ee | | | | | | 426 | | |
| 3 | 10.0 g | BH₃-Me₂S | 100 ml THF | Zugabe: | 20%ige wäßr. | 97.6 / 2.4 | 93.0 % (XIX) | 7.05 g | 79.3 % |
| | (XII) | (5 Äquiv) | | 3°C / 15 min | KOH, | | (95.2% ee), | (77.2 %) | |
| | 16.08 | | | Reaktion: | dann 60°C / 20 h | | 1.5% dia-(XIX), | | |
| | mmol freie | | | 0°C nach RT / | | | 5.5 % . | | |
| | Mannich-Base | | | 30 min | | | Oxazaborinan (C) | | |
| | 95.1 % ee | | | | | | | | |
| 4 | 5.0 g (XII) | BH₃-Me₂S | 50 ml MtBE | Zugabe: 0-5°C ; 5 Min | keine Reaktion | | | | |
| | 8.0 mmol | (5 Äquiv) | | | Ansatz | | | | |
| | freie Mannich-Base | | | Reaktion : RT ; 2.5 h | verworfen | | | | |
| | 95.1 % ee | | | | | | | | |
| 5 | 10.0 g | BH₃-Me₂S | 100 ml THF | Zugabe: 0-5°C ; 15 Min | 45 ml H₂O und 10ml HCl | 97.8 / 2.2 | 94.8 % (XIX) | 8.11 g | 88.8 % |
| | (XII) | (5 Äquiv) | | | | | (96.8% ee) | (75.1 %) | |
| | 16.08 mmol freie Mannich-Base | | | Reaktion : 0°C nach RT / 20 Min | (37%ig), dann 60°C / 15min; mit 30 ml NaOH (33%ig) auf pH 13, CH₂Cl₂-Extr., eingeengt, | | | | |
| | 95.1 % ee | | | | | | | | |
| 6 | 15.0 g | BH₃-Me₂S | 150 ml THF | Zugabe: 0-5°C ; 15 Min | 50 ml H₂O und 15 ml HCl (37%ig), dann 60°C / 15min; | 97.5 / 2.5 | 96.2 % (XIX) | 10.43 g | 84.0% |
| | (XII) | (5 Äquiv) | | | | | (96.6% ee) | (82.9 %) . | |
| | 24.12 | | | Reaktion : 0°C auf RT / 20 | | | | | |
| | mmol freie Mannich-Base | | | | | | | | |
| | | | | Min | mit 35 ml NaOH (33%ig) auf pH 13-14; CH₂Cl₂-Extr., eingeengt | | | | |
| | 95.1 % ee | | | | | | | | |
| | | | | | | | | | |
| 7 | 15.0 g | BH₃-Me₂S | 150 ml THF | Zugabe : | 50 ml H₂O und | >97.2 / <2.8 | 92.1 % (XIX) | 12. 18 g | 87.3 % |
| | (XII) | (4 Äquiv) | | 0-5°C ; 15 Min | 15 ml HCl | | (93.1% ee) | (77.4 %) | |
| | 25.29 mmol freie Mannich-Base | | | Reaktion : 0°C auf RT / 20 Min | (37%ig), dann 60°C / 30 min; 15 g Oxone bei RT zugegeben und 30 min RT; mit 35 ml NaOH (33%ig) auf pH 13-14; CH₂Cl₂-Extr., eingeengt | | | | |
| | 92.5 % ee | | | | | | | | |
| 8 | 4.4 g (XII) | BH₃-Me₂S | 44 ml THF | Zugabe: | 15 ml H₂O und | 97.8/2.2 | 92.8 % (XIX) | 2.9 g | (89.8 %) |
| | 7.43 mmol | (3.18 Äquiv) | | 0-5°C ; 5 Min | ml HCl (37%ig), | | (91.8% ee), | | |
| | freie Mannich-Base | | | Reaktion: 0°C auf RT / 1.5 h | dann 40°C / 3h, mit 5 ml NaOH (30%ig) auf pH 11; CH₂Cl₂-Extraktion, eingeengt; mit 50 ml iPr₂O bei 0°C kristallisiert; HV | | 2.1% dia-(XIX), 1.6% Oxazaborinan (C), 3.5% Nebenprodukte | | |
| | 92.5 % ee | | | | | | | | |

| Nr. | Edukt (XII); darin enthaltene mmol freie Mannich-Base; optische Reinheit | Reduktionsmittel; Reagenzien; (Mol-Äquiv bez. auf freie Mannich-Base) | Lösungsmittel | Temperatur, Zeit | Aufarbeitung | Verhältnis (XIX) / dia-(XIX) (HPLC); (Reaktionsgemisch) isoliertes Produkt | HPLC bzw. HPLC/MS (Reaktiongemisch) isoliertes Produkt | Auswaage (Gehalt laut HPLC-Assay bezogen auf Standard) | Ausbeute % der Theorie Assay-korrigiert |
|---|---|---|---|---|---|---|---|---|---|
| 9 | 15.3 g | BH₃-Me₂S | 125 ml THF | Zugabe: 0-5°C ; 5 Min | Bei 5°C 20.9 g | (98.1 / 1.9) | 99.2% (XIX) | 9.46 g | 86.2% |
| | (XII) | (2.5 Äquiv) | | | MeOH gefolgt von 4.92 g MeSO₃H, 35°C / 6h; THF/ MeOH im Vakuum abdestilliert; plus 75 ml H₂O und 10 ml 25% NH₄OH, 1h 25°C ; Feststoff abgesaugt; digeriert in 66 ml iPr₂O, abgesaugt; HV-getrocknet | 99.2/0.8 | (95.2% ee), | (97.9%) | |
| | 25.3 mmol freie Mannich-Base | | | Reaktion: 0°C auf 25°C / 2 h | | | 0.8% dia-(XIX) | | |
| | 93.2 % ee | | | | | | | | |

Mit Natriumborhydrid in Butanol / Wasser entstand nur wenig gewünschtes Produkt (XIX) (Tabelle 10, Nr. 1). Mit Natriumborhydrid in Ethanol in Gegenwart katalytischer Mengen eines quarternären Ammoniumsalzes war der Umsatz zwar besser, aber die Diastereoselektivität nur sehr gering (Tabelle 10, Nr. 2). Mit dem Reduktionsmittel Boran-Dimethylsulfid-Komplex wurden in THF hervorragende Umsätze und Diastereoselektivitäten erzielt (Tab. 9, Nr. 5-9), während in Methyl-tert.-butylether keine Reaktion erfolgte (Tab. 9, Nr. 4). Das Verhältnis von (XIX) zu dia-(XIX) (Diastereoselektivität der Carbonylreduktion) reichte im rohen Reaktionsgemisch, nach Solvolyse des intermediären (C), bis zu 98 : 2 (Tabelle 10, Nr. 9). In den isolierten Produkten (XIX) (84-89% der Theorie Ausbeute bezogen auf eingesetztes Salz (XII)) betrug das Diastereomerenverhältnis bis zu 99.2: 0.8 und die Enantiomerenreinheit 95.2% ee, obwohl die eingesetzte Charge Mannich-Salz (XII) eine für die Vierkomponenten-Kupplung mäßige optische Reinheit von nur 93.2% ee hatte und obwohl die Aufarbeitungsprozedur keinen Kristallisationsschritt des Dihydrochlorids von (XIX) aus Butanol enthielt (Tabelle 10, Nr. 9). Bezüglich der chemischen Reinheit waren per HPLC mit Ausnahme von dia-(XIX) keine UV-aktiven Verunreinigungen nachweisbar und der Gehalt des isolierten Produkts an (XIX) betrug gemäß einem HPLC-Assay (bezogen auf einen gereinigten Referenzstandard von (XIX)) 97.9%.

Gemäß der vorliegenden Erfindung lassen sich Verbindungen der Formeln (I) (II) und (III) ausgehend von achiralen, kommerziell erhältlichen, preiswerten oder sehr einfach herstellbaren Edukten (IV), (V), (VI) auf sehr kurzem Wege unter Anwendung preiswerter, gut verfügbarer Hilfsstoffe (VII) und milder, technisch leicht realisierbarer Reaktionsbedingungen in hohen Ausbeuten mit hoher Stereoselektivität herstellen. Das in der vorliegenden Erfindung beschriebene Verfahren ist darum besonders zur industriellen Produktion von optisch aktiven Verbindungen der Formeln (I) und (11) geeignet.

Das folgende Schema beschreibt das erfindungsgemäße Verfahren im Überblick.

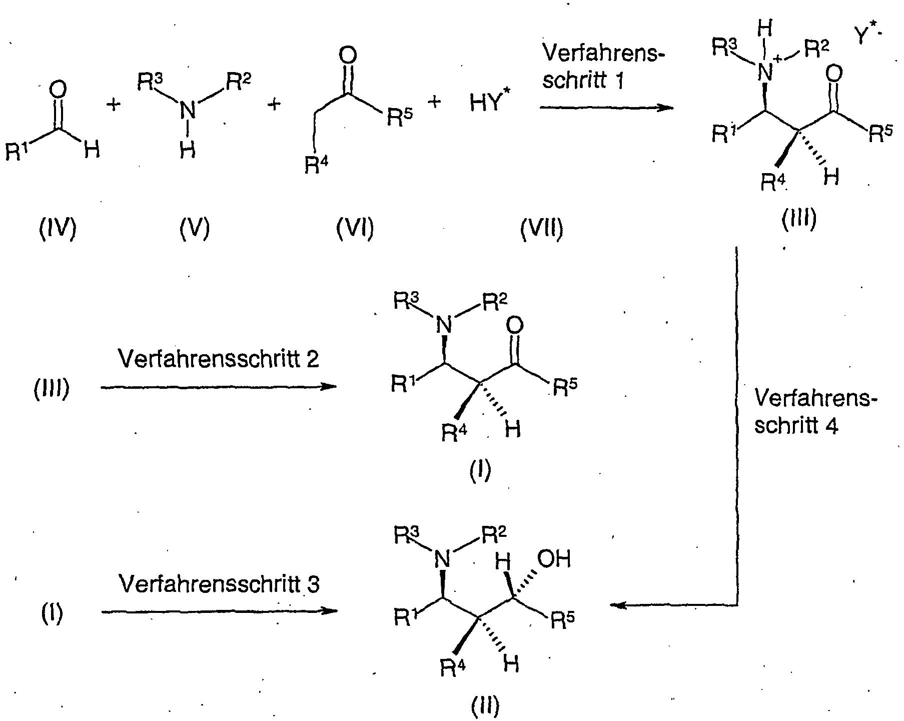

Die vorgenannten Tabellen und beispielhaften Reaktionen enthalten insgesamt 162 Beispiele, die die große Bandbreite möglicher Variationen von Reaktionsparametern innerhalb des erfindungsgemäßen Verfahrens veranschaulichen. Von diesen 162 tabellarisch erfaßten Beispielen wurden die besonders repräsentativen Prozeduren detailliert beschrieben. Bei diesen Prozeduren handelt es sich um bevorzugte Durchführungsformen des erfindungsgemäßen Verfahrens.

In den folgenden Beispielen sind Methoden, Arbeitsweisen und Prozeduren beschrieben, deren Kenntnis notwendig ist, um den Erfindungsgegenstand problemlos reproduzieren oder verifizieren zu können.

### Beispiel 1:

### Bestimmung des Enantiomerenüberschusses von Mannich-Basen der allgemeinen Formel (I) oder von Mannich-Salzen der allgemeinen Formel (III), wobei R¹ = o-Nitrophenyl, R² = 2-Pyridyl, R³ = H, R⁴= 2-Pyridyl, R⁵ = Phenyl, und HY^{*} = (S)-(+)-Mandelsäure ist, per Derivatisierung mit (-)-Camphanoylchlorid.

In einem 10ml Meßkolben werden 10 mg der im Titel genannten Mannich-Base (I) bzw. seines Salzes (III) eingewogen und mit 200 mg (-)-Camphansäurechlorid versetzt. Dazu gibt man 1 ml Triethylamin und füllt mit ca. 9 ml Acetonitril (HPLCgrade) auf exakt 10 ml auf. Das Gemisch wird im Ultraschallbad innerhalb 30 Sekunden gelöst. Von der zunächst hellgelben Lösung werden 1 ml in ein HPLC-Vial überführt und nach 10 Min. Vorlaufzeit werden davon 8.0 µl auf eine HPLC-Säule von Machery-Nagel CC 250mm x 4mm Nucleosil 100-5 C18 / 5 µm HD injiziert. Die Elution erfolgt bei einem Fluß von 1.00 ml/min mit einem linearen Gradienten, der aus den beiden folgenden Laufmitteln zusammengesetzt ist:
Laufmittel 1: Wasser / Acetonitril / Trifluoressigsäure = 900 / 100 / 1.00
Laufmittel 2: Wasser / Acetonitril / Trifluoressigsäure = 100 / 900 / 0.75
bei dem folgenden Gradientenverlauf:

| | | | | | |
|---|---|---|---|---|---|
| Zeit (in Min) | 0 | 2 | 22 | 26 | 27 |
| Laufmittel 1 (in Vol-%) | 75 | 75 | 35 | 35 | 75 |
| Laufmittel 2 (in Vol-%) | 25 | 25 | 65 | 65 | 25 |

Die Detektion erfolgt bei 254 nm. Die Derivatisierungsprodukte werden mit folgenden Retentionszeiten eluiert:

Entsprechendes Amid der allgemeinen Formel (IX A) (vom unerwünschten Enantiomer von (I) herrührend): 19.59 Min.
Amid der Formel (IX) (vom erwünschten Enantiomer von (I) herrührend): 20.50 Min.
Amid vom anti-Diastereomer von (I) herrührend: 23.12 Min.
Amid vom anti-Diastereomer des (I)-Enantiomeren herrührend: 24.09 Min.

Außerdem ist ein Peak (Retentionszeit 20.01 Min.) sichtbar, der von einer Derivatisierungs-Komponente herrührt.

Der Enantiomerenüberschuß von (I) wird anhand des Chromatogramms folgendermaßen berechnet: Man setzt die Summe der Peakflächen von (IX) und (IX A) auf 100%. Man berechnet die Anteile von (IX) und (IX A) (z.B. (IX) = 97.0%, (IX A) = 3.0%). Man zieht den Anteil von (IX A) vom Anteil von (IX) ab.

Beim angegebenen Beispiel hatte die freie Mannich Base (I) bzw. die dem Mannich-Salz (III) zugrundeliegende Mannich-Base (I) eine Enantiomerenreinheit von 94.0% ee.

### Beispiel 2:

### Bestimmung des Enantiomerenüberschusses von Mannich-Basen der allgemeinen Formel (I) oder von Mannich-Salzen der allgemeinen Formel (III), wobei R¹ = o-Nitrophenyl, R² = 2-Pyridyl, R³ = H, R⁴ = 2-Pyridyl, R⁵ = Phenyl und HY^{*} = (S)-(+)-Mandelsäure ist, per Derivatisierung mit Pivaloylchlorid

In einem 2 ml HPLC-Vial werden 1 mg der im Titel genannten Mannich-Base (I) bzw. seines Salzes (III) in 20 µl Pivaloylchlorid, 100 µl Triethylamin und 500 µl Acetonitril (HPLC grade) gelöst. Nach exakt 5 Minuten wird die Reaktion durch Zugabe von 500 µl Wasser gestoppt. Das Vial wird sofort mit der Septenkappe verschlossen, in den Autosampler der HPLC-Anlage gestellt und nach 10 Min. Vorlaufzeit werden davon 5 µl auf eine 250mm x 4 mm 5 µm CHIRADEX-Säule (β-Cyclodextrin) der Firma Merck Darmstadt (Best.-Nr. 1.51333.0001, Cartridge-Nr. 971324) injiziert. Die Elution erfolgt bei einem Fluß von 1.00 ml/min isokratisch mit dem folgenden Laufmittelgemisch:
Laufmittel 1: 1% Triethylamin in Essigsäure (pH 4.1)
Laufmittel 2: 100% Acetonitril
Laufmittel 1 Laufmittel 2 = 82.5 : 17.5.
Detektiert wird bei 254 nm.

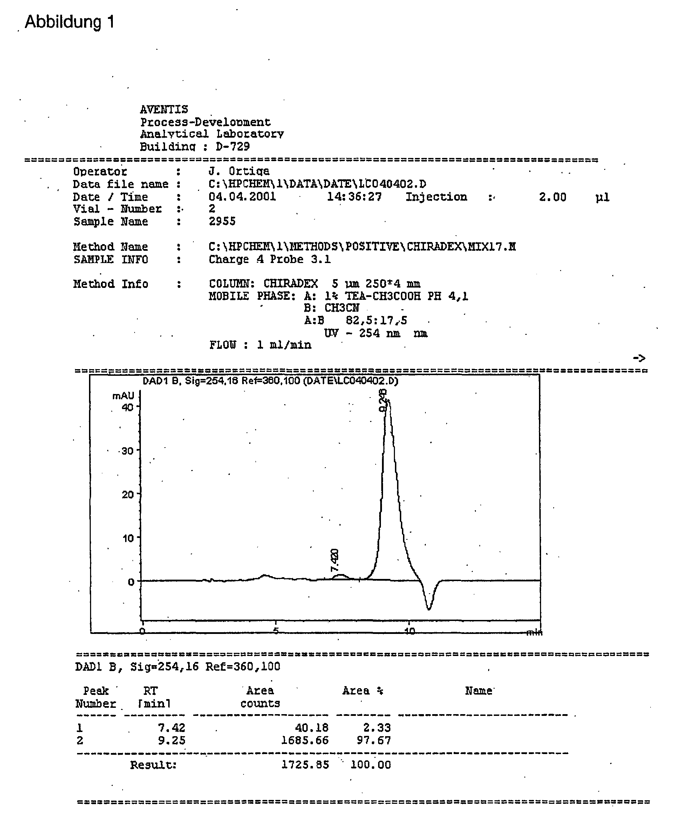

Abbildung 1 zeigt ein typisches Chromatogramm ausgehend von der Mannich-Base der allgemeinen Formel (I) mit den im Titel genannten Substituenten und einer Enantiomerenreinheit von 95.3% ee.

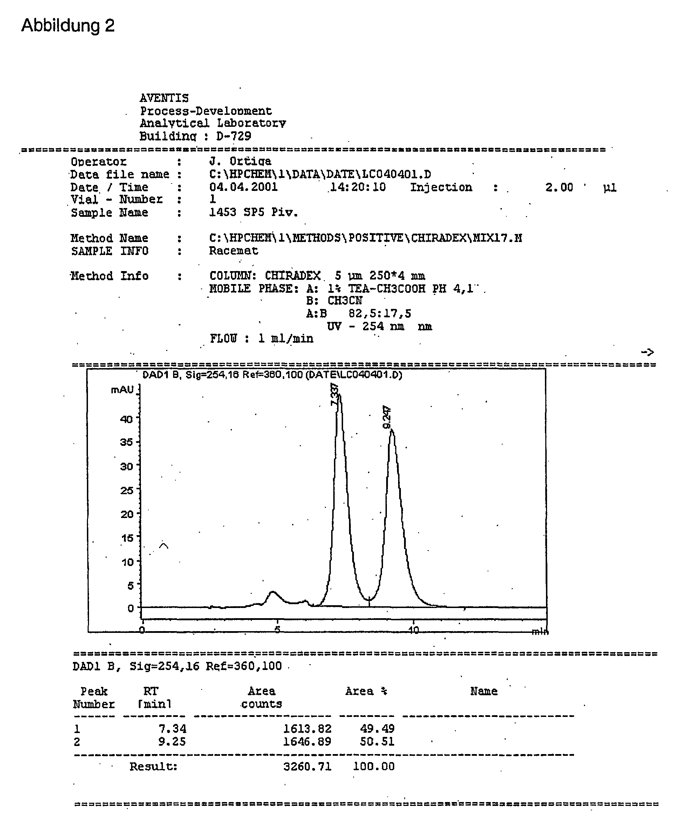

Abbildung 2 zeigt ein Chromatogramm einer entsprechenden racemischen Mannich-Base der allgemeinen Formel (I) mit den im Titel genannten Substituenten.

### Beispiel 3:

### Herstellung von freier racemischer Mannich-Base rac.-(I) [R¹ = o-Nitrophenyl, R² = 2-Pyridyl, R³ = H, R⁴ = 2-Pyridyl, R⁵ = Phenyl] per Katalyse der Dreikomponenten-Kupplung mit 1 Mol-% p-Toluolsulfonsäure

In einen 250ml - Vierhalskolben mit KPG-Rührer werden unter Stickstoff nacheinander 70 ml Ethanol abs., 5.91 g (30 mmol) 1-Phenyl-2-(pyridin-2-yl)-ethanon, 3.53 g (37.5 mmol) 2-Aminopyridin, 5.44 g (36.0 mmol) 2-Nitrobenzaldehyd und 57 mg (0.30 mmol) 4-Toluolsulfonsäure Monohydrat eingetragen. Die Lösung wird bei 25°C unter Stickstoff gerührt. Nach ca. 18 Stunden setzt die Kristallisation des Produkts rac.-(I) ein. DC (n-Heptan / EtOAc) zeigt zu diesem Zeitpunkt einen Umsatz von ca. 40% an. Nach insgesamt 96 Stunden zeigt ein Dünnschichtchromatogramm (DC) praktisch quantitativen Umsatz an. Der Niederschlag wird abgesaugt, mit Mutterlauge, dann mit 10 ml Ethanol gewaschen und im Vakuum bei 30°C getrocknet. Man erhält 11.9 g (28.0 mmol; 93.2% der Theorie) gelbe Kristalle.
Das Integral des ¹H-NMR-Spektrums (CDCl₃, sofort nach dem Lösen vermessen) zeigt ein Verhältnis der gewünschten Verbindung zum anti-Diastereomer von 97:3 an.

### Beispiel 4:

### Umsetzung von rac.-(I) mit Pivaloylchlorid in Aceton zum Amid rac.-(IX) [R¹ = o-Nitrophenyl, R² = 2-Pyridyl, R³ = H, R⁴ = 2-Pyridyl, R⁵ = Phenyl, R = tert.-Bu]

In einem 500 ml-Vierhalskolben legt man unter Stickstoff bei 0°C 15.02 g (35.4 mmol) der racemischen Mannich-Base rac.-(I) aus Beispiel 3 vor. Man läßt unter Kühlung bei 0°C IT 90 ml Aceton zulaufen und dosiert dann parallel zueinander, aus zwei Tropftrichtern, 6.44 g (53.3 mmol) Pivaloylchlorid und 13.82 g (106.9 mmol) Diisopropylethylamin zu. Nach dreistündigem Rühren bei 0°C zeigt HPLC-Analyse 95.9% des gewünschten rac.-(IX), 1.1 % des entsprechenden trans-Diastereomers und 1.9% unumgesetztes rac.-(I) an. 40 ml Aceton werden im Vakuum abdestilliert (Badtemperatur <35°C). Zum Rückstand läßt man 200 ml Wasser zulaufen und rührt dann 2 Stunden bei 0°C Innentemperatur (IT) nach. Der Niederschlag wird abgesaugt, mit 20 ml eiskaltem Ethylacetat auf der Nutsche gewaschen und dann im Vakuum bei 40°C getrocknet. Man erhält 16.4 g (32.2 mmol, 91 % der Theorie) hellgelben kristallinen Feststoff, Schmp. 162°C. Die HPLC-Reinheit beträgt 99.4%.

### Beispiel 5:

### Klassische Racematspaltung von rac.-(I) [R¹ = o-Nitrophenyl, R² = 2-Pyridyl, R³ = H, R⁴ = 2-Pyridyl, R⁵ = Phenyl] mit (S)-(+)-Mandelsäure in Aceton

Zu 503.9 mg (1.19 mmol) rac.-(I) aus Beispiel 3 und 359.0 mg (2.36 mmol, 1.98 Äquiv.) (S)-(+)-Mandelsäure wurden 6 ml Aceton gegeben. Das Reaktionsgemisch wurde im dicht verschlossenen Kolben 20 Stunden bei 25°C magnetisch gerührt, der Niederschlag abgesaugt und im Vakuum getrocknet. Erhalten wurden 446 mg (0.773 mmol) des entsprechenden Mandelat-Salzes (III), das gemäß ¹H-NMR aus Mannich-Base (I) und Mandelsäure im Verhältnis 1 : 1.00 bestand. Derivatisierung einer Probe mit (-)-Camphansäurechlorid und anschließende HPLC-Analyse gemäß Beispiel 1 lieferte ein Verhältnis des Amids (IX A) zum Amid (IX) von 5.0 zu 95.0. Der Enantiomerenüberschuß der Mannich-Base (I) im Mandelat-Salz (III) betrug somit 90% ee.

### Beispiel 6:

### Klassische Racematspaltung von rac.-(I) [R¹ = o-Nitrophenyl, R² = 2-Pyridyl, R³ = H, R⁴ = 2-Pyridyl, R⁵ = Phenyl] mit L-(-)-Äpfelsäure in Aceton

Zu 504.2 mg (1.19 mmol) rac.-(I) aus Beispiel 3 und 161.5 mg (1.20 mmol, 1.01 Äquiv.) L-(-)-Äpfelsäure wurden 6 ml Aceton gegeben. Das Reaktionsgemisch wurde im dicht verschlossenen Kolben 20 Stunden bei 25°C magnetisch gerührt, der Niederschlag abgesaugt und im Vakuum getrocknet. Erhalten wurden 400 mg (0.716 mmol) des entsprechenden Äpfelsäure-Salzes (III), das gemäß ¹H-NMR aus Mannich-Base (I) und Äpfelsäure im Verhältnis 1 : 1.04 bestand. Derivatisierung einer Probe mit (-)-Camphansäurechlorid und anschließende HPLC-Analyse gemäß Beispiel 1 lieferte ein Verhältnis des Amids (IX A) zum Amid (IX) von 2.4 zu 97.6. Der Enantiomerenüberschuß der Mannich-Base (I) im Äpfelsäure-Salz (III) betrug somit 95.2% ee.

### Beispiel 7:

### Klassische Racematspaltung von rac.-(I) [R¹ = o-Nitrophenyl, R² = 2-Pyridyl, R³ = H, R⁴ = 2-Pyridyl, R⁵ = Phenyl] mit (-)-Di,O,O'-pivaloyl-D-Weinsäure [(-)-DPWS] in Aceton

Zu 506.2 mg (1.19 mmol) rac.-(I) aus Beispiel 3 und 379.2 mg (1.19 mmol, 1.00 Äquiv.) (-)-DPWS wurden 6 ml Aceton gegeben. Das Reaktionsgemisch wurde im dicht verschlossenen Kolben 20 Stunden bei 25°C magnetisch gerührt, der Niederschlag abgesaugt und im Vakuum getrocknet. Erhalten wurden 557 mg des entsprechenden DPWS-Salzes (III), das gemäß ¹H-NMR aus Mannich-Base (I) und DPWS im Verhältnis 1 : 0.57 bestand. Derivatisierung einer Probe mit (-)-Camphansäurechlorid und anschließende HPLC-Analyse gemäß Beispiel 1 lieferte ein Verhältnis des Amids (IX A) zum Amid (IX) von 97.6 zu 2.4. Der Enantiomerenüberschuß der entsprechenden Mannich-Base (I) im DPWS-Salz (III) betrug somit 95.2% ee.

### Beispiel 8:

### Versuch einer klassischen Racematspaltung von rac.-(I) [R¹ = o-Nitrophenyl, R² = 2-Pyridyl, R³ = H, R⁴ = 2-Pyridyl, R⁵ = Phenyl] mit (S)-(+)-Mandelsäure in Ethanol

Zu 500 mg (1.18 mmol) rac.-(I) aus Beispiel 3 und 358.5 mg (2.36 mmol, 2.00 Äquiv.) (S)-(+)-Mandelsäure wurden 6 ml Ethanol gegeben. Das Reaktionsgemisch wurde im dicht verschlossenen Kolben 18 Stunden bei 20-25°C magnetisch gerührt, der Niederschlag abgesaugt, mit wenig Ethanol gewaschen und im Vakuum getrocknet.

Erhalten wurden 590 mg (1.02 mmol) des entsprechenden Mandelat-Salt (III). HPLC-Analyse gemäß Beispiel 1 lieferte ein Verhältnis des Amids (IX A) zum Amid (IX) von 47.9 zu 52.1 . Der Enantiomerenüberschuß der Mannich-Base (I) im Mandelat-Salz (III) betrug somit nur 4% ee.

### Beispiel 9:

### Synthese des Imins (X) aus dem Aldehyd (IV) und dem Amin (V) [R¹ = o-Nitrophenyl, R² = 2-Pyridyl]

Zu 9.97 g (106 mmol) 2-Aminopyridin , 15.12 g (100 mmol) 2-Nitrobenzaldehyd und 190.3 mg (1 mmol) 4-Toluolsulfonsäure Monohydrat werden 50 ml Toluol gegeben und das Reaktionsgemisch unter Stickstoff unter azeotropem Abdestillieren des Toluol / Wasser-Azeotrops am Wasserabscheider 1 Std. zum Rückfluß erwärmt. Man kühlt dann auf Raumtemperatur ab, wobei das entsprechende Imin (X) mit R¹ = o-Nitrophenyl, R² = 2-Pyridyl auskristallisiert. Das Produkt wird abgesaugt und im Vakuum getrocknet. Man erhält 18.2 g (80 mmol, 80% der Theorie) gelbe Kristalle. Gemäß ¹H-NMR (300 MHz, CDCl₃; nach dem Auflösen sofort messen) besteht das Produkt zu 80% aus dem Imin (X) [δ = 7.24 (m, 1H), 7.38 (d, 1H), 7.63 (td, 1H), 7.70 - 7.83 (m, 2H), 8.06 (dd, 1H), 8.36 (dd, 1H), 8.53 (dm, 1H), 10.28 (s, 1H)] und zu je 10% aus den Edukten 2-Aminopyridin und 2-Nitrobenzaldehyd. IR (KBr): v = 1513 (s), 1435 (m), 1352 (m), 1339 (s), 788 (m) cm⁻¹. MS (DCl): C₁₂HgN₃O₂ (M = 227), m/z = 228 (100%, M + H⁺).

### Beispiel 10:

### Synthese des Aminals (XI) aus dem Aldehyd (IV) und dem Amin (V) [R¹ = o-Nitrophenyl, R² = 2-Pyridyl]

In einem 250 ml Vierhalsrundkolben mit KPG-Rührer, Thermometer, Wasserabscheider und Rückflußkühler werden unter Stickstoff in 53 ml Dichlormethan 9.97 g (106 mmol) 2-Aminopyridin und 15.12 g (100 mmol) 2-Nitrobenzaldehyd gelöst, wobei die IT auf 12°C abfällt. Es werden 1.5 g stark saurer Ionenaustauscher (Amberlite IR 120, Merck) eingetragen und das Reaktionsgemisch dann bei einer Badtemperatur von 75°C zum Rückfluß erhitzt. Im Wasserabscheider sammeln sich ca. 1.5 ml Wasser (Theorie: 1.8 ml aus der Reaktion plus 0.8 ml aus dem Ionenaustauscher). Nach 5.5 Stunden ist keine Wasserabscheidung mehr feststellbar. Beim Abschalten des Rührers erkennt man über dem abgesetzten Ionenaustauscherharz eine klare Lösung, die kaum dunkler als die ursprüngliche Eduktlösung ist. Nach Stehen über Nacht bei RT sind gelbe Kristalle in erheblicher Menge abgeschieden. Die Suspension wird zum Rückfluß erhitzt und soviel Dichlormethan zugesetzt (ca. 100 ml), daß sich das Kristallisat gerade vollständig in der Siedehitze löst. Der Ansatz wird heiß durch ein Faltenfilter filtriert, um den Ionenaustauscher zu entfernen. Das Filtrat wird mit 250 ml Toluol versetzt und das Dichlormethan im Vakuum (Beginn: 400 mbar, Ende 100 mbar) bei 40°C Badtemperatur abgedampft. Gegen Ende des Einengens fällt ein blaßgelber Niederschlag aus. Durch Verbesserung des Vakuum auf 15 mbar wird dann auch 2/3 des Toluols abgedampft. Die Suspension wird über Nacht gut verschlossen im Kühlschrank bei ca. 0°C aufbewahrt, wobei sich die Kristallisation des Produkts vervollständigt. Der Feststoff wird abgesaugt, mit 20 ml kaltem Toluol gewaschen und bei 40°C im Vakuum getrocknet. Man erhält 14.50 g (45.1 mmol, 45.1% der Theorie) blaßgelben Feststoff, Schmelzpunkt 134-135°C , nach erneuter Umkristallisation aus Toluol Schmelzpunkt 140-142°C. ¹H-NMR (300 MHz, DMSO-d₆): δ = 6.53 (tm, 2H), 6.58 (d, 2H), 7.20 (d, 2H), 7.30 - 7.44 (m, 3H), 7.53 (td, 1H), 7.67 (td, 1H), 7.78 (dt, 1H), 7.88 (d, 1H), 7.94 (m, 2H). IR (KBr): v =3227 (m), 3074 (m) and 3020 (m), 1599 (s), 1576 (m), 1532 (s), 1459 (m), 1435 (s), 1320 (m), 1149 (m), 771 (m) cm⁻¹. MS (DCl): C₁₇H₁₅N₅O2 (M = 321), m/z = 228.1 (100%, M + H⁺ - Aminopyridin), 94.8 (Aminopyridin).

### Beispiel 11:

### Rückgewinnung von (S)-(+)-Mandelsäure aus der wäßrigen Mutterlauge der Freisetzung von Mannich-Base (I) aus einem Mannich-Salz der Formel (III) [R¹ = o-Nitrophenyl, R² = 2-Pyridyl, R³ = H, R⁴ = 2-Pyridyl, R⁵ = Phenyl, HY^{*} = (S)-(+)-Mandelsäure]

Aus 256.5 g (445.0 mmol) Mannich-Salz (III) mit den im Titel genannten Substituenten wurde in 1280 ml Wasser und 128 ml Ethanol mittels 222.0 ml 2N Natronlauge (444.0 mmol) bei pH-Stat 8.5 die entsprechende Mannich-Base (I) freigesetzt, abgesaugt, mit 3 x 150 ml Wasser gewaschen und im Vakuum getrocknet, wobei 188.52 g (I) (444.1 mmol, 99.8% der Theorie) erhalten wurden.
Die gelbe wäßrige Mutterlauge (pH 7.62), die zuvor 5 Tage bei Raumtemperatur gestanden hatte, wurde zunächst mit 2 x 250 ml Methyl-tert.butylether, dann mit 250 ml Ethylacetat gewaschen. Die genannten Waschphasen waren alle deutlich gelb, enthielten nach dem Einengen zur Trockne im Vakuum 0.21 g, 0.06 g und 0.04 g Rückstand und wurden sämtlich verworfen. Die nur noch sehr blaß gelbe wäßrige Mutterlauge (pH 7.83) wurde mittels 12 ml 37%iger Salzsäure auf den pKs-Wert der Mandelsäure (pH 3.85) eingestellt (geeichte Glaselektrode). Die Lösung trübte sich, aber es fiel keine Mandelsäure aus. Es wurde mit 500 ml Ethylacetat extrahiert. Dieses "Extrakt 1" enthielt nach dem Einengen zur Trockne im Vakuum 14.10 g (92.67 mmol, 20.8% d.Th) Rückstand. Zu der Wasserphase wurden unter Rühren weitere 19 ml 37%ige Salzsäure zugetropft, wobei der pH von 4.2 auf 2.44 fiel und wieder eine Trübung auftrat. Es wurde mit 500 ml Ethylacetat extrahiert. Dieses "Extrakt 2" enthielt nach dem Einengen zur Trockne im Vakuum 29.57 g (194.35 mmol, 43.7 % der Theorie) Rückstand. Zu der Wasserphase wurden unter Rühren 18.5 ml 37%ige Salzsäure zugetropft, wobei der pH von 2.99 auf 1.08 fiel. Es wurde mit 500 ml Ethylacetat extrahiert. Dieses "Extrakt 3" enthielt nach dem Einengen zur Trockne im Vakuum 12.62 g (82.94 mmol, 18.6% der Theorie) Rückstand. Die Wasserphase (pH 1.4) wurde nochmals mit 500 ml Ethylacetat extrahiert. Dieses "Extrakt 4" enthielt nach dem Einengen zur Trockne im Vakuum 3.71 g (24.38 mmol, 5.5% der Theorie) Rückstand. Die Schmelzpunkte (DSC-Messungen) aller vier Rückstände (Extrakte 1 bis 4) lagen bei 133.2°C bis 133.5°C. Gemäß ¹H-NMR-Spektren (400 MHz, DMSO-d₆) bestanden alle vier Rückstände aus Mandelsäure hoher Reinheit. Eine Probe jedes Rückstands wurde mit einer Lösung von Diazomethan in Diethylether zum Methylester derivatisiert und dieser per GC auf einer Kapillarsäule mit Chiralphase auf den Enantiomerenüberschuß analysiert [50 m x 0.25 mm ID fused silica Kapillarsäule, belegt mit 0.25 µm Lipodex-E (Ser.-Nr. 723369, Säulen-Nr. 20174-32). Ofentemperatur: 115°C isotherm, Injektor: 200°C, Detektor 220°C, Fluß: 2.0 ml He / min. Split: 1:100. Die Retentionszeit der (S)-(+)-Mandelsäure (als Methylester) betrug 24.73 min. An einer racemischen Vergleichsprobe wurde ermittelt, daß die Retentionszeit der (R)-(-)-Mandelsäure (als Methylester) 25.90 min betrug]. In keinem der Rückstande (Extrakte 1 bis 4) konnte (R)-(-)-Mandelsäure nachgewiesen werden. Insgesamt 60.0 g (394.35 mmol, 88.6% der Theorie) (S)-(+)-Mandelsäure mit 100% ee wurden somit zurückgewonnen.
Für eine Rückgewinnung der (S)-(+)-Mandelsäure im technischen Maßstab bietet es sich also an, die wäßrige Mutterlauge kontinuierlich, z.B. im Gegenstrom-Verfahren, mit z.B. Ethylacetat zu extrahierten, wobei man den pH-Wert durch kontinuierliche Zugabe von 37%iger Salzsäure im Bereich von 2.5 - 1.0 hält.

### Beispiel 12:

### Synthese und Isolierung des Gemischs von Oxaborinanen mit der Hauptkomponente der Formel (C) [R¹ = o-Nitrophenyl, R² = 2-Pyridyl, R³ = H, R⁴ = 2-Pyridyl, R⁵ = Phenyl]

In einem 250 ml-Vierhalskolben mit KPG-Rührer, Innenthermometer und Septum wurde unter Stickstoff die Suspension von 6.37 g (15 mmol) einer Mannich-Base (I) [R¹ = o-Nitrophenyl, R² = 2-Pyridyl, R³ = H, R⁴ = 2-Pyridyl, R⁵ = Phenyl] in 75 ml Toluol mit einem Eisbad auf +1°C IT gekühlt. Innerhalb von 2 Minuten wurden per Spritze 4.47 ml (45 mmol, 3.0 Äquiv.) Boran-Dimethylsulfid (95 %ig in Dimethylsulfid) zugesetzt, wobei die IT bis auf maximal +3°C anstieg. Das Kühlbad wurde entfernt und die Suspension innerhalb von 15 Minuten auf +18°C erwärmt. Die hellgelbe Suspension wurde 45 Min. bei dieser Temperatur heftig gerührt.

HPLC-Analyse der Suspension [Injektion von 8.0 µl einer Lösung in Acetonitril auf eine 250 x 4 mm Stahlsäule Nucleosil 100-5 C18, 5 µm, Fluß 1.0 ml / Min., Det. 254 nm, Laufmittel A: Wasser (900 ml) / Acetonitril (100 ml) / Trifluoressigsäure (1.00 ml), Laufmittel B: Wasser (100 ml) / Acetonitril (900 ml) / Trifluoressigsäure (0.75 ml); Elution mit linearem Gradienten: 0-2 min (75% A, 25% B), 22-26 min (35% A, 65% B), 27 min (75% A, 25% B)] zeigte an, daß sich die Mannich-Base (I) bis auf weniger als 2% umgesetzt hatte ((I) und die auf der Säule entstehenden Retro-Mannich-Produkte geben einen breiten Peak mit Schultern bei tᵣₑₜ 3-4 min). Neben dem Toluol-Peak (tᵣₑₜ 20.8 min), mehreren schwachen Peaks und 3% des 1,3-Aminoalkohols (II) (tᵣₑₜ 12.4 min) wurden zwei Peaks langer Retentionszeit angezeigt ("Peak 1" tᵣₑₜ 25.5 min, "Peak 2" tᵣₑₜ 28.6 min), deren Peakfläche zusammen 93% aller Peaks (außer Toluol) ausmachte. Zwischen diesen beiden Peaks wurde die Grundlinie nicht wieder erreicht (Aufsitzer auf einem Plateau), was eine Umwandlung der Verbindung "Peak 1" in die Verbindung "Peak 2" auf der Säule hindeutet.

Die Suspension wurde auf +5°C gekühlt und zügig mit 5 ml Wasser versetzt, dann 5 min bei RT gerührt. Die Suspension wurde über einen Büchner-Trichter filtriert. Der sehr blaß gelbliche Feststoff wurde mit Toluol gewaschen (2 x 10 ml) und bei +45°C /150 mbar unter Stickstoff getrocknet. Es wurden 6.22 g (14.26 mmol berechnet auf Formel (C), 95% der Theorie) farbloses Pulver erhalten.

Dieses Pulver zeigte im DSC einen schwachen endothermen Peak bei 104.6°C (-9.5 J/g) und einen sehr stark exothermen (1718 J/g) Zersetzungspeak bei 166.8°C (Onset bei 157°C).

HPLC/MS (API positiv) ergab für "Peak 1" M+H⁺ : m/z = 437.3 , was der Summenformel C₂₅H₂₁BN₄O₃ (Molgewicht 436.28) der Formel (C) entspricht. Für "Peak 2" wurden folgende Massenpeaks angezeigt: m/z= 488.3, 449.2 und 439.3 . Möglicherweise handelt es sich dabei um das Borsäure-Addukt des 1,3-Aminoalkohols (II) [ C₂₅H₂₂N₄O₃ x H₃BO₃ , Molgewicht 488.3 ]. Borsäure und Aminoalkohol (II) sind die erwarteten Hydrolyseprodukte des Oxazaborinans (C) in saurem wäßrigem Milieu.

Schließlich wurde eine Probe des farblosen Pulvers (C) mit 3.0 Äquiv. Methansulfonsäure in einem Überschuß Methanol bei +20°C solvolysiert. HPLC-Analyse des Reaktionsgemischs zeigte das fast vollständige Verschwinden (<1%) von "Peak 1" und "Peak 2" unter gleichzeitigem kontinuierlichen Anwachsen der Peaks des Aminoalkohols (II) (94%) und seines Diastereomeren dia-(II) (tᵣₑₜ 8.2 min, 4%) an. Aufarbeitung analog Beispiel 19 lieferte das Dihydrochlorid des reinen Aminoalkohols (II) (100% ee, 99.5% de) in einer Ausbeute von 75% der Theorie.

### Beispiel 13:

### Synthese des optisch aktiven Mannich-Salzes der Formel (III) [R¹ = o-Nitrophenyl, R² = 2-Pyridyl, R³ = H, R⁴ = 2-Pyridyl, R⁵ = Phenyl] durch Vierkomponenten-Kupplung unter dynamischer Racematspaltung bei Raumtemperatur; Monitoring des zeitlichen ee-Verlaufs (Tabelle 1); Verwendung von (+)-Dipivaloyl-weinsäure als chiralem Hilfsstoff [HY^{*} = (+)-DPWS] und Ethanol als Lösungsmittel (Tabelle 2, Nr. 5):

In einen 100ml-Dreihals-Rundkolben mit KPG-Rührer, Stickstoffzuleitung und Blasenzähler wurden 60 ml Ethanol (mit Toluol vergällt) unter Rühren vorgelegt und nacheinander 4,63 g (23,5 mmol, 1,00 Äquiv.) 2-Pyridylmethyl-phenyl-keton, 2,77 g (29,4 mmol, 1,25 Äquiv.) 2-Aminopyridin, 4,26 g (28,2 mmol, 1,20 Äquiv.) 2-Nitrobenzaldehyd und 7,48 g (23,5 mmol, 1,00. Äquiv.) (+)-Dipivaloylweinsäure eingetragen. Nach ca. 10 Min. entstand eine klare, gelbe Lösung, die sich ca. 15 Min. später einzutrüben begann. Es wurden Impfkristalle (10 mg) von enantiomerenreinem (+)-DPWS-Salz zugegeben und es entstand eine gelbe Suspension, die 14 Tage bei Raumtemperatur unter Stickstoffatmosphäre gerührt wurde. Zu den aus Tabelle 1 ersichtlichen Zeitpunkten wurden jeweils kleine Aliquote der Reaktionssuspension entnommen, der darin enthaltene Feststoff per Mikrofiltration von der Mutterlauge getrennt und wie in Beispiel 1 beschrieben mit (-)-Camphanoylchlorid derivatisiert, und mittels HPLC-analysiert. Der beobachtete zeitliche ee-Verlauf ist in Tabelle 1 wiedergegeben. Am 14. Tag betrug das Verhältnis von gewünschtem Enantiomer zu unerwünschtem Enantiomer 97,67 : 2,33, entsprechend 95,34% ee. Die nun weiße Suspension wurde filtriert, der Filterrückstand mit der Mutterlauge und dann zweimal mit je 10 ml Ethanol gewaschen. Der Feststoff wurde 2 Stunden bei 45°C im HV getrocknet. Man erhielt 11,45 g (9,81 mmol, 83,6% der Theorie) des weißen Salzes, das gemäß ¹H-NMR und Titration zwei Mannich-Base-Kationen pro DPWS-Dianion enthielt. Es läßt sich abschätzen, daß die tatsächliche Ausbeute deutlich über 90% der Theorie lag, da durch die 10 zwischenzeitlichen Probenahmen signifikante Produktmengen verbraucht wurden.

### Beispiel 14:

### Synthese des optisch aktiven Mannich-Salzes der Formel (III) [R¹ = o-Nitrophenyl, R² = 2-Pyridyl, R³ = H, R⁴ = 2-Pyridyl, R⁵ = Phenyl] durch Vierkomponenten-Kupplung unter dynamischer Racematspaltung bei +40°C. Verwendung von (+)-Dipivaloyl-weinsäure als chiralem Hilfsstoff [HY^{*} = (+)-DPWS] und Ethanol als Lösungsmittel (Tabelle 2, Nr. 6):

In einem 250ml-Vierhals-Rundkolben mit KPG-Rührer, Stickstoffzuleitung, Rückflußkühler mit Blasenzähler löste man unter Rühren 5,06 g (25,65 mmol, 1,00 Äquiv.) 2-Pyridylmethyl-phenyl-keton in 60 ml absolutem Ethanol. Innerhalb von 10 Min. gab man bei 40°C Innentemperatur nacheinander 2,99 g (31,76 mmol, 1,24 Äquiv.) 2-Aminopyridin, 4,61 g (30,53 mmol, 1,19 Äquiv.) 2-Nitrobenzaldehyd und 8,08 g (25,38 mmol, 0,99 Äquiv.) (+)-DPWS zu, wobei man nach jeder Zugabe gerade die Zeit abwartete, bis der Feststoff vollständig in Lösung gegangen war. Man erhielt eine klare gelbe Lösung, die nach 25 Min. in eine gelbe Suspension überging. Das Reaktionsgemisch rührte dann über Nacht bei 40°C. Zwischenzeitlich. entnommene und derivatisierte Proben zeigten, daß der Enantiomerenüberschuß des Feststoffs 55,7% ee nach 4,16 Stunden und 93,0 % ee nach 20 Stunden betrug. Nach 23 Stunden wurde das Heizbad entfernt, die Suspension innerhalb 15 Minuten aufs 23°C abgekühlt, der Niederschlag abgesaugt, mit 2 mal 10 ml Ethanol gewaschen, dann bei 45°C im HV getrocknet. Erhalten wurden 14,89 g (12,76 mmol, 25,52 mmol der Mannich-Base (I) mit den im Titel genannten Substituenten enthaltend, 99,5% der Theorie) sehr blaßgelber Feststoff. Gemäß ¹H-NMR und Titration bestand das Salz aus (I) und DPWS im Verhältnis 2:1. Der Enantiomeren-überschuß betrug 95,9% ee.

### Beispiel 15:

### Synthese des optisch aktiven Mannich-Salzes der Formel (III) [R¹ = o-Nitrophenyl, R² = 2-Pyridyl, R³ = H, R⁴ = 2-Pyridyl, R⁵ = Phenyl] durch Vierkomponenten-Kupplung unter dynamischer Racematspaltung bei +60°C. Verwendung von (S)-(+)-Mandelsäure als chiralem Hilfsstoff [HY^{*} = (+)-MDLS] und Ethanol als Lösungsmittel (Tabelle 3, Nr. 7):

In einem 2-Liter-Doppelmantelreaktor (angeschlossen an einen Umlaufthermostaten) mit Temperaturfühler und mechanischem Turbinenrührer wurden bei Raumtemperatur unter Stickstoffatmosphäre 97,2 g (492,8 mmol, 1,00 Äquiv.) 2-Pyridylmethyl-phenyl-keton in 1200 ml Ethanol (mit Methylethylketon vergällt) gelöst. Die Innentemperatur wurde während 15 Min. auf 40°C erhöht. Bei dieser Temperatur wurden nacheinander 55,66 g (591,4 mmol, 1,20 Äquiv.) 2-Aminopyridin, 89,37 g (591,4 mmol, 1,20 Äquiv.) 2-Nitrobenzaldehyd und 149,96 g (985,6 mmol, 2,00 Äquiv.) (S)-(+)-Mandelsäure zugegeben. Unmittelbar anschließend wurde die Innentemperatur des Reaktionsgemischs auf 60°C erhöht und eine klare Lösung erhalten. Dieser Aufheizvorgang dauerte 30 Min., 15 Min. später war eine erste Niederschlagsbildung zu erkennen. Per Probenahme / Derivatisierung / HPLC-Analyse gemäß Beispiel 1 wurde ein Enantiomerenüberschuß des Niederschlags von 91,5% ee nach 2 Std. , von 93,0% ee nach 3,5 Std. und von 94,4% ee nach 4,5 Std. ermittelt. Das Reaktionsgemisch wurde innerhalb von 2 Std. auf 20°C abgekühlt. Der Niederschlag wurde abgesaugt, mit 3 mal 50 ml Ethanol gewaschen, dann im Vakuum bei 50 mbar und 40°C bis zur Gewichtskonstanz getrocknet. Man erhielt 262,4 g (455,2 mmol, 92,4% der Theorie) des Mandelat-Salzes (III) mit den im Titel genannten Substituenten. Der Schmelzpunkt betrug 153-154 °C. Gemäß ¹H-NMR enthielt es die entsprechende Mannich-Base (I) und Mandelsäure im Verhältnis 1:1. Die Enantiomerenreinheit betrug 94,4% ee gemäß Derivatisierung mit Camphanoylchlorid und 97,5% ee gemäß der genaueren Methode der Pivaloyl-Derivatisierung entsprechend Beispiel 2.

### Beispiel 16:

### Synthese des optisch aktiven Mannich-Salzes der Formel (III) [R¹ = o-Nitrophenyl, R² = 2-Pyridyl, R³ = H, R⁴ = 2-Pyridyl, R⁵ = Phenyl] durch Vierkomponenten-Kupplung unter dynamischer Racematspaltung bei +40°C. Verwendung von (S)-(+)-Mandelsäure als chiralem Hilfsstoff [HY^{*} = (+)-MDLS] und Aceton als Lösungsmittel (Tabelle 3, Nr. 20):

In einem 2-Liter-Doppelmantelreaktor (angeschlossen an einen Umlaufthermostaten) mit Temperaturfühler und mechanischem Turbinenrührer wurden bei Raumtemperatur unter Stickstoffatmosphäre 97,2 g (492,8 mmol, 1,00 Äquiv.) 2-Pyridylmethyl-phenyl-keton in 1200 ml Aceton vorgelegt. Die Innentemperatur wurde während 15 Min. auf 40°C erhöht. Bei dieser Temperatur wurden nacheinander 55,66 g (591,4 mmol, 1,20 Äquiv.) 2-Aminopyridin, 89,37 g (591,4 mmol, 1,20 Äquiv.) 2-Nitrobenzaldehyd und 149,96 g (985,6 mmol, 2,00 Äquiv.) (S)-(+)-Mandelsäure zugegeben, wonach eine klare Lösung erhalten wurde, die weiter bei 40°C gerührt wurde. Nach 4,5 Std. war eine erste Niederschlagsbildung zu erkennen. Nach 24 Std. ergab Probenahme / Derivatisierung / HPLC-Analyse gemäß Beispiel 1 97,0% ee des Niederschlags. Die Suspension wurde innerhalb 2,5 Std. auf eine Innentemperatur von 25°C abgekühlt. Der Niederschlag wurde abgesaugt, mit 3 mal 50 ml Aceton gewaschen und im Vakuum bei 50 mbar und 40°C getrocknet. Erhalten wurden 250,4 g (434,4 mmol, 88,2% der Theorie) des Mandelat-Salzes (III) mit den im Titel genannten Substituenten als fast farblosen Feststoff mit einem Schmelzpunkt von 156-158°C. Gemäß ¹H-NMR enthielt es die entsprechende Mannich-Base (I) und Mandelsäure im Verhältnis 1:1. Die Enantiomerenreinheit betrug 95,7% ee gemäß Derivatisierung mit Camphanoylchlorid (Beispiel 1) und 97,0% ee gemäß der genaueren Methode der Piv-Derivatisierung (Beispiel 2).

### Beispiel 17:

### Synthese des optisch aktiven Mannich-Salzes der Formel (III) [R¹ = o-Nitrophenyl, R² = 2-Pyridyl, R³ = H, R⁴ = 2-Pyridyl, R⁵ = Phenyl] durch Kupplung mit in situ vorgebildeter Schiffscher Base unter dynamischer Racematspaltung bei 40°-60°C; Verwendung von (S)-(+)-Mandelsäure als chiralem Hilfsstoff [HY^{*} = (+)-MDLS] und n-Butylacetat als Lösungsmittel (Tabelle 3, Nr. 23):

In einem 1-Liter-Vierhalsrundkolben mit Wasserabscheider mit aufgesetztem Rückflußkühler, KPG-Rührer, Stickstoffzuleitung, Vakuumanschluß, wurde die Lösung von 25,87 g (275 mmol) 2-Aminopyridin und 37,75 g (250 mmol) 2-Nitrobenzaldehyd in 500 ml n-Butylacetat bei 100 mbar auf 70°C Badtemperatur (50-60°C Innentemperatur) zum Rückfluß erhitzt, wobei sich im Wasserabscheider innerhalb von 2,2 Std. ca. 4,7 ml Wasser abschieden.
Das Gemisch wurde dann über Nacht bei 22°C unter Stickstoffatmosphäre stehen gelassen. Es wurden dann unter Rühren 49,2 g (250 mmol) 2-Pyridylmethyl-phenyl-keton und, nachdem alles gelöst war, 45,6 g (300 mmol) (S)-(+)-Mandelsäure zugegeben und auf 40°C Innentemperatur erwärmt. Eine Niederschlagsbildung wurde nach 5 Min. beobachtet. Nach 3 Std. bei 40°C wurde weiter auf 60°C erwärmt und 24 Std. bei dieser Temperatur gerührt. Die Suspension wurde unter Rühren auf 25°C abgekühlt, der Niederschlag abgesaugt, mit 2 mal 50 ml n-Butylacetat gewaschen und im Vakuum bei 50°C getrocknet. Erhalten wurden 134,6 g (233,4 mmol, 93,4% der Theorie) des Mandelat-Salzes (III) mit den im Titel genannten Substituenten. Gemäß ¹H-NMR enthielt es die entsprechende Mannich-Base (I) und Mandelsäure im Verhältnis 1:1. Die Enantiomerenreinheit betrug 95,4% ee gemäß Derivatisierung mit Camphanoylchlorid (Beispiel 1) und 98,0% ee gemäß der genaueren Methode der Pivaloyl-Derivatisierung (Beispiel 2).

### Beispiel 18:

### Typische Prozedur für Tabelle 8: Diastereoselektive Reduktion der optisch aktiven freien Mannich-Base (I) [R¹ = o-Nitrophenyl, R² = 2-Pyridyl, R³ = H, R⁴ = 2-Pyridyl, R⁵ = Phenyl, entsprechend einer Verbindung der Formel (XVII)] zum enantiomerenreinen 1,3-Aminoalkohol (XIX) und anschließende Aufarbeitung (Tabelle 8, Nr. 29):

In einem 500 ml-Vierhalsrundkolben mit KPG-Rührer, Tropftrichter und Innenthermometer wurden unter Stickstoff-Atmosphäre 21.39 g (50.39 mmol, 1.0 Äquiv.) der Mannich-Base (XVII) (chem. Reinh. >99%, 95.6% ee, 0.36% H₂O) in 160 ml Toluol suspendiert und mit einem Eisbad auf eine Innentemperatur von +1 °C abgekühlt. Bei dieser Temperatur wurden innerhalb von 25 Min. 10.18 g (125.97 mmol, 2.5 Äquiv.) Boran-Dimethylsulfid-Komplex (94%ig in Dimethylsulfid) zugetropft, wobei die Innentemperatur auf +2°C anstieg. Nach beendeter Zugabe wurde der Ansatz innerhalb von 30 Min. auf +20°C erwärmt und anschließend weiter bei dieser Temperatur gerührt, wobei die gelbe Suspension beige wurde. Eine Reaktionskontrolle nach 15 Min. (HPLC-System wie in Beispiel 12) zeigte den nahezu vollständigen Verbrauch von (XVII) unter Bildung eines Gleichgewichts der entsprechenden Oxazaborinanen der allgemeinen Formel (C) bzw. Oligomere davon an. Nach insgesamt 1.5 Std. Rührzeit bei 20°C wurden unter Eisbadkühlung bei einer Innentemperatur des Reaktionsgemischs zwischen +15°C und +22°C innerhalb von 10 Min. 70 ml Methanol zugetropft. Hierbei wurde eine Gasentwicklung beobachtet. Anschließend wurden bei +20°C Innentemperatur unter Eiskühlung innerhalb von 10 Min. 6.5 ml (100.78 mmol, 2.0 Äquiv.) Methansulfonsäure zugetropft, wobei eine heftige Gasentwicklung und Exothermie beobachtet wurden. Gegen Ende der Zugabe bildete sich eine gelbe Lösung, die bei mittlerer bis hoher Drehzahl bei +40 bis +45°C Innentemperatur gerührt wurde. Nach 1.25 Std. Rührzeit zeigte HPLC-Reaktionskontrolle bei 254 nm insgesamt 6.1 % "Retro-Mannich"-Zersetzungsprodukte, vollständiges Verschwinden der intermediären Oxazaborinane und eine Diastereoselektivität der Reduktion von 94.3 : 5.6 an. Nach insgesamt 1.75 Std. bei 40-45°C wurde der Ansatz am Rotationsverdampfer bei +40°C Badtemperatur / 350 bis 150 mbar eingeengt, wobei 78 ml Destillat (Methanol, Trimethylborat, etwas Toluol) entfernt wurden. Das resultierende Zweiphasengemisch (Toluol und abgeschiedenes gelbes Öl) wurde mit 30 ml 2N Salzsäure versetzt und extrahiert. Die gelbe, saure wäßrige Phase wurde abgetrennt und die Toluolphase erneut mit 5 ml 2N Salzsäure plus 10 ml Wasser extrahiert. Danach enthielt die Toluolphase gemäß HPLC kein Produkt (XIX) mehr und wurde verworfen. Die vereinigten sauren, wäßrigen, produkthaltigen Wasserphasen wurden in 200 ml 1-Butanol gelöst und innerhalb von 10 Min. bei +20°C Innentemperatur in einem 500ml-Vierhalskolben mit KPG-Rührer und Tropftrichter mit 95 ml (190 mmol, 3.77 Äquiv.) 2N Natronlauge versetzt, wobei eine orange-gelbe Emulsion entstand, die 5 Min. nachgerührt wurde. Die produkthaltige, orange-gelbe Butanolphase (oben) wurde von der farblosen, klaren Wasserphase (unten, pH 10) abgetrennt und am Rotationsverdampfer bei +50°C Badtemperatur und 250 bis 45 mbar 85 ml 1-Butanol / Wasser azeotrop abdestilliert. Die erhaltene konzentrierte Lösung von (XIX) in Butanol wurde in einem 500 ml Vierhalskolben mit KPG-Rührer, Tropftrichter und Innenthermometer unter Stickstoff auf eine Innentemperatur von +45°C erwärmt und innerhalb von 5 Min. per Tropftrichter mit 11.1 ml (110 mmol, 2.18 Äquiv.) 30%iger Salzsäure versetzt, wobei die Innentemperatur auf +48°C anstieg und eine gelbe Lösung entstand. Diese wurde innerhalb von 1 Std.. auf +20°C Innentemperatur abgekühlt, wobei die Kristallisation des weißen Dihydrochlorid einsetzte und eine breiige Suspension entstand. Anschließend wurde der Ansatz innerhalb von 10 Min. weiter auf +5°C abgekühlt und 15 Min. bei dieser Temperatur nachgerührt. Es erfolgte Filtration der viskosen Suspension über einen Büchner-Trichter wobei ein weißer Filterkuchen und ein gelbes Filtrat erhalten wurden. Der Filterkuchen wurde mit 2 x 20 ml 1-Butanol gewaschen, trockengesaugt und dann im Vakuumtrockenschrank bei 40°C / 100 mbar getrocknet. Erhalten wurden 20.22 g (40.48 mmol berechnet als (XIX) x 2 HCl) weißer kristalliner Feststoff. Gemäß HPLC enthielt er 99.8% (XIX) und <0.1 % des Diastereomeren dia-(XIX). Die Enantiomerenreinheit betrug 100% ee. Gemäß Titration (Säure/Base- sowie Chlorid-Titration) und ¹H-NMR lag (XIX) als Dihydrochlorid vor. Gemäß ¹H-NMR waren 11.5 Gew.% (entsprechend 87.5 Mol%) 1-Butanol enthalten. Das Butanol ließ sich auch beim längeren Trocknen bei 40-50°C im HV nicht entfernen. Dieses Verhalten wurde bei allen Dihydrochloriden der Tabelle 8 beobachtet, die aus 1-Butanol gefällt worden waren. Die Butanol-Gehalte lagen durchweg bei 85-97 Mol%, so daß man das Produkt als Monobutanol-Solvat von (II)-Dihydrochlorid betrachten kann. Die Ausbeute betrug 80.3% der Theorie, wenn man das Produktgewicht unter Vernachlässigung des Butanol-Gehalts als (XIX)-Dihydrochlorid berechnet und auf das Gewicht des eingesetzten Edukts (XVII) ohne Berücksichtigung von dessen unvollständiger Enantiomerenreinheit (93.4% ee) bezieht [sogenannte telquel-Ausbeute].

### Beispiel 19:

### Diastereoselektive Reduktion der optisch aktiven freien Mannich-Base (I) [R¹ = o-Nitrophenyl, R² = 2-Pyridyl, R³ = H, R⁴ = 2-Pyridyl, R⁵ = Phenyl, entsprechend einer Verbindung der Formel (XVII)] zum enantiomerenreinen 1,3-Aminoalkohol (XIX); Verwendung von Boran-Dimethylsulfid- Komplex als Reduktionsmittel gemäß Tabelle 8, Nr. 33; optimierte Aufarbeitung.

In einem 1L-Vierhalsrundkolben mit KPG-Rührer, Tropftrichter und Innenthermometer wurden unter Stickstoff-Atmosphäre 63.63 g (150 mmol, 1.0 Äquiv.) der Mannich-Base (XVII) (chem. Reinh. >99%, 93.4% ee, 0.02% H₂O) in 400 ml Toluol suspendiert und mit einem Eisbad auf eine Innentemperatur von +1°C abgekühlt. Bei dieser Temperatur wurden innerhalb von 15 Min. 31.60 g (391.1 mmol, 2.6 Äquiv.) Boran-Dimethylsulfid-Komplex (94%ig in Dimethylsulfid) zugetropft, wobei die Innentemperatur auf +4°C anstieg. Nach beendeter Zugabe wurde der Ansatz innerhalb von 30 Min. auf +20°C erwärmt und anschließend weiter bei dieser Temperatur gerührt, wobei die gelbe Suspension beige wurde. Eine Reaktionskontrolle nach 2.5 Std. (HPLC-System wie in Beispiel 12) zeigte den nahezu vollständigen Verbrauch von (XVII) unter Bildung eines Gleichgewichts von Oxazaborinanen an. Nach insgesamt 4 Std. Rührzeit bei 20°C wurden unter Eisbadkühlung bei einer Innentemperatur des Reaktionsgemischs zwischen +15°C und +22°C innerhalb von 10 Min. 190 ml Methanol zugetropft. Hierbei wurde eine Gasentwicklung beobachtet. Anschließend wurden, ebenfalls in einem Innentemperaturintervall von +15°C bis +22°C innerhalb von 20 Min. 31.1 ml (478.9 mmol, 3.19 Äquiv.) Methansulfonsäure zugetropft, wobei eine heftige Gasentwicklung beobachtet wurde. Nachdem 2/3 der gesamten Säuremenge eingetragen waren, wurde eine gelbe Lösung erhalten. Nach beendeter Zugabe wurde mit weiteren 53 ml Methanol nachgespült und bei +20°C bis +22°C gerührt. Nach 1 Std. Rührzeit zeigte HPLC-Reaktionskontrolle bei 254 nm insgesamt 5.4% Mannich Base (XVII) und "Retro-Mannich"-Zersetzungsprodukte, 5.3% dia-(XIX) und 88.4% (XIX) an, sowie vollständiges Verschwinden der intermediären Oxazaborinane. Die Diastereoselektivität in der rohen Reaktionslösung betrug somit 94.4 : 5.6 . Der Ansatz wurde über Nacht bei Raumtemperatur (+18 - +22°C Innentemperatur) gerührt und am nächsten Tag am Rotationsverdampfer bei +40°C Badtemperatur bei 400 bis 150 mbar auf ein Endvolumen von 380 ml eingeengt, wobei Methanol, Trimethylborat und ein Teil des Toluols entfernt wurden. Das resultierende Zweiphasengemisch wurde bei +10°C bis +25°C Innentemperatur mit 212 ml Wasser versetzt. Nachdem 5 Min. nachgerührt wurde, erfolgte eine Phasentrennung. Die Toluolphase wurde verworfen. Die gelbe, saure, produkthaltige Wasserphase (ca. 330 ml) wurde in 303 ml 1-Butanol gelöst und innerhalb von 10 Min. bei +10°C bis +15°C Innentemperatur in einem 1L-Vierhalskolben mit KPG-Rührer und Tropftrichter mit 61.72 g (509.2 mmol, 3.39 Äquiv.) 33%iger Natronlauge versetzt, wobei eine orange-gelbe Emulsion erhalten wurde. Nach beendeter Zugabe wurde der Ansatz 5 Min. nachgerührt. Die produkthaltige, orange-gelbe Butanolphase (ca. 390 ml, oben) wurde von der fast farblosen, klaren Wasserphase (unten, ca. pH 9) abgetrennt und am Rotationsverdampfer bei +50°C Badtemperatur und 300 bis 50 mbar so eingeengt, daß 115 ml Destllat (1-Butanol / Wasser) azeotrop entfernt wurden. Die erhaltene konzentrierte Lösung von (XIX) in Butanol wurde in einem 500 ml Vierhalskolben mit KPG-Rührer, Tropftrichter und Innenthermometer unter Stickstoff auf eine Innentemperatur von +49°C erwärmt und innerhalb von 5 Min. per Tropftrichter mit 39.24 g (322.9 mmol, 2.15 Äquiv.) 30%iger Salzsäure versetzt, wobei die Innentemperatur auf +53°C anstieg und eine gelbe Lösung entstand. Diese wurde innerhalb von 15 Min. auf +20°C Innentemperatur abgekühlt, wobei die Kristallisation des weißen Dihydrochlorid einsetzte und eine breiige Suspension entstand. Nach 30 Min. Rührzeit bei +20°C wurde der Ansatz innerhalb von 30 Min. auf +1°C abgekühlt und 1 Std. bei dieser Temperatur nachgerührt. Es erfolgte Filtration über einen Büchner-Trichter wobei ein weißer Filterkuchen und ein gelbes Filtrat erhalten wurden. Der Filterkuchen wurde mit 2 x 60 ml 1-Butanol gewaschen, trockengesaugt und dann im Vakuumtrockenschrank bei 40°C und 50 mbar unter einem leichten Stickstoffstrom getrocknet. Erhalten wurden 62.7 g (125.55 mmol) (XIX) x 2 HCl) als weißer kristalliner Feststoff. Gemäß HPLC enthielt er 99.68% (XIX) und 0.14% des Diastereomeren dia-(XIX). Die Enantiomerenreinheit betrug 100% ee. Gemäß Titration und ¹H-NMR lag (XIX) als Dihydrochlorid vor. Gemäß ¹H-NMR waren 11.5 Gew.% (entsprechend 87.5 Mol%) 1-Butanol enthalten. Die Ausbeute betrug 83.7% der Theorie, wenn man das Produktgewicht (62.7 g) unter Vernachlässigung des Butanol-Gehalts als (XIX)-Dihydrochlorid berechnet und auf das Gewicht des eingesetzten Edukts (XVII) ohne Berücksichtigung von dessen unvollständiger Enantiomerenreinheit (93.4% ee) bezieht [sogenannte telqel-Ausbeute]. Berücksichtigt man den Butanol-Gehalt von (XIX)-Dihydrochlorid und zieht vom eingesetzten Edukt (XVII) dessen racemischen Anteil (6.6%), der bei der Aufarbeitung entfernt wurde, ab, dann betrug die Ausbeute 79.4% der Theorie. Bezieht man die um Butanol korrigierte Ausbeute auf das gesamte Edukt (XVII), dann betrug die Ausbeute 74.1%.

### Beispiel 20:

### Diastereoselektive Reduktion der optisch aktiven freien Mannich-Base (I) [R¹ = o-Nitrophenyl, R² = 2-Pyridyl, R³ = H, R⁴ = 2-Pyridyl, R⁵ = Phenyl, entsprechend einer Verbindung der Formel (XVII)] zum enantiomerenreinen 1,3-Aminoalkohol (XIX) mit in situ aus Chlortrimethylsilan und Natriumborhydrid erzeugtem Boran (Tabelle 8, Nr. 34) In einem 500 ml-Vierhalsrundkolben mit KPG-Rührer, Rückflußkühler, Innenthermometer und Septum wurden 1,70 g (45.0 mmol, 3,0 Äquiv.)

Natriumborhydrid in 215 ml Tetrahydrofuran suspendiert. Nach Zugabe von 4,89 g (45.0 mmol, 3,0 Äquiv.) Chlortrimethylsilan (per Spritze) wurde die Suspension bei mittlerer bis hoher Drehzahl 45 Min. bei 50°C Innentemperatur gerührt, wobei ein feinkristalliner weißer Feststoff ausfiel. Anschließend wurde die Suspension auf +1°C abgekühlt und innerhalb von 5 Min. mit 6,36 g (15,0 mmol, 1,0 Äquiv.) der Mannich-Base (XVII) versetzt, wobei die Innentemperatur auf +3°C anstieg und eine blaßgelbe Suspension erhalten wurde. Der Ansatz wurde innerhalb von 15 Min. auf 20°C erwärmt und bei dieser Temperatur weiter gerührt. Eine HPLC-Kontrolle nach 30 Min. zeigte nahe vollständige Umsetzung von (XVII) zum Oxazaborinan (C) an. Nach insgesamt 2 Std. Rührzeit bei 20°C wurden dem Ansatz bei 10 bis 15°C innerhalb von 5 Min. 25 ml Methanol zugetropft. Anschließend wurden innerhalb 5 Min. 3,1 ml (47,9 mmol, 3,19 Äquiv.) Methansulfonsäure zugesetzt. Anschließend wurde der Ansatz bei 20°C Innentemperatur nachgerührt. Eine HPLC-Kontrolle nach 15 Min. zeigte 23% (XVII) und 72% (C). Nach 30 Min. Rührzeit wurden dem Ansatz bei 20°C weitere 50 ml Methanol und 3,1 ml (47,9 mmol, 3,19 Äquiv.) Methansulfonsäure zugesetzt. Anschließend wurde der Ansatz bei 40-43 °C Innentemperatur gerührt. Eine weitere HPLC-Kontrolle nach 30 Min. zeigte die vollständige Umsetzung von (C) zu (XIX) (85,1%), dia-(XIX) (5,4%), sowie (XVII) und Retro-Mannich-Zersetzungsprodukten (zusammen 8,5%) an. Nach insgesamt 1 Std. Rührzeit bei 40-43°C wurde die gelbe Suspension zur Entfernung von Salzen filtriert und das Filtrat am Rotationsverdampfer bei 40°C und 400 bis 20 mbar vollständig eingeengt. Das zurückbleibende gelbe, viskose Öl wurde in 50 ml Wasser und über Nacht bei +4°C gelagert. Die wäßrige Produktphase wurde in einem 250 ml Vierhalsrundkolben mit KPG-Rührer, Tropftrichter und Innenthermometer unter Stickstoff in 60 ml 1-Butanol gelöst und bei 15 bis 22°C innerhalb 5 Min. mit 11,96 g (98,7 mmol, 6,58 Äquiv.) 33%iger wäßriger Natronlauge versetzt. Die orange-gelbe Suspension wurde 5 Min. gerührt und die gelbe Butanolphase von der farblosen wäßrigen Phase (pH 13-14) getrennt. Die Butanolphase wurde bei 50°C und 200 bis 20 mbar so eingeengt, daß 22 ml Destillat (Butanol / Wasser) azeotrop entfernt wurden. Die erhaltene konzentrierte butanolische Lösung wurde in einem 100 ml Vierhalskolben mit KPG-Rührer, Tropftrichter und Innenthermometer unter Stickstoff auf 47°C Innentemperatur erwärmt und innerhalb von 5 Min. mit 4,00 g (33,0 mmol, 2,20 Äquiv.) Salzsäure versetzt, wobei die Innentemperatur auf 50°C anstieg und eine klare orange-rote Lösung erhalten wurde. Innerhalb von 15 Min. wurde sie auf 15°C abgekühlt, wobei Kristallisation des weißen Dihydrochlorids einsetzte und eine breiige Suspension erhalten wurde. Nach 30 Min. Rührzeit wurde der Ansatz innerhalb 15 Min. weiter auf 1°C abgekühlt und eine Stunde bei dieser Temperatur nachgerührt. Der Niederschlag wurde abgesaugt, mit 2 mal 10 ml Butanol gewaschen und bei 40°C und 50 mbar unter einem leichten Stickstoffstrom getrocknet. Erhalten wurden 5,60 g (11,21 mmol, 74,8% der Theorie) weißer Feststoff, der gemäß HPLC >99% ee hatte, zu 99,6% aus (XIX) und zu 0,2% aus dia-(XIX) bestand. Ein ¹H-NMR zeigte einen 1-Butanol-Gehalt von 12,0 Gew.-% an. Der Wassergehalt (Karl-Fischer-Titration) betrug 0,99%. Die Chloridtitration ergab 1,97 Äquiv. Chlorid-Ionen pro Mol (XIX).

### Beispiel 21:

### Freisetzung der Mannich-Base (I) [R¹ = o-Nitrophenyl, R² = 2-Pyridyl, R³ = H, R⁴ = 2-Pyridyl, R⁵ = Phenyl, entsprechend einer Verbindung der Formel (XVII)] aus dem Mandelat-Salz (XVIII) [Y^{*} = (S)-(+)-Mandelsäuresalz] mit NaHCO₃ in Wasser / Aceton gemäß Tabelle 7, Nr. 23:

In einem 2-Liter-Doppelmantelreaktor (angeschlossen an einen Umlaufkryostaten) mit Temperaturfühler und mechanischem Turbinenrührer wurden bei Raumtemperatur unter Stickstoffatmosphäre und unter Rühren 228,6 g (396,6 mmol, 1,0 Äquiv.) Mandelat-Salz (XVIII) (95,6% ee der enthaltenen Mannich-Base (XVII)) in 1143 ml Wasser suspendiert. Die weiße Suspension wurde dann auf +10°C Innentemperatur abgekühlt. 66,64 g (793,24 mmol, 2,0 Äquiv.) Natriumhydrogencarbonat wurden zugegeben, gefolgt nach 5 Min. von 114 ml Aceton. Die sich langsam gelb färbende Suspension wurde bei +10°C Innentemperatur gerührt. Die Umsatzkontrolle erfolgte durch Probenahme, Filtration, ¹H-NMR des Feststoffs. Nach 4,5 Stunden waren noch 15.4% Mandelsäure vorhanden, nach 7,4 Stunden noch 9,1%. Nach Rühren über Nacht wurde keine Mandelsäure mehr detektiert. Die Suspension wurde abgesaugt und der Filterkuchen 3 mal mit je 50 ml Wasser gewaschen. Der Feststoff wurde im Vakuumtrockenschrank bei 40°C und ca. 50 mbar getrocknet. Erhalten wurden 168,25 g (396,4 mmol, 99,95% der Theorie) der freien Mannich-Base (XVII) als gelbes Pulver, 96,8% ee (Camph-Methode gemäß Beispiel 1) bzw. 96,2% ee (Piv-Methode gemäß Beispiel 2), Schmp. 153-154°C, Restwassergehalt gemäß Karl-Fischer-Titration: 0,32 Gew.-%. ¹H-NMR und HPLC bestätigten, daß es sich um eine einheitliche Verbindung handelte, die keine Mandelsäure mehr enthielt. ¹H-NMR zeigte außerdem, daß der Gehalt am anti-Diastereomer von (XVII) unter 1% lag.

### Beispiel 22:

### Freisetzung der Mannich-Base (I) [R¹ = o-Nitrophenyl, R² = 2-Pyridyl, R³ = H, R⁴ = 2-Pyridyl, R⁵ = Phenyl, entsprechend einer Verbindung der Formel (XVII)] aus dem Mandelat-Salz (XVIII) [Y^{*} = (S)-(+)-Mandelsäuresalz] mit 2N Natronlauge bei pH-stat 8,5 in Wasser / Ethanol gemäß Tabelle 7, Nr. 21

Die Reaktion wurde in einem 10-Liter-Doppelmantelreaktor (angeschlossen an einen Umlaufkryostaten) mit Temperaturfühler und mechanischem Glocken-Rührer durchgeführt, an den ein Autotitrator Metrohm STAT-Titrino 718 angeschlossen war. Der Autotitrator war mit 1150 ml 2,00 N Natronlauge befüllt, wurde durch eine in die Reaktionssuspension eintauchende Glaselektrode gesteuert und war auf folgende Parameter eingestellt: maximale Dosierrate 20 ml/min, minimale Dosierrate 4 ml/min, Zeitintervall der Aufzeichnung alle 60 Sek., pHₘₐₓ 8,5. Die Eintropfspitze des Autotitrators tauchte in die Reaktionssuspension ein. Die Manteltemperatur des Reaktors wurde so gesteuert, daß die Temperatur der Reaktionssuspension im Bereich 20-25°C gehalten wurde.

Bei Raumtemperatur wurde unter Stickstoffatmosphäre und unter Rühren 1311,3 g (2,274 Mol, 1,0 Äquiv.) Mandelat-Salz (XVIII) (94,4% ee der enthaltenen Mannich-Base (XVII), ca. 1,3% Gehalt an anti-Diastereomer von (XVII)) in 5686 ml Wasser suspendiert und 569 ml Ethanol (mit Methylethylketon vergällt) zugefügt. Der pH-Wert dieser Suspension (vor Titrationsbeginn) betrug 4,8 . Nach Einschalten des Titrators erreichte der pH-Wert kurzfristig maximal pH 9,7. Bereits nach 30 Sek. hatte sich die Reaktionssuspension von blaßgelb nach kräftig gelb verfärbt. Die anfangs hohe Dosiergeschwindigkeit verlangsamte sich mit der Zeit zusehends. Nach 4 Stunden waren 92% der theoretischen Menge Natronlauge zudosiert. Der Ansatz rührte über Nacht unter pH-Stat-Bedingungen (pH 8,5). Am nächsten Morgen war die Dosierung zum Erliegen gekommen. Der pH-Wert der Suspension betrug 8,72 und es waren insgesamt 1139,6 ml (100,2% der Theorie) zutitriert worden. Die Suspension wurde abgesaugt und der Filterkuchen mit 4 mal 500 ml Wasser gewaschen. Der Feststoff wurde im Vakuumtrockenschrank im Stickstoffstrom 28 Stunden bei 40°C und ca. 100 mbar, dann 70 Stunden bei 25°C und 100 mbar und abschließend noch 20 Stunden bei 40°C im HV (10⁻² mbar) getrocknet. Erhalten wurden 960,9 g (2,26 Mol, 99,5% der Theorie) der freien Mannich-Base (XVII) als feines hellgelbes Pulver, 95,6% ee (Piv-Methode gemäß Beispiel 2), Schmp. 150-152°C, Restwassergehalt gemäß Karl-Fischer-Titration: 0,35 Gew.-%. ¹H-NMR und HPLC bestätigten, daß es sich um eine einheitliche Verbindung handelte, die keine Mandelsäure mehr enthielt. ¹H-NMR zeigte außerdem, daß der Gehalt am anti-Diastereomer von (XVII) bei ca. 1,2% lag.

### Beispiel 23:

### Diastereoselektive Reduktion des optisch aktiven Mandelat-Salzes (III) [R¹ = o-Nitrophenyl, R² = 2-Pyridyl, R³ = H, R⁴ = 2-Pyridyl, R⁵ = Phenyl, HY^{*} = (S)-(+)-Mandelsäure, entsprechend einer Verbindung der Formel (XVIII)] zum 1,3-Aminoalkohol (XIX) gemäß Tabelle 9, Nr. 18; Solvolyse des Oxazaborinans mit Salzsäure.

In einem 1L-Vierhalsrundkolben mit KPG-Rührer, Tropftrichter mit aufgesetztem Blasenzähler, Innenthermometer und Stickstoffzuleitung wurden 30,0 g (52,0 mmol, 1,0 Äquiv.) des Mandelat-Salzes (XVIII) (96,5% ee der enthaltenen Mannich-Base (XVII)) in 400 ml THF suspendiert und mittels Eisbad auf +1 °C abgekühlt. Innerhalb von 10 Min. wurden unter Stickstoffatmosphäre 15,5 ml (156 mmol, 3,0 Äquiv.) Boran-Dimethylsulfid-Komplex (95% ig) bei einer Reaktionstemperatur von +1 bis +3°C zugetropft. Nach beendeter Zugabe wurde das Eisbad entfernt und das Reaktionsgemisch innerhalb 15 Min. auf 23°C gebracht, dann 1,5 Stunden nachgerührt. Probenahme / HPLC-Analyse zeigte, daß die Umsetzung von (XVIII) zum Oxazaborinan (C) bereits nach 1 Stunde vollständig war. Das Reaktionsgemisch wurde wieder mit dem Eisbad auf 1 °C abgekühlt und dann bei maximal 12°C Innentemperatur langsam 25 ml Wasser zugetropft. Dabei trat starke Gasentwicklung auf und die Lösung wurde schwachgelb. Es wurde solange bei Raumtemperatur nachgerührt, bis die Gasentwicklung beendet war (30 Min.). Dabei fiel einer weißer Niederschlag aus. Bei 40°C und ca. 100 mbar wurde das THF aus dem Reaktionsgemisch abdestilliert. Gegen Ende der Destillation wurde für 5 Min. volles Wasserstrahlvakuum (ca. 20 mbar) angelegt. Nach dem Abkühlen auf +5°C wurden bei max. 20°C Innentemperatur des Reaktionsgemischs langsam 200 ml (2400 mmol) konz. Salzsäure (37% ig) zugetropft, dann 1 Stunde bei 40°C gerührt. Der 1,3-Aminoalhohol (XIX) ging dabei als Hydrochlorid in Lösung und Borsäure fiel aus. Die Suspension wurde über Nacht bei 4°C im Kühlschrank stehen gelassen um die Kristallisation zu vervollständigen. Die Borsäure wurde abgesaugt und mit 40 ml Wasser gewaschen. Sie wog nach der Trocknung im Vakuum 7,23 g (116,9 mmol, 75% der Theorie). Das saure Filtrat hatte ein Gesamtvolumen von 250 ml. In einem 1 L-Vierhalskolben mit KPG-Rührer und Tropftrichter wurden 96 g (2400 mmol) Natronlauge in 520 ml Wasser gelöst, auf 13°C abgekühlt und dazu, innerhalb von 60 Min. bei max. 15°C Innentemperatur das obige saure Filtrat langsam zugetropft. Der rohe 1,3-Aminoalkohol (XIX) fiel dabei grobkristallin aus. Die Suspension wurde 1 Stunde bei Raumtemperatur nachgerührt, der Niederschlag abgesaugt und mit 250 ml Wasser nachgewaschen (Der Niederschlag der beim Einlaufen des Waschwassers ins Filtrat entstand, bestand überwiegend aus polaren Verunreinigungen und wurde deshalb verworfen). Das rohe (XIX) wurde im Vakuumtrockenschrank bei 40°C und ca. 100 mbar getrocknet. Erhalten wurden 20,7 g (48,54 mmol, 93,4% der Theorie) blaßgelber Feststoff. Er wurde in 100 ml Diisopropylether suspendiert und 1 Stunde bei 55°C heftig verrührt. Der Feststoff wurde abgesaugt, mit 100 ml Diisopropylether nachgewaschen und im Vakuum bei 40°C und ca. 100 mbar getrocknet. Erhalten wurden 17,5 g (41,0 mmol, 78,9% der Theorie) blaßgelbes Pulver, das gemäß HPLC-Analyse 95%ig war, 3,1% des Diastereomeren dia-(XIX) und 1,8% Nebenprodukte enthielt.

### Beispiel 24:

### Diastereoselektive Reduktion eines optisch aktiven Mannich-Salzes (III) [R¹ = o-Nitrophenyl, R² = 2-Pyridyl, R³ = H, R⁴ = 2-Pyridyl, R⁵ = Phenyl, HY^{*} = (+)-DPWS] zum 1,3-Aminoalkohol der allgemeinen Formel (II), entsprechend einer Verbindung der Formel (XIX) gemäß Tabelle 10, Nr. 5; Solvolyse des Oxazaborinans mit Salzsäure.

In einem 250ml-Vierhalsrundkolben mit KPG-Rührer, Septum, Blasenzähler, Innenthermometer und Stickstoffzuleitung wurden 10,0 g (8,57 mmol; gemäß ¹H-NMR-Bestimmung des Verhältnisses der Verbindung (XVII) zu DPWS, 16,08 mmol (XVII) enthaltend; 1,0 Äquiv.) des DPWS-Salzes (III) (95,1% ee der enthaltenen Mannich-Base (XVII)) in 100 ml THF suspendiert, anschließend auf 0 bis 5°C Innentemperatur abgekühlt. Unter Stickstoff wurden 7,63 ml (80.45 mmol, 5,0 Äquiv.) Boran-Dimethylsulfid-Komplex (95%ig) per Spritze innerhalb 15 Min. zugetropft. Dann wurde das Eisbad entfernt und die Suspension auf Raumtemperatur erwärmt. Nach 20 Min. bei Raumtemperatur war eine klare Lösung entstanden. Probenahme und HPLC-Analyse zeigte, daß sich (III) quantitativ zum Oxazaborinan (C) umgesetzt hatte und daß nur wenig Nebenprodukte entstanden waren. Innerhalb 15 Min. wurden 45 ml Wasser zugetropft (Gasentwicklung, starkes Schäumen), wobei die Innentemperatur auf 40°C anstieg. Innerhalb 15 Min. wurden 10 ml 37%ige Salzsäure zugetropft, dann die Innentemperatur auf 60°C erhöht. Nach 15 Min. bei 60°C zeigte HPLC-Analyse keine Borverbindungen mehr an und es war (XIX) als Hauptprodukt entstanden. Mit 30 ml 33%iger Natronlauge wurde der pH-Wert auf 13 gestellt, das Reaktionsgemisch dann auf Raumtemperatur abgekühlt und mit 2 mal 100 ml Dichlormethan extrahiert. Die vereinigten organischen Extrakte wurden im Vakuum zur Trockne eingeengt und der Rückstand (fester Schaum) im Vakuumtrockenschrank bei 40°C und 50 mbar getrocknet. Erhalten wurden 8,11g blaßgelbes Pulver, das gemäß HPLC-Assay einen Gehalt von 75.1 % bezogen auf einen reinen Referenzstandard von (XIX) hatte. Die Ausbeute an (XIX) betrug somit 6,09 g (14,28 mmol, 88.8% der Theorie). Die HPLC-100%-Reinheit war 94,8%, das Verhältnis (XIX) / dia-(XIX) 97,8 : 2,2 , die Enantiomerenreinheit 96,8% ee.

### Beispiel 25:

### Diastereoselektive Reduktion eines optisch aktiven Mannich-Salzes (III) [R¹ = o-Nitrophenyl, R² = 2-Pyridyl, R³ = H, R⁴ = 2-Pyridyl, R⁵ = Phenyl, HY^{*} = (+)-DPWS] zum 1,3-Aminoalkohol der allgemeinen Formel (II), entsprechend einer Verbindung der Formel (XIX) gemäß Tabelle 10, Nr. 3; Solvolyse des Oxazaborinans mit Kalilauge.

In einem 250ml-Vierhalsrundkolben mit KPG-Rührer, Septum, Blasenzähler, Innenthermometer und Stickstoffzuleitung wurden 10,0 g (8,57 mmol; gemäß ¹H-NMR-Bestimmung des Verhältnisses von (XVII) zu DPWS 16,08 mmol (XVII) enthaltend; 1,0 Äquiv.) des DPWS-Salzes (III) (95,1% ee der enthaltenen Mannich-Base (XVII)) in 100 ml THF suspendiert, anschließend auf 0 bis 5°C Innentemperatur abgekühlt. Unter Stickstoff wurden 7,63 ml (80.45 mmol, 5,0 Äquiv.) Boran-Dimethylsulfid-Komplex (95%ig) per Spritze innerhalb 15 Min. zugetropft. Das Eisbad wurde entfernt und das Reaktionsgemisch unter Erwärmung auf Raumtemperatur rühren gelassen. Nach 30 Min. war eine klare Lösung entstanden. Probenahme und HPLC-Analyse zeigte die vollständige Umwandlung des Edukts zu 91% Oxazaborinan und 9% (XIX) an. Innerhalb 15 Min. wurden 45 ml Wasser, gefolgt innerhalb 15 Min. von 45 ml 20%iger wäßriger Kaliumhydroxid-Lösung, zugetropft. Dabei trat Gasentwicklung und starkes Schäumen ein und die Innentemperatur stieg auf 40°C an. Das Reaktionsgemisch wurde auf 60°C geheizt und die Solvolyse des Oxazaborinans zum 1,3-Aminoalkohol (XIX) per HPLC-Monitoring verfolgt. Nach 3 Stunden bei 60°C betrug das Verhältnis (C) / (XIX) 53.3 : 46,7 , nach 10 Stunden 19,4 : 80,6 , nach 16 Stunden 6,9 : 93,1. Die Solvolyse wurde an dieser Stelle abgebrochen und das Reaktionsgemisch auf Raumtemperatur abgekühlt. Es wurde mit 2 mal 100 ml Dichlormethan extrahiert und die vereinigten organischen Extrakte mit 50 ml gesättigter Kochsalzlösung gewaschen. Die Dichlormethanlösung wurde dann im Vakuum zur Trockne eingeengt und der Rückstand im Vakuum bei 40°C und 50 mbar getrocknet. Erhalten wurden 7,05 g blaßgelbes Pulver, das gemäß HPLC-Assay einen Gehalt von 77.2% bezogen auf einen reinen Referenzstandard von (XIX) hatte. Die Ausbeute an (XIX) betrug somit 5,44 g (12,76 mmol, 79,3% der Theorie). Die HPLC-100%-Reinheit war 93,0%, das Verhältnis (XIX) / dia-(XIX) 98,5 : 1,5 , die Enantiomerenreinheit 95,2% ee. 5,5% Oxazaborinan (C) lagen noch unsolvolysiert vor.

### Beispiel 26:

### Diastereoselektive Reduktion eines optisch aktiven Mannich-Salzes (III) [R¹ = o-Nitrophenyl, R² = 2-Pyridyl, R³ = H, R⁴ = 2-Pyridyl, R⁵ = Phenyl, HY^{*} = (+)-DPWS] zum 1,3-Aminoalkohol der allgemeinen Formel (II), entsprechend einer Verbindung der Formel (XIX) gemäß Tabelle 10, Nr. 9; Solvolyse des Oxazaborinans mit Methanol / Methansulfonsäure.

In einem 250ml-Vierhalsrundkolben mit Magnetrührer, Septum, Blasenzähler, Innenthermometer und Stickstoffzuleitung wurden 15,33 g (13,13 mmol; gemäß ¹H-NMR-Bestimmung des Verhältnisses von (XVII) zu DPWS 25,30 mmol (XVII) enthaltend; 1,0 Äquiv.) des DPWS-Salzes (III) (93,2% ee der enthaltenen Mannich-Base (XVII)) in 125 ml THF suspendiert, anschließend auf 0 bis 5°C Innentemperatur abgekühlt. Unter Stickstoff wurden 4,86 g (63.94 mmol, 2,5 Äquiv.) Boran-Dimethylsulfid-Komplex (95%ig) per Spritze innerhalb 15 Min. zugetropft. Das Eisbad wurde entfernt und das Reaktionsgemisch unter Erwärmung auf Raumtemperatur rühren gelassen. Nach 45 Min. war eine klare Lösung entstanden. Nach 2 Std. war per HPLC kein Edukt mehr nachweisbar. Bei 5°C wurden 20,9 g Methanol innerhalb 15 Min. zugetropft, unmittelbar gefolgt von 4,92 g Methansulfonsäure. Die gelbe Lösung wurde auf 35°C Innentemperatur erwärmt und die Solvolyse des Oxazaborinans (C) per HPLC-Moniotoring verfolgt. Nach 4,5 Std, wurden 3,7% (C), 94,2% (XIX) und 2,1% des Diastereomeren dia-(XIX) angezeigt. Nach 6,5 Std. bei 35°C und stehen der Lösung über Nacht bei Raumtemperatur wurden 1,8% (C), 96,9% (XIX) und 1,8% dia-(XIX) detektiert. Die klare, gelbe Lösung wurde im Vakuum am Rotationsverdampfer auf einen Rückstand von 22,95 g eingeengt (gelbes Öl plus Feststoff) und in 15 ml Methanol klar gelöst (Ultraschallbad, 35°C). Diese hochkonzentrierte Methanollösung wurde innerhalb 15 Min. in die Lösung von 10 ml 25%iger Ammoniaklösung in 75 ml Wasser (25°C) getropft, wobei (XIX) sofort ausfiel. Die Suspension wurde 1 Stunde bei Raumtemperatur gerührt, dann abgesaugt. Dieses Rohprodukt hatte gemäß HPLC-Assay gegen einen reinen Referenzstandard von (XIX) einen Gehalt von 88% und ein Verhältnis von (XIX) / dia-(XIX) von 98,1 : 1,9. Es wurde in einer Lösung von 1 ml konz. Ammoniaklösung in 75 ml Wasser resuspendiert und zwei Stunden heftig bei Raumtemperatur verrührt, dann abgesaugt und bei 45°C und 150 mbar getrocknet. Erhalten wurden 11,0 g
(25,79 mmol, 101,9 % der Theorie) hellgelbes Pulver, das gemäß HPLC-Assay 96,1%ig gegen Standard war (d.h. korrigierte Ausbeute: 97,9% der Theorie); 93,2% ee und ein unverändertes Verhältnis (XIX) / dia-(XIX) von 98,1 : 1,9 hatte. Dieses grob gereinigte (XIX) wurde in 66 ml siedendem Diisopropylether 30 Min. heftig verrührt, eine weitere Stunde unter Eisbadkühlung gerührt, dann abgesaugt und bei 50°C im HV (10⁻² mbar) getrocknet. Erhalten wurden 9,50 g (22,28 mmol, 88,1 % der Theorie) hellgelbes Pulver, das gemäß HPLC-Assay 97,9%ig gegen Standard war (d.h. korrigierte Ausbeute: 86,2% der Theorie), 95,2% ee und ein Verhältnis (XIX) / dia-(XIX) von 99,2 : 0,8 hatte.

### Beispiel 27:

### Synthese des optisch aktiven Mannich-Salzes der Formel (III) [R¹ = p-Tolyl, R² = 2-Pyridyl, R³ = H, R⁴ = 2-Pyridyl, R⁵ = Phenyl] durch Vierkomponenten-Kupplung unter dynamischer Racematspaltung bei Raumtemperatur ; Verwendung von (S)-(+)-Mandelsäure als chiralem Hilfsstoff [HY* = (+)-MDLS)] und Ethanol als Lösungsmittel:

In einem 100ml-Dreihalskolben mit KPG-Rührer wurden 30 ml Ethanol (vergällt mit Methylethylketon) vorgelegt. Dazu gab man unter N₂-Atmosphäre bei Raumtemperatur (22°C) nacheinander 2,32 g (11,76 mmol, 1,00 Äquiv.) 2-Pyridylmethyl-phenyl-keton, 1,40 g (14,70 mmol, 1,25 Äquiv.) 2-Aminopyridin, 1,75 g (14,11 mmol, 1,20 Äquiv.) 4-Tolylaldehyd, 3,65 g (23,52 mmol, 2,00 Äquiv.) (S)-(+)-Mandelsäure. Der mechanische Rührer wurde eingeschaltet und es bildete sich nach wenigen Minuten eine klare gelbe Lösung. Nach 1 Std. waren erhebliche Mengen Niederschlag entstanden. Die Suspension wurde weiter bei Raumtemperatur gerührt. Nach 40 Std. und 64 Std. Reaktionszeit wurden Proben der Suspension (jeweils ca. 50 mg Niederschlag enthaltend) entnommen und der darin befindliche Niederschlag jeweils abgesaugt. Das syn/anti-Verhältnis wurde 1H-NMR-spektroskopisch (nach dem Auflösen der Probe in DMSO-d6 unmittelbar messen!) bestimmt. Das Diastereomerenverhältnis läßt sich prinzipiell aus den Integralen mehrerer Signale berechnen, am einfachsten aus dem Methyl-Singulett, das für das syn-Isomer bei δ = 2,15 ppm, für das anti-Isomer bei δ = 2,11 ppm liegt. Die optische Reinheit der Mannich-Base wurde, mit der am Ende von Beispiel 27 beschriebenen Prozedur, per Chiralphasen-HPLC-Analyse nach Piv-Derivatisierung bestimmt.
Für beide Proben betrug das aus den NMR-Integralen berechnete syn/anti-Verhältnis 95 : 5. Unter Berichsichtigung des Zeitraums von 3,5 Min., der nach dem Auflösen der Probe fürs Einbringen der Probe ins NMR-Gerät, Proben-Shimmen und
Datenakkumulation benötigt wurde, extrapoliert man aus der Kinetik (Beispiel 28) der syn/anti-Isomerisierung ein ursprüngliches syn/anti-Verhältnis des Niederschlags von >99 : <1. Das Molverhältnis von Mannich-Base zu Mandelsäure betrug in beiden Fällen genau 1:1. Der Enantiomerenüberschuß der Mannich-Base betrug 96,0% ee bei der Probe nach 40 Std. und 97,0 % ee bei der Probe nach 64 Stdunden.
Der Niederschlag des Reaktionsansatzes wurde abgesaugt, mit Mutterlauge, dann wenig Ethanol gewaschen, trockengesaugt und im HV getrocknet. Man erhielt 5,66 g (10,4 mmol, 88,2% d.Th.) blaßgelbes Pulver. Unter Berücksichtigung der beiden vorher entnommenen Proben (ca. 100 mg) betrug die Ausbeute 90% d.Th..
¹H-NMR (400 MHz, DMSO-d6): δ = 2,15 (s, 3H), 5,02 (s, 1H, CHOH des Mandelat-Anions), 5,65 (d, 1H), 5,95 (t, 1H), 6,32 (d, 1H), 6,37 (t, 1H), 6,89 (d, 1H), 6,99 (d, 2H), 7,20 (m, 2H), 7,25 - 7,48 (m, 11H), 7,50 - 7,60 (m, 2H), 7,68 (td, 1H), 7,87 (d, 2H), 7,92 (∼d, 1H), 8,46 (∼d, 1H).
¹³C-NMR (100,62 MHz, DMSO-d6): δ = 20,52 (CH3), 55,20 (CH), 60,55 (CH), 72,44 (CHOH des Mandelat-Anions), 107,84 (CH), 111,87 (CH), 119,10 (CH), 121,80 (CH), 126,60 - 128,70 (12 Signale, CH), 133,13 (CH), 135,40 (C), 136,50 (CH), 136,63 (CH), 138,95 (C), 140,20 (C), 147,25 (CH), 148,87 (CH), 156,10 (C), 157,90 (C), 174,20 (CO2-), 196,8 (C=O).

### Derivatisierung und ee-Bestimmung:

Zu 2-5 mg des Mannich-Salzes in einem Reacti-Vial gibt man 20 µl Pivaloylchlorid, gefolgt von 10 µl Triethylamin. Man beschallt die Lösung 2 Min. im Ultraschallbad. Dann gibt man 500 µl Acetonitril (HPLC grade) hinzu und injiziert 1 µl dieser Lösung auf eine Säule 250 mm x 4,6 mm Chiralpak AS. Isokratische Elution bei 25°C mit 1,0 ml / Min. des Laufmittels 50% Isopropanol / 50% n-Hexan / 0.1% Trifluoressigsäure und UV-Detektion bei 254 nm. Das Hauptisomer (98,5%) wurde mit t(ret) 12,14 Min. eluiert, das Spiegelbild (1,5%) mit t(ret) 7,34 Min.. Eine entsprechend derivatisierte racemische Vergleichsprobe liefert diese beiden Peaks zu je 50%.

### Beispiel 28:

### syn/anti-Isomerisierung des Mannich-Base-Mandelats aus Beispiel 26 in DMSO-d6-Lösung bei 300K. Kinetik und Gleichgewichtslage der Retro-Mannich / Mannich - Reaktionen:

8 mg des Produkts aus Beispiel 27 wurden so rasch wie möglich bei Raumtemperatur in einem ¹H-NMR-Röhrchen in DMSO-d6 gelöst. Die Probe wurde unverzüglich in das NMR-Gerät (400 MHz, 300,0 K) eingebracht, rasch geshimmt und gemessen. Das erste Spektrum wurde 3,5 Min. nach der Probenauflösung erhalten. Es zeigte syn- und anti-Isomer des Mannichsalzes im Verhältnis 95,1 : 4,9 an. Weitere Spektren der Lösung wurden im Abstand von jeweils 3-4 Min. erhalten. Sie zeigten eine kontinuierliche Zunahme des anti-Isomers zu Lasten des syn-Isomers an. Der Verlauf kann der Graphik und der Tabelle der Anlage entnommen werden. 69 Min. nach Auflösung des Mannich-Salzes wurde das NMR-Monitoring bei einem syn/anti-Verhältnis von 50 : 50 abgebrochen. Eine erneute Messung, 20,5 Std. nach Auflösung des Mannich-Salzes, zeigte ein syn/anti-Verhältnis von 41,5 : 58,5 an. Nach insgesamt 44,5 Std. war dieses Verhältnis unverändert. Das thermodynamische Gleichgewicht der beiden Isomeren wird also in weniger als 20 Std. erreicht und bevorzugt in Lösung das anti-Isomer. Hingegen kristallisierte bei der Vierkomponenten-Kupplung (Beispiel 27) fast reines syn-Isomer, offenbar aufgrund geringerer Löslichkeit, aus. Bereits das 3,5 Min. nach Probenauflösung erhaltene Spektrum zeigt (neben syn- und anti-Isomer des Mannich-Salzes) das Vorliegen der Retro-Mannich-Produkte 2-Pyridylmethyl-phenyl-keton (Formel VI ; Singulett bei δ = 4.53 ppm) und Tolylaldehyd (bzw. entsprechendes Imin) (Formel IV bzw. X, Singuletts bei δ = 2,40 und 9,12 ppm) in geringer, aber signifikanter Menge an. Die beste Ausgleichskurve zwischen den Meßpunkten der Graphik wurde durch Polynombildung 3. Ordnung erzielt. Extrapolation dieser Kurve auf den Zeitpunkt t=0 ergibt, daß der Feststoff ein syn / anti - Verhältnis >99 : <1 hatte.

| NMR-Messung Nr. | Zeit nach Proben-auflösung [min] | cis-isomer [%] | trans-isomer [%] |
|---|---|---|---|
| 1 | 3,5 | 95,1 | 4,9 |
| 2 | 6,5 | 92,5 | 7,5 |
| 3 | 10,5 | 89,0 | 11,0 |
| 4 | 13,5 | 85,0 | 15,0 |
| 5 | 17,5 | 81,7 | 18,3 |
| 6 | 20,5 | 77,3 | 22,7 |
| 7 | 24,5 | 76,8 | 23,2 |
| 8 | 27,5 | 71,8 | 28,2 |
| 9 | 31,5 | 68,0 | 32,0 |
| 10 | 34,5 | 66,2 | 33,8 |
| 11 | 37,5 | 63,8 | 36,2 |
| 12 | 41,5 | 61,7 | 38,3 |
| 13 | 44,5 | 60,1 | 39,9 |
| 14 | 48,5 | 58,7 | 41,3 |
| 15 | 51,5 | 56,8 | 43,2 |
| 16 | 55,5 | 54,5 | 45,5 |
| 17 | 58,5 | 53,6 | 46,4 |
| 18 | 61,5 | 52,9 | 47,1 |
| 19 | 65,5 | 52,0 | 48,0 |
| 20 | 68,5 | 50,5 | 49,5 |
| 21 | 1230 | 41,5 | 58,5 |
| 22 | 2670 | 41,6 | 58,4 |

### Beispiel 29:

### Synthese des optisch aktiven Mannich-Salzes der Formel (III) [R¹ = o-Chlorphenyl, R² = 2-Pyridyl, R³ = H, R⁴ = 2-Pyridyl, R⁵ = Phenyl] durch Vierkomponenten-Kupplung unter dynamischer Racematspaltung bei Raumtemperatur ; Verwendung von (S)-(+)-Mandelsäure als chiralem Hilfsstoff [HY* = (+)-MDLS)] und Ethanol als Lösungsmittel:

In einem 50ml-Zweihalskolben mit Magnetrührkern wurden 30 ml Ethanol (vergällt mit Methylethylketon) vorgelegt. Dazu gab man unter N₂-Atmosphäre bei Raumtemperatur (20°C) nacheinander 2,32 g (11,76 mmol, 1,00 Äquiv.) 2-Pyridylmethyl-phenyl-keton, 1,41 g (14,70 mmol, 1,25 Äquiv.) 2-Aminopyridin, 2,03 g (14,11 mmol, 1,20 Äquiv.) 2-Chlorbenzaldehyd, 3,65 g (23,52 mmol, 2,00 Äquiv.) (S)-(+)-Mandelsäure. Der Magnetrührer wurde eingeschaltet und es bildete sich eine gelbe, leicht trübe Lösung. Nach 30 Min. Reaktionszeit hatte die Trübung deutlich zugenommen, nach 1 Std. waren bereits deutliche Mengen Niederschlag entstanden. Der Ansatz rührte bei Raumtemperatur übers Wochenende. Nach insgesamt 3, 4, 5, 6 und 7 Tagen Reaktionszeit wurden Proben (jeweils ca. 50 mg) entnommen. Derivatisierung mit Pivaloylchlorid und HPLC-Analyse, analog zu Beispiel 27, ergab folgende Enantiomerenüberschüsse: 97,2% ee, 97,4% ee, 97,6% ee, 98,2% ee, 98,4% ee. Das Hauptisomer wurde mit t(ret) = 9,38 Min eluiert, das Spiegelbild mit t(ret) = 6,31 Min.. Eine entsprechend derivatisierte racemische Vergleichsprobe liefert diese beiden Peaks zu je 50%.
In ¹H-NMR-Spektren (400 MHz, DMSO-d6) der Proben war das anti-Isomer nicht nachweisbar (d.h. syn/anti >>99:1), ebenso kein o-Chlorbenzaldehyd bzw. dessen Imin. Das Retro-Mannich-Produkt 2-Pyridylmethyl-phenylketon war in Spuren nachweisbar.
Der Niederschlag des Reaktionsansatzes wurde abgesaugt, mit Mutterlauge, dann wenig Ethanol gewaschen, trockengesaugt und im HV getrocknet. Man erhielt 5,77 g (10,19 mmol, 86,7% d.Th.) blaßgelbes Pulver. Unter Berücksichtigung der fünf vorher entnommenen Proben (ca. 250 mg) betrug die isolierte Ausbeute 90.4% d.Th.. ¹H-NMR (400 MHz, DMSO-d6): δ = 5,02 (s, 1H, CHOH des Mandelat-Anions), 5,73 (d, 1H), 6,22 (t, 1H), 6,38 (d, 1H), 6,40 (t, 1H), 6,90 (d, 1H), 7,14 (t, 2H), 7,18 (∼td, 1H), 7,25 - 7,30 (m, 2H), 7,30 - 7,38 (m,3H), 7,38 - 7,45 (m, 4H), 7,45 - 7.57 (m, 3H), 7,67 (td, 1H), 7,87 (m,3H), 8,48 (dd, 1H).
¹³C-NMR (100,62 MHz, DMSO-d6): δ = 52,65 (CH), 58,92 (CH), 72,41 (CHOH des Mandelat-Anions), 107,37 (CH), 112,25 (CH), 122,35 (CH), 124,66 (CH), 126,60 - 129,29 (9 Signale, CH), 132,83 (CH), 133,02 (C), 136,30 (C), 136,71 (CH), 136,77 (CH), 139,68 (C), 140,22 (C), 147,37 (CH), 148,90 (CH), 156,36 (C), 157,44 (C), 174,09 (CO2-), 196,43 (C=O).

### Beispiel 30:

### Synthese des racemischen Mannich-Salzes der Formel (III) [R¹ = Phenyl, R² = 2-Pyridyl, R³ = H, R⁴ = 2-Pyridyl, R⁵ = Phenyl] durch Vierkomponenten-Kupplung bei Raumtemperatur; Verwendung von (S)-(+)-Mandelsäure als chiralem Hilfsstoff [HY* = (+)-MDLS)] und Ethanol als Lösungsmittel:

In einem 100ml-Dreihalskolben mit KPG-Rührer wurden 30 ml Ethanol (vergällt mit Methylethylketon) vorgelegt. Dazu gab man unter N₂-Atmosphäre bei Raumtemperatur (22°C) nacheinander 2,32 g (11,76 mmol, 1,00 Äquiv.) 2-Pyridylmethyl-phenyl-keton, 1,41 g (14,70 mmol, 1,25 Äquiv.) 2-Aminopyridin, 1,51 g (14,11 mmol, 1,20 Äquiv.)

Benzaldehyd, 3,65 g (23,52 mmol, 2,00 Äquiv.) (S)-(+)-Mandelsäure.Der mechanische Rührer wurde eingeschaltet und es bildete sich eine gelbe, leicht trübe Lösung. Nach 20 Min. war ein Niederschlag entstanden. Die Suspension wurde 3 Tage weiter bei Raumtemperatur gerührt. Eine Probe wurde analog zu Beispiel 27 entnommen und mit Pivaloylchlorid derivatisiert. Analyse erfolgte auf einer 250mm x 4,6 mm Säule Chiralpak AD isokratisch mit dem Eluenten 25% Isopropanol / 75% n-Hexan / 0,1 % Trifluoressigsäure. Bild und Spiegelbild wurden, ebenso wie bei einer entsprechend derivatisierten racemischen Referenzprobe, im Verhältnis 50 : 50 eluiert [ t(ret) = 12.25 und 14.46 Min.]. ¹H-NMR zeigte, daß das Mannich-Mandelatsalz in hoher Reinheit vorlag. Diastereomer oder Retro-Mannich-Produkte waren in der NMR-Lösung (DMSO-d6) in sehr geringer Menge nachweisbar.
Das Reaktionsgemisch wurde daraufhin 7 Std. auf 60°C erhitzt, dann auf RT abkühlen gelassen, der Feststoff abgesaugt, mit wenig Ethanol gewaschen und im HV getrocknet. Man erhielt 5,55 g (10,44 mmol, 88,8% d.Th.) blaßgelbes Pulver. Das ¹H-NMR-Spektrum war unverändert. Derivatisierung ergab, daß die Mannich-Base unverändert racemisch vorlag. Im Gegensatz zu Beispielen 27 und 29 bewirkt (S)-(+)-Mandelsäure im Lösungsmittel Ethanol hier also zwar Bildung der Mannich-Base aus den Reaktanten (IV), (V) und (Vl), sowie Kristallisation des Mandelatsalzes, aber keine dynamische Racematspaltung.
¹H-NMR (400 MHz, DMSO-d6): δ = 5,02 (s, 1H, CHOH des Mandelat-Anions), 5,68 (d, 1 H), 5,99 (t, 1H), 6,32 (d, 1H), 6,37 (t, 1H), 6,97 (d, 1H), 7,07 (t, 1H), 7,15 - 7,25 (m, 5H), 7,41 (t, 2H), 7,50 - 7,60 (m, 3H), 7,70 (t, 1H), 7,87 (d + m, 3H), 8,47 (d, 1H).

## Patentansprüche

1. Verfahren zur Herstellung einer Verbindung der Formel (III) oder deren Diastereomer, 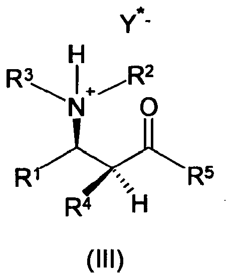 wobei
R¹
1. Wasserstoff,
2. tert.-Butyl-Gruppe oder
3. ein carbocyclischer oder heterocyclischer Arylrest R⁶ ist, wobei der Arylrest R⁶ ein carbocyclischer Arylrest mit 5-14 C-Atomen oder ein heterocyclischer Arylrest mit 5-14 C-Atomen ist, wobei 1 bis 4 C-Atome durch N, O oder S ersetzt sind,
wobei R⁶ unsubstituiert ist oder 1 bis 5 Substituenten trägt, die unabhängig voneinander (C₁-C₇)Alkyl, (C₃-C₇)Cycloalkyl, Alkanoyl (-CO-(C₁-C₇)Alkyl), Aroyl (-CO-(C₅-C₁₄)Aryl), Fluoro, Chloro, Bromo, lodo, Hydroxy, (C₁-C₇)Alkoxy, (C₃-C₇)Cycloalkoxy, (C₅-C₁₄)Aryloxy, (C₁-C₇)Alkanoyloxy, (C₅-C₁₄)Aroyloxy, -O-CO-NHR, -O-CO-NRR', -O-CO-OR, -O-CO-SR, -O-CS-NHR, -O-CS-NRR', -O-CS-OR, -O-CS-SR, -O-SO₂-(C₁-C₇)Alkyl, -O-SO₂-(C₅-C₁₄)Aryl), Nitro, -NH-CO-R, -NR'-CO-R, -NH-CO-OR, -NR'-CO-OR, -NH-CO-NHR, -NR'-CO-NHR, -NR'-CO-NRR", Di(C₁-C₇)alkylamino, Di(C₅-C₁₄)arylamino, N-(C₁-C₇)Alkyl-N-(C₅-C₁₄)aryl-amino, (C₁-C₇)Alkylthio, (C₅-C₁₄)Arylthio, (C₁-C₇)Alkylsulfonyl, (C₅-C₁₄)Arylsulfonyl, (C₅-C₁₄)Arylsulfoxidyl, oder ein unsubstituierter Arylrest R⁶ bedeuten,
wobei R, R' und R" jeweils unabhängig voneinander (C₁-C₇)Alkyl, (C₃-C₇)Cycloalkyl oder (C₅-C₁₄)Aryl bedeuten,
R², R³ und R⁴ unabhängig voneinander
1. Wasserstoff,
2. (C₁-C₇)Alkyl,
wobei (C₁-C₇)Alkyl unsubstituiert oder substituiert ist mit einem Arylrest R⁶,
3. (C₃-C₇)Cycloalkyl oder
4. ein Arylrest R⁶ bedeutet,
R⁵ ein Arylrest R⁶ ist,
und wobei das Anion Y^{*-} die konjugierte Base einer optisch aktiven, organischen Brönstedt-Säure (Protonen-Säure) ist,
**dadurch gekennzeichnet, daß**
die Verbindungen der Formeln (IV), (V), (VI) und (VII) 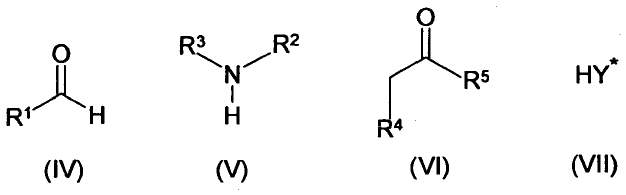 wobei die Reste R¹, R², R³, R⁴, R⁵ und Y^{*-} in den Verbindungen der Formeln (IV), (V), (VI) und (VII) wie oben definiert sind,
in einem geeigneten Lösungsmittel zur Verbindung der Formel (III) umgesetzt werden,
wobei entweder die Verbindungen der Formeln (IV), (V), (VI) und (VII) in einer direkten Mannich-Reaktion gleichzeitig umgesetzt werden,
oder zunächst die Verbindungen der Formeln (IV) und (V) zu einem Imin der Formel (X) oder zu einem Aminal der Formel (XI) reagieren, das optional isoliert werden kann, 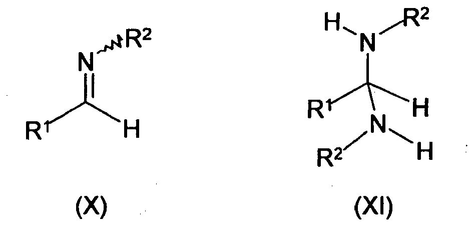 und die Verbindung der Formel (X) oder (XI) anschliessend unter Zusatz der Verbindungen der Formel (VI) und (VII) zu einer Verbindung der Formel (III) umgesetzt werden.

2. Verfahren gemäß Anspruch 1, wobei Y^{*-} eine optisch aktive natürlich vorkommende oder industriell hergestellte Carbonsäure ist.

3. Verfahren gemäß Anspruch 2, wobei die optisch aktive natürlich vorkommende oder industriell hergestellte Carbonsäure (R)-(-)-Mandelsäure, (S)-(+)-Mandelsäure, D-(-)-Weinsäure, L-(+)-Weinsäure, (+)-Di-O,O'-pivaloyl-D-weinsäure [(+)-DPWS], (-)-Di-O,O'-pivaloyl-L-weinsäure, [(-)-DPWS], (+)-O,O'-Dibenzoyl-D-weinsäure, (-)-O,O'-Dibenzoyl-L-weinsäure, (-)-Di-O,O'-benzoyl-L-weinsäure-mono(dimethylamid), (+)-O,O'-Dianisoyl-D-weinsäure [(+)-DAWS], (-)-O,O'-Dianisoyl-L-weinsäure [(-)-DAWS], (+)-Di-O,O'-p-Toluyl-D-weinsäure, (-)-Di-O,O'-p-Toluyl-L-weinsäure, D-(+)-Äpfelsäure, L-(-)-Äpfelsäure, L-(+)-Milchsäure, D-(-)-Milchsäure, (S)-(-)-2-(Phenylaminocarbonyloxy)-propionsäure, (R)-(+)-2-(Phenylaminocarbonyloxy)-propionsäure, D-(+)-Gluconsäure, (-)-2,3,4,6-Di-O-isopropyliden-2-keto-L-gulonsäure, (D)-(-)-Chinasäure, (-)-3,4,5-Trihydroxy-1-cyclohexen-1-carbonsäure [Shikimisäure], (S)-(+)-(2,2-Dimethyl-5-oxodioxolan-4-yl)-essigsäure, (+)-Camphersäure, (-)-Camphersäure, (1R)-(+)-Camphansäure, (1S)-(-)-Camphansäure, (R)-(-)-O-Acetylmandelsäure, (S)-(+)-O-Acetylmandelsäure, (R)-2-Phenoxy-propionsäure, (S)-2-Phenoxy-propionsäure, (S)-(+)-α-Methoxyphenylessigsäure, (R)-(-)-α-Methoxyphenylessigsäure, (R)-(+)-α-Methoxy-a-trifluormethylphenylessigsäure, (S)-(-)-α-Methoxy-a-trifluormethyl-phenylessigsäure, (S)-(+)-2-Phenyl-propionsäure, (R)-(-)-2-Phenylpropionsäure, (R)-(+)-2-Chlor-propionsäure, (S)-(-)-2-Chlor-propionsäure, (R)-(+)-N-(α-Methylbenzyl)phthalsäuremonoamid, (S)-(-)-N-(α-Methylbenzyl) phthalsäuremonoamid, (R)-(-)-5-Oxotetrahydrofuran-2-carbonsäure, (S)-(+)-5-Oxotetrahydrofuran-2-carbonsäure, D-(+)-3-Phenylmilchsäure, L-(-)-3-Phenylmilchsäure, L-(+)-α-Hydroxyisovaleriansäure, D-(-)-α-Hydroxyisovaleriansäure, (+)-Menthyloxyessigsäure, (-)-Menthyloxyessigsäure, (+)-mono-(1S)-Menthylphthalat, (-)-mono-(1R)-Menthylphthalat, (+)-trans-5-Norbornen-2,3-dicarbonsäure, (-)-trans-5-Norbornen-2,3-dicarbonsäure, (R)-(+)-Methylbernsteinsäure, (S)-(-)-Methylbernsteinsäure, (R)-(+)-6-Hydroxy-2,5,7,8-tetramethylchroman-2-carbonsäure [(R)-(+)-Trolox^{®}], (S)-(-)-6-Hydroxy-2,5,7,8-tetramethylchroman-2-carbonsäure [(S)-(-)-Trolox^{®}], (S)-(+)-2-(4-Isobutylphenyl)-propionsäure [(S)-Ibuprofen], (R)-(-)-2-(4-Isobutylphenyl)-propionsäure [(R)-Ibuprofen], (+)-2-(6-Methoxy-2-naphthyl)-propionsäure [(+)-Naproxen], (-)-2-(6-Methoxy-2-naphthyl)-propionsäure [(-)-Naproxen], sowie die verfügbaren natürlichen oder unnatürlichen α- oder β-Aminosäuren,
oder eine optisch aktive Sulfonsäure,
oder ein optisch aktives Phosphorsäure-, Phosphinsäure- oder Phosphonsäure-Derivat,
oder ein optisch aktives Phenol ist.

4. Verfahren gemäß einem der Ansprüche 1 bis 3, wobei das geeignete Lösungsmittel Wasser oder ein organisches Lösungsmittel, oder ein Gemisch von Wasser mit einem organischem Lösungsmittel ist, optional enthaltend ein löslichkeitsverbesserndes Additiv, wobei organische Lösungsmittel in 100%iger Reinheit oder in technischer Qualität vorliegen können.

5. Verfahren gemäß einem der Ansprüche 1 bis 4, wobei in der Reaktion vorhandenes Wasser durch azeotrope Destillation oder den Zusatz wasserbindender Additive entfernt wird.

6. Verfahren zur Herstellung einer Verbindung der Formel (I) oder dessen Enantiomer, 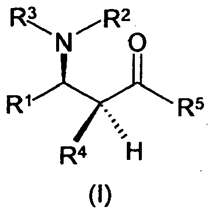 wobei die Reste R¹, R², R³, R⁴ und R⁵ in den Verbindungen der Formeln (IV), (V), (VI) und (VII) wie in Anspruch 1 definiert sind,
wobei in Verfahrensschritt 1 die Verbindungen der Formeln (IV), (V), (VI) und (VII) 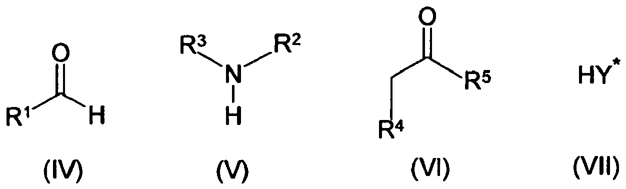 in einem geeigneten Lösungsmittel zur Verbindung der Formel (III) 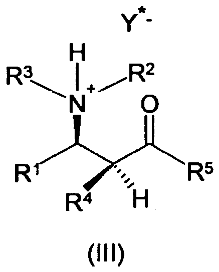 umgesetzt werden,
wobei Y^{*-} wie in Anspruch 1 definiert ist,
wobei entweder die Verbindungen der Formeln (IV), (V), (VI) und (VII) in einer direkten Mannich-Reaktion gleichzeitig umgesetzt werden,
oder zunächst die Verbindungen der Formeln (IV) und (V) zu einem Imin der Formel (X) oder zu einem Aminal der Formel (XI) reagieren, das optional isoliert werden kann, 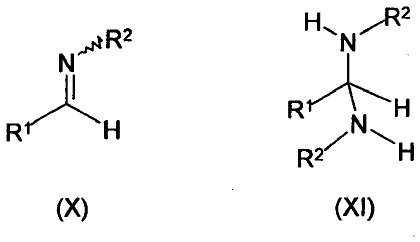 und die Verbindung der Formel (X) oder (XI) anschliessend unter Zusatz der Verbindungen der Formel (VI) und (VII) zu einer Verbindung der Formel (III) umgesetzt werden,
und in Verfahrensschritt 2 die Verbindung der Formel (III) unter Zusatz einer geeigneten Base in einem geeigneten Lösungsmittel umgesetzt wird.

7. Verfahren gemäß Anspruch 6, wobei eine geeignete Base ein organisches Amin oder ein Alkali- oder Erdalkali-hydrogencarbonat, -carbonate oder -hydroxide ist.

8. Verfahren gemäß Anspruch 7, wobei ein geeignetes Lösungsmittel Wasser oder organische Lösungsmittel, oder ein Gemisch von Wasser mit einem organischem Lösungsmittel ist, optional enthaltend ein löslichkeitsverbesserndes Additiv

9. Verfahren gemäß einem der Ansprüche 7 bis 8, wobei das organische Lösungsmittel ein C₁-C₈-Alkohol, verzweigt oder unverzweigt, oder ein ketonisches Lösungsmitteln, oder ein Ester, oder ein Ether, oder ein Kohlenwasserstoff, aliphatisch oder aromatisch, oder ein superkritisches Medium, oder ein halogenierter Kohlenwasserstoff, oder ein aprotisch-polares Lösungsmittel ist.

10. Verfahren zur Herstellung einer Verbindung der Formel (II) oder deren Enantiomer, 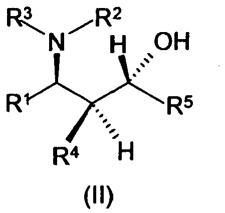 wobei die Reste R¹, R², R³, R⁴ und R⁵ in den Verbindungen der Formeln (IV), (V), (VI) und (VII) wie in Anspruch 1 definiert sind,
wobei in Verfahrensschritt 1 die Verbindungen der Formeln (IV), (V), (VI) und (VII) 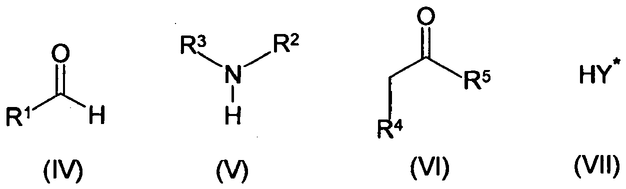 in einem geeigneten Lösungsmittel zur Verbindung der Formel (III) 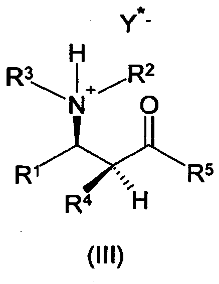 umgesetzt werden,
wobei Y^{*-} wie in Anspruch 1 definiert ist,
wobei entweder die Verbindungen der Formeln (IV), (V), (VI) und (VII) in einer direkten Mannich-Reaktion gleichzeitig umgesetzt werden,
oder zunächst die Verbindungen der Formeln (IV) und (V) zu einem Imin der Formel (X) oder zu einem Aminal der Formel (XI) reagieren, das optional isoliert werden kann, 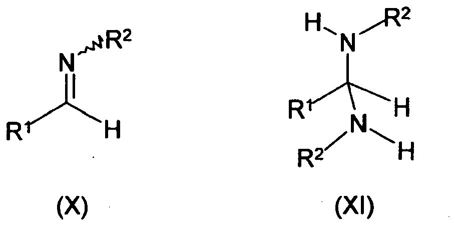 und die Verbindung der Formel (X) oder (XI) anschliessend unter Zusatz der Verbindungen der Formel (VI) und (VII) zu einer Verbindung der Formel (III) umgesetzt werden,
und in einem an Verfahrensschritt 1 anschliessenden Verfahrensschritt 2 die Verbindung der Formel (III) unter Zusatz einer geeigneten Base in einem geeigneten Lösungsmittel zu einer Verbindung der Formel (I) 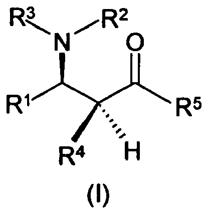 umgesetzt wird,
und in einem an Verfahrensschritt 2 anschliessenden Verfahrensschritt 3 die Verbindung der Formel (I) mit einem geeigneten Reduktionsmittel reduziert wird, und anschliessend optional aufgearbeitet und isoliert wird;
oder wobei alternativ zu den Verfahrensschritten 2 und 3 in einem an Verfahrensschritt 1 anschliessenden Verfahrensschritt 4 die Verbindung der Formel (III) mit einem geeigneten Reduktionsmittel zu der Verbindung der Formel (II) reduziert wird, und anschliessend optional aufgearbeitet und isoliert wird.

11. Verfahren gemäß Anspruch 10, wobei das Reduktionsmittel ein Boran- oder Borhydrid-Reagenz, optional in Gegenwart eines chiralen Katalysators ist.

12. Verfahren gemäß einem der Ansprüche 10 bis 11, wobei das Reduktionsmittel ein achirales Reduktionsmittel oder ein Reduktionsmittel enthaltend einen oder mehrere optisch aktive Katalysatoren ist.

13. Verfahren gemäß Anspruch 12, wobei das achirale Reduktionsmittel ausgewählt ist aus der Gruppe:
1. ein Boran-Sulfid-Komplex;
2. ein Boran-Etherat;
3. Catecholboran;
4. ein Boran-Sulfid-Komplex oder ein Boran-Etherat oder Catecholboran in Gegenwart einer Lewis-Säure;
5. ein Boran-Amin-Komplex;
6. ein Boran-Amin-Komplex in Gegenwart einer Lewissäure;
7. ein Boran-Phosphin-Komplex;
8. eine Kombination eines Borhydrids mit einem Reagenz, das zur in situ - Erzeugung von Boran führt;
9. ein Borhydrid eines ein- oder zweiwertigen Metallkations; oder
10. Diboran (B₂H₆).

14. Verfahren gemäß Anspruch 12, wobei das Reduktionsmittel enthaltend einen oder mehrere optisch aktive Katalysatoren ausgewählt ist aus der Gruppe
1. ein Borhydrid eines ein- oder zweiwertigen Metallkations in Gegenwart katalytischer Mengen eines optisch aktiven Aldiminato-Kobalt(II)-Komplexes mit oder ohne Gegenwart von Tetrahydrofurfurylalkohol als Co-Ligand;
2. ein Borhydrid eines ein- oder zweiwertigen Metallkations katalysiert durch einen Rhodium-Komplexes, der durch Koordination von zwei Molekülen optisch reinem 1,3-Aminoalkohol (II) pro Molekül [(µ⁵)-Pentamethyl-cyclopentadienyl]-rhodiumdichlorid-Dimer entsteht;
3. CATHy™ - Katalysatoren aus dem Cyclopentadienyl-rhodiumchlorid-Dimer und chiralen 1,2-Aminoalkoholen.

15. Verfahren gemäß einem der Ansprüche 10 bis 14, wobei das Reduktionsmittel ein Boran-Sulfid-Komplex, ein Boran-Etherat, Natriumborhydrid oder ein asymmetrischer Natriumborhydrid-Reduktionsmittel enthaltend einen in situ Katalysators, der durch Koordination von [(µ⁵)-Pentamethylcyclopentadienyl]-rhodiumdichlorid-Dimer an den optisch aktiven 1,3-Aminoalkohol (II) erzeugt wird, ist.

16. Verfahren gemäß einem der Ansprüche 10 bis 15, wobei das Reduktionsmittel ein Boran-Dimethylsulfid- oder Boran-Tetrahydrofuran-Komplex ist.

17. Verfahren gemäß einem der Ansprüche 10 bis 16, wobei die Verbindung der Formel (II) durch saure Solvolyse und/oder durch Kristallisation aufgearbeitet wird.

## Claims

1. A process for preparing a compound of the formula (III) or its diastereomer 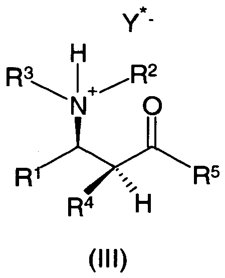 where
R¹ is
1. hydrogen,
2. a tert-butyl group or
3. a carbocyclic or heterocyclic aryl radical R⁶, where the aryl radical R⁶ is a carbocyclic aryl radical having 5-14 carbon atoms or a heterocyclic aryl radical having 5-14 carbon atoms, where from 1 to 4 carbon atoms are replaced by N, O or S,
where R⁶ is unsubstituted or bears from 1 to 5 substituents which are each independently (C₁-C₇)alkyl, (C₃-C₇)cycloalkyl, alkanoyl (-CO-(C₁-C₇)alkyl), aroyl (-CO-(C₅-C₁₄)aryl), fluoro, chloro, bromo, iodo, hydroxyl, (C₁-C₇)alkoxy, (C₃-C₇)cycloalkoxy, (C₅-C₁₄)aryloxy, (C₁-C₇)alkanoyloxy, (C₅-C₁₄)aroyloxy, -O-CO-NHR, -O-CO-NRR', -O-CO-OR, -O-CO-SR, -O-CS-NHR, -O-CS-NRR', -O-CS-OR, -O-CS-SR, -O-SO₂-(C₁-C₇)alkyl, -O-SO₂-(C₅-C₁₄)aryl, nitro, -NH-CO-R, -NR'-CO-R, -NH-CO-OR, -NR`-CO-OR, -NH-CO-NHR, -NR'-CO-NHR, -NR'-CO-NRR", di(C₁-C₇)alkylamino, di(C₅-C₁₄)arylamino, N-(C₁-C₇)alkyl-N-(C₅-C₁₄)arylamino, (C₁-C₇)alkylthio, (C₅-C₁₄)arylthio, (C₁-C₇)alkylsulfonyl, (C₅-C₁₄)arylsulfonyl, (C₅-C₁₄)arylsulfoxidyl, or an unsubstituted aryl radical R⁶,
where R, R' and R" are each independently (C₁-C₇)alkyl, (C₃-C₇)cycloalkyl or (C₅-C₁₄)aryl,
R² , R³ and R⁴ are each independently
1. hydrogen,
2. (C₁-C₇)alkyl,
where (C₁-C₇)alkyl is unsubstituted or substituted by an aryl radical R⁶,
3. (C₃-C₇)cycloalkyl or
4. an aryl radical R⁶, and
R⁵ is an aryl radical R⁶,
and where the anion Y^{*-} is the conjugated base of an optically active, organic Bronsted acid (protic acid),
which comprises
converting the compounds of the formulae (IV), (V), (VI) and (VII) 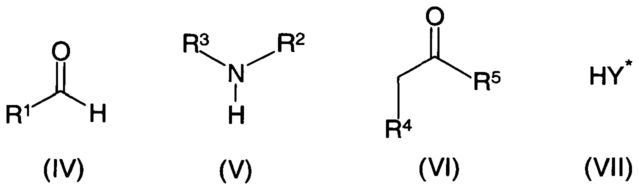 where the R¹, R², R³, R⁴, R⁵ and Y^{*-} radicals in the compounds of the formulae (IV), (V), (VI) and (VII) are defined as above,
in a suitable solvent to the compound of the formula (III),
by either reacting the compounds of the formulae (IV), (V), (VI) and (VII) at the same time in a direct Mannich reaction,
or initially reacting the compounds of the formulae (IV) and (V) to give an imine of the formula (X) or to an aminal of the formula (XI) which can optionally be isolated 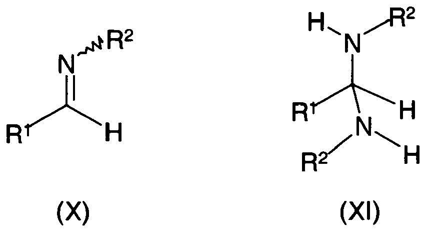 and then converting the compound of the formula (X) or (XI) with the addition of the compounds of the formula (VI) and (VII) to a compound of the formula (III).

2. The process as clalimed in claim 1, where Y^{*-} is an optically active, naturally occurring or industrially prepared carboxylic acid.

3. The process as claimed in claim 2, wherein the optically active, naturally occurring or industrially prepared carboxylic acid is (R)-(-)-mandelic acid, (S)-(+)-mandelic acid, D-(-)-tartaric acid, L-(+)-tartaric acid, (+)-di-O,O'-pivaloyl-D-tartaric acid [(+)-DPTA], (-)-di-O,O'-pivaloyl-L-tartaric acid, [(-)-DPTA], (+)-O,O'-dibenzoyl-D-tartaric acid, (-)-O,O`-dibenzoyl-L-tartaric acid, (-)-di-O,O'-benzoyl-L-tartaric mono(dimethylamide), (+)-O,O'-dianisoyl-D-tartaric acid [(+)-DATA], (-)-O,O'-dianisoyl-L-tartaric acid [(-)-DATA], (+)-di-O,O'-p-tolyl-D-tartaric acid, (-)-di-O.O'-p-tolyl-L-tartaric acid, D-(+)-malic acid, L-(-)-malic acid, L-(+)-lactic acid, D-(-)-lactic acid, (S)-(-)-2-(phenylaminocarbonyloxy)propionic acid, (R)-(+)-2-(phenylaminocarbonyloxy)propionic acid, D-(+)-gluconic acid, (-)-2,3,4,6-di-O-isopropylidene-2-keto-L-gulonic acid, (D)-(-)-quinic acid, (-)-3,4,5-trihydroxy-1-cyclohexene-1-carboxylic acid [shikimic acid], (S)-(+)-(2,2-dimethyl-5-oxodioxolan-4-yl)acetic acid, (+)-camphoric acid, (-)-camphoric acid, (1 R)-(+)-camphanic acid, (1 S)-(-)-camphanic acid, (R)-(-)-O-acetylmandelic acid, (S)-(+)-O-acetylmandelic acid, (R)-2-phenoxypropionic acid, (S)-2-phenoxypropionic acid, (S)-(+)-α-methoxyphenylacetic acid, (R)-(-)-α-methoxyphenylacetic acid, (R)-(+)-α-methoxy-α-trifluoromethylphenylacetic acid, (S)-(-)-α-methoxy-α-trifluoromethylphenylacetic acid, (S)-(+)-2-phenylpropionic acid, (R)-(-)-2-phenylpropionic acid, (R)-(+)-2-chloropropionic acid, (S)-(-)-2-chloropropionic acid, (R)-(+)-N-(α-methylbenzyl)phthalic monoamide, (S)-(-)-N-(α-methylbenzyl)phthalic monoamide, (R)-(-)-5-oxotetrahydrofuran-2-carboxylic acid, (S)-(+)-5-oxotetrahydrofuran-2-carboxylic acid, D-(+)-3-phenyllactic acid, L-(-)-3-phenyllactic acid, L-(+)-α-hydroxyisovaleric acid, D-(-)-α-hydroxyisovaleric acid, (+)-menthyloxyacetic acid, (-)-menthyloxyacetic acid, (+)-mono-(1S)-menthyl phthalate, (-)-mono-(1R)-menthyl phthalate, (+)-trans-5-norbornene-2,3-dicarboxylic acid, (-)-trans-5-norbornene-2,3-dicarboxylic acid, (R)-(+)-methylsuccinic acid, (S)-(-)-methylsuccinic acid, (R)-(+)-6-hydroxy-2,5,7,8-tetramethylchroman-2-carboxylic acid [(R)-(+)-Trolox^{®}], (S)-(-)-6-hydroxy-2,5,7,8-tetramethylchroman-2-carboxylic acid [(S)-(-)-Trolox^{®}], (S)-(+)-2-(4-isobutylphenyl)propionic acid [(S)-ibuprofen], (R)-(-)-2-(4-isobutylphenyl)propionic acid [(R)-ibuprofen], (+)-2-(6-methoxy-2-naphthyl)propionic acid [(+)-naproxen], (-)-2-(6-methoxy-2-naphthyl)propionic acid [(-)-naproxen], and also the available natural or unnatural α- or β-amino acids,
or an optically active sulfonic acid,
or an optically active phosphoric acid, phosphinic acid or phosphonic acid derivative,
or an optically active phenol.

4. The process as claimed in one of claims 1 to 3, wherein the suitable solvent is water or an organic solvent, or a mixture of water with an organic solvent, optionally comprising a solubility-enhancing additive, where organic solvents may be present in 100% purity or technical quality.

5. The process as claimed in one of claims 1 to 4, wherein water present in the reaction is removed by azeotropic distillation or by adding water-binding additives.

6. A process for preparing a compound of the formula (I) or its enantiomer 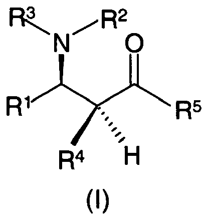 where the R¹, R², R³, R⁴ and R⁵ radicals in the compounds of the formulae (IV), (V), (VI) and (VII) are each as defined in claim 1,
wherein, in process step 1, the compounds of the formulae (IV), (V), (VI) and (VII) 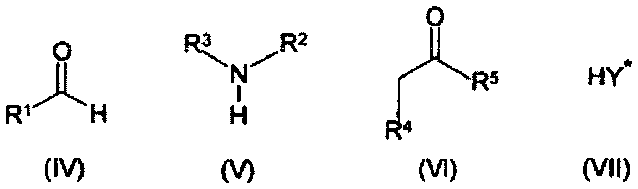 are converted in a suitable solvent to the compound of the formula (III) 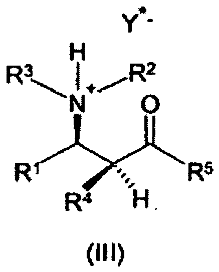 where Y^{*-} is as defined in claim 1,
by either reacting the compounds of the formulae (IV), (V), (VI) and (VII) at the same time in a direct Mannich reaction,
or initially reacting the compounds of the formulae (IV) and (V) to give an amine of the formula (X) or to give an aminal of the formula (XI) which can optionally be isolated 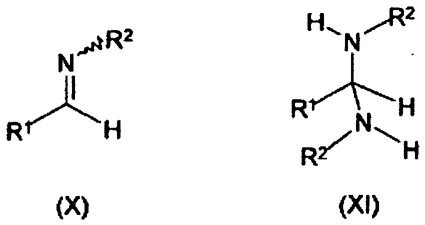 and then converting the compound of the formula (X) or (XI) with the addition of the compounds of the formula (VI) and (VII) to a compound of the formula (III),
and, in process step 2, the compound of the formula (III) is converted with the addition of a suitable base in a suitable solvent.

7. The process as claimed in claim 6, wherein a suitable base is an organic amine or an alkali metal hydrogencarbonate, carbonate or hydroxide, or an alkaline earth metal hydrogencarbonate, carbonate or hydroxide.

8. The process as claimed in claim 7, wherein a suitable solvent is water or an organic solvent, or a mixture of water with an organic solvent, optionally comprising a solubility-enhancing additive.

9. The process as claimed in one of claims 7 and 8, wherein the organic solvent is a C₁-C₈-alcohol, branched or unbranched, or a ketonic solvent, or an ester, or an ether, or a hydrocarbon, aliphatic or aromatic, or a supercritical medium, or a halogenated hydrocarbon, or a polar, aprotic solvent.

10. A process for preparing a compound of the formula (II) or its enantiomer, 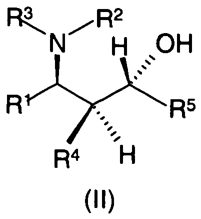 where the R¹, R², R³, R⁴ and R⁵ radicals in the compounds of the formulae (IV), (V), (VI) and (VII) are each as defined in claim 1,
wherein, in process step 1, the compounds of the formulae (IV), (V), (VI) and (VII) 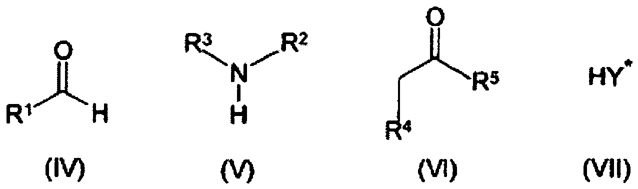 are converted in a suitable solvent to the compound of the formula (III) 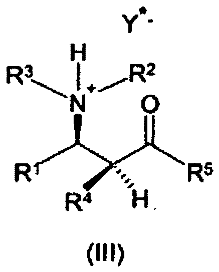 where Y^{*-} is as defined in claim 1,
by either reacting the compounds of the formulae (IV), (V), (VI) and (VII) at the same time in a direct Mannich reaction,
or initially reacting the compounds of the formulae (IV) and (V) to give an amine of the formula (X) or to give an aminal of the formula (XI) which can optionally be isolated 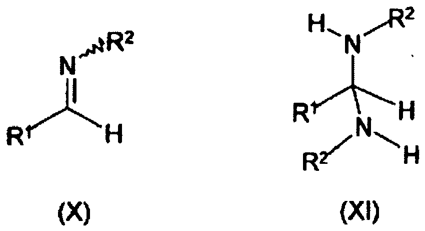 and then converting the compound of the formula (X) or (XI) with the addition of the compounds of the formula (VI) and (VII) to a compound of the formula (III),
and, in a process step 2 following process step 1, the compound of the formula (III) is converted with the addition of a suitable base in a suitable solvent to a compound of the formula (I), 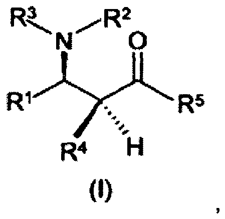 and, in a process step 3 following process step 2, the compound of the formula (I) is reduced with a suitable reducing agent, and then optionally worked up and isolated;
or where, as an alternative to process steps 2 and 3, in a process step 4 following process step 1, the compound of the formula (III) is reduced with a suitable reducing agent to the compound of the formula (II), and then optionally worked up and isolated.

11. The process as claimed in claim 10, wherein the reducing agent is a borane or borohydride reagent, optionally in the presence of a chiral catalyst.

12. The process as claimed in one of claims 10 and 11, wherein the reducing agent is an achiral reducing agent or a reducing agent comprising one or more optically active catalysts.

13. The process as claimed in claim 12, wherein the achiral reducing agent is selected from the group of:
1. a borane-sulfide complex;
2. a borane etherate;
3. catecholborane;
4. a borane-sulfide complex or a borane etherate or catecholborane in the presence of a Lewis acid;
5. a borane-amine complex;
6. a borane-amine complex in the presence of a Lewis acid;
7. a borane-phosphine complex;
8. a combination of a borohydride with a reagent which leads to in situ generation of borane;
9. a borohydride of a mono- or bivalent metal cation; or
10. diborane (B₂H₆).

14. The process as claimed in claims 12, wherein the reducing agent comprising one or more optically active catalysts, is selected from the group of
1. a borohydride of a mono- or bivalent metal cation in the presence of catalytic amounts of an optically active aldiminato cobalt(II) complex, in the presence or absence of tetrahydrofurfuryl alcohol as a coligand;
2. a borohydride of a mono- or bivalent metal cation catalyzed by a rhodium complex which results from the coordination of two molecules of optically pure 1,3-amino alcohol (II) per molecule of [(µ⁵)-pentamethylcyclopentadienyl]rhodium dichloride dimer;
3. CATHy™ - catalysts composed of the cyclopentadienylrhodium chloride dimer and chiral 1,2-amino alcohols.

15. The process as claimed in one of claims 10 to 14, wherein the reducing agent is a borane-sulfide complex, a borane etherate, sodium borohydride or an asymetric sodium borohydride reducing agent comprising an in situ catalyst which is obtained by the coordination of the [(µ⁵)-pentamethylcyclopentadienyl]rhodium dichloride dimer to the optically active 1,3-amino alcohol (II).

16. The process as claimed in one of claims 10 to 15, wherein the reducing agent is a borane-dimethyl sulfide or borane-tetrahydrofuran complex.

17. The process as claimed in one of claims 10 to 16, wherein the compound of the formula (II) is worked up by acidic solvolysis and/or by crystallization.

## Revendications

1. Procédé pour la préparation d'un composé de formule (III) ou son
diastéréo-isomère, 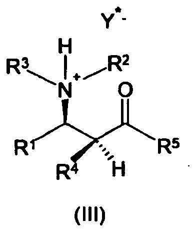 où
R¹
1. signifie hydrogène,
2. signifie un groupe tert-butyle ou
3. représente un radical aryle carbocyclique ou hétérocyclique R⁶, le radical aryle R⁶ représentant un radical aryle carbocyclique comprenant 5-14 atomes de carbone ou un radical aryle hétérocyclique comprenant 5-14 atomes de carbone, 1 à 4 atomes de carbone étant remplacés par N, O ou S,
R⁶ étant non substitué ou portant 1 à 5 substituants, qui signifient, indépendamment l'un de l'autre, (C₁-C₇)alkyle, (C₃-C₇)cycloalkyle, alcanoyle (-CO-(C₁-C₇)alkyle), aroyle (-CO-(C₅-C₁₄)aryle), fluoro, chloro, bromo, iodo, hydroxy, (C₁-C₇)alcoxy, (C₃-C₇)cycloalcoxy, (C₅-C₁₄)aryloxy, (C₁-C₇)alcanoyloxy, (C₅-C₁₄)aroyloxy, -O-CO-NHR, -O-CO-OR, -O-CO-SR, -O-CS-NHR, -O-CS-NRR', -O-CS-NRR', -O-CS-OR, -O-CS-SR, -O-SO₂-(C₁-C₇)alkyle, -O-SO₂-(C₅-C₁₄)aryle, nitro, -NH-CO-R, -NR'-CO-R, -NH-CO-OR, -NR'-CO-OR, -NH-CO-NHR, -NR'-CO-NHR, -NR'-CO-NRR", di(C₁-C₇)alkylamino, di(C₅-C₁₄)arylamino, N-(C₁-C₇)alkyl-N-(C₅-C₁₄)arylamino, (C₁-C₇)alkylthio, (C₅-C₁₄)arylthio, (C₁-C₇)alkylsulfonyle, (C₅-C₁₄)arylsulfonyle, (C₅-C₁₄)arylsulfoxydyle, ou un radical aryle R⁶ non substitué, R, R' et R" signifiant, à chaque fois indépendamment l'un de l'autre, (C₁-C₇)alkyle, (C₃-C₇)cycloalkyle ou (C₅-C₁₄)aryle,
R², R³ et R⁴ indépendamment l'un de l'autre, signifient
1. hydrogène,
2. (C₁-C₇)alkyle, (C₁-C₇)alkyle étant non substitué ou substitué par un radical aryle R⁶,
3. (C₃-C₇)cycloalkyle ou
4. un radical aryle R⁶,
R⁵ signifie un radical aryle R⁶,
et l'anion Y^{*-} étant la base conjuguée d'un acide de Brönstedt organique, optiquement actif (acide protonique),
**caractérisé en ce que**
les composés des formules (IV), (V), (VI) et (VII) 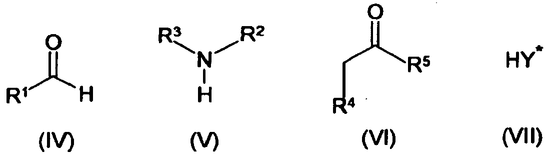 les radicaux R¹, R², R³, R⁴, R⁵ et Y^{*-} dans les composés de formule (IV), (V), (VI) et (VII) étant définis comme ci-dessus,
sont transformés dans un solvant approprié en composé de formule (III) soit les composés des formules (IV), (V), (VI) et (VII) étant transformés simultanément dans une réaction de Mannich directe,
soit les composés des formules (IV) et (V) réagissant d'abord en une imine de formule (X) ou en un aminal de formule (XI), qui peut éventuellement être isolé(e), 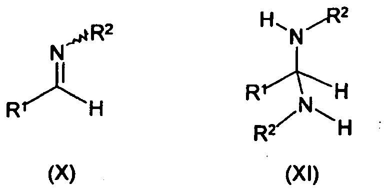 et le composé de formule (X) ou (XI) étant ensuite transformé, en ajoutant les composés de formule (VI) et (VII), en un composé de formule (III).

2. Procédé selon la revendication 1, Y^{*-} étant un acide carboxylique optiquement actif naturel ou préparé par voie industrielle.

3. Procédé selon la revendication 2, l'acide carboxylique optiquement actif, naturel ou préparé par voie industrielle étant l'acide (R)-(-)-mandélique, l'acide (S)-(+)-mandélique, l'acide D-(-)-tartrique, l'acide L-(+)-tartrique, l'acide (+)-di-O,O'-pivaloyl-D-tartrique [(+)-DPWS], l'acide (-)-di-O,O'-pivaloyl-L-tartrique, [(-)-DPWS], l'acide (+)-O,O'-dibenzoyl-D-tartrique, l'acide (-)-O,O'-dibenzoyl-L-tartrique, le mono(diméthylamide) de l'acide (-)-di-O,O'-benzoyl-L-tartrique, l'acide (+)-O,O'-dianisoyl-D-tartrique [(+)-DAWS], l'acide (-)-O,O'-dianisoyl-L-tartique [(-)-DAWS], l'acide (+)-di-O,O'-p-toluyl-D-tartrique, l'acide (-)-di-O,O'-p-toluyl-L-tartrique, l'acide D-(+)-malique, l'acide L-(-)-malique, l'acide L-(+)-lactique, l'acide D-(-)-lactique, l'acide (S)-(-)-2-(phénylaminocarbonyloxy)-propionique, l'acide (R)-(+)-2-(phénylaminocarbonyloxy)-propionique, l'acide D-(+)-gluconique, l'acide (-)-2,3,4,6-di-O-isopropylidène-2-céto-L-gulonique, l'acide (D)-(-)-quinique, l'acide (-)-3,4,5-trihydroxy-1-cyclohexène-1-carboxylique [l'acide shikimique], l'acide (S)-(+)-(2,2-diméthyl-5-oxodioxolan-4-yl)-acétique, l'acide (+)-camphorique, l'acide (-)-camphorique, l'acide (1R)-(+)-camphanique, l'acide (1 S)-(-)-camphanique, l'acide (R)-(-)-O-acétylmandélique, l'acide (S)-(+)-O-acétylmandélique, l'acide (R)-2-phénoxypropionique, l'acide (S)-2-phénoxypropionique, l'acide (S)-(+)-α-méthoxyphénylacétique, l'acide (R)-(-)-α-méthoxyphénylacétique, l'acide (R)-(+)-α-méthoxy-α-trifluorométhylphénylacétique, l'acide (S)-(-)-α-méthoxy-α-trifluorométhylphénylacétique, l'acide (S)-(+)-2-phénylpropionique, l'acide (R)-(-)-2-phénylpropionique, l'acide (R)-(+)-2-chloropropionique, l'acide (S)-(-)-2-chloropropionique, le monoamide de l'acide (R)-(+)-N-(α-méthylbenzyl)phtalique, le monoamide de l'acide (S)-(-)-N-(α-méthylbenzyl)phtalique, l'acide (R)-(-)-5-oxotétrahydrofuranne-2-carboxylique, l'acide (S)-(+)-5-oxotétrahydrofuranne-2-carboxylique, l'acide D-(+)-3-phényllactique, l'acide L-(-)-3-phényllactique, l'acide L-(+)-α-hydroxyisovalérianique, l'acide D-(-)-α-hydroxyisovalérianique, l'acide (+)-menthyloxyacétique, l'acide (-)-menthyloxyacétique, le phtalate de (+)-mono-(1S)-menthyle, le phtalate de (-)-mono-(1R)-menthyle, l'acide (+)-trans-5-norbornène-2,3-dicarboxylique, l'acide (-)-trans-5-norbornène-2,3-dicarboxylique, l'acide (R)-(+)-méthylsuccinique, l'acide (S)-(-)-méthylsuccinique, l'acide (R)-(+)-6-hydroxy-2,5,7,8-tétraméthylchromane-2-carboxylique [(R)-(+)-Trolox®], l'acide (S)-(-)-6-hydroxy-2,5,7,8-tétraméthylchromane-2-carboxylique [(S)-(-)-Trolox®], l'acide (S)-(+)-2-(4-isobutylphényl)-propionique [(S)-ibuprofène], l'acide (R)-(-)-2-(4-isobutylphényl)-propionique [(R)-ibuprofène], l'acide (+)-2-(6-méthoxy-2-naphtyl)-propionique [(+)-naproxène], l'acide (-)-2-(6-méthoxy-2-naphtyl)-propionique [(-)-naproxène], ainsi que les acides α-aminés ou ß-aminés naturels ou non naturels disponibles,
ou un acide sulfonique optiquement actif,
ou un dérivé optiquement actif de l'acide phosphorique, phosphinique ou phosphonique,
ou un phénol optiquement actif.

4. Procédé selon l'une quelconque des revendications 1 à 3, le solvant approprié étant l'eau ou un solvant organique ou un mélange d'eau et d'un solvant organique, contenant éventuellement un additif améliorant la solubilité, le solvant organique pouvant se trouver en une pureté de 100% ou sous forme de qualité technique.

5. Procédé selon l'une quelconque des revendications 1 à 4, l'eau dans la réaction présente étant éliminée par distillation azéotrope ou par l'addition d'additifs liant l'eau.

6. Procédé pour la préparation d'un composé de formule (I) ou son énantiomère 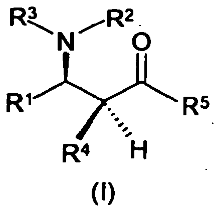 les radicaux R¹, R², R³, R⁴ et R⁵ dans les composés des formules (IV), (V), (VI) et (VII) étant définis comme dans la revendication 1,
où, dans l'étape de procédé 1, les composés des formules (IV), (V), (VI) et (VII) 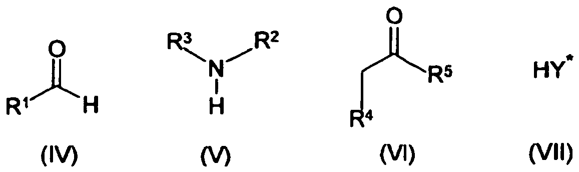 sont transformés dans un solvant approprié en composé de formule (III) 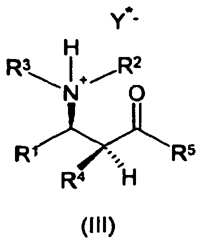 Y^{*-} étant défini comme dans la revendication 1,
soit les composés des formules (IV), (V), (VI) et (VII) étant transformés simultanément dans une réaction de Mannich directe,
soit les composés des formules (IV) et (V) réagissant d'abord en une imine de formule (X) ou en un aminal de formule (XI), qui peut éventuellement être isolé(e), 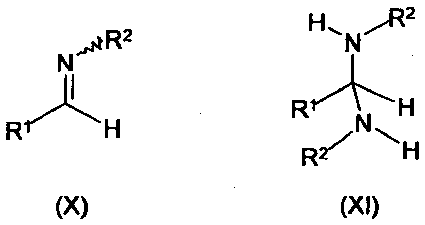 et le composé de formule (X) ou (XI) étant ensuite transformé, en ajoutant les composés de formule (VI) et (VII), en un composé de formule (III),
et dans l'étape de procédé 2, le composé de formule (III) est transformé en ajoutant une base appropriée dans un solvant approprié.

7. Procédé selon la revendication 6, une base appropriée étant une amine organique ou un hydrogénocarbonate, un carbonate ou un hydroxyde de métal alcalin ou alcalino-terreux.

8. Procédé selon la revendication 7, un solvant approprié étant l'eau ou un solvant organique ou un mélange d'eau et d'un solvant organique, contenant éventuellement un additif améliorant la solubilité.

9. Procédé selon l'une quelconque des revendications 7 à 8, le solvant organique étant un C₁-C₈-alcool, ramifié ou non ramifié, ou un solvant cétonique ou un ester ou un éther ou un hydrocarbure, aliphatique ou aromatique, ou un milieu supercritique ou un hydrocarbure halogéné ou un solvant aprotique polaire.

10. Procédé pour la préparation d'un composé de formule (II) ou son
énantiomère 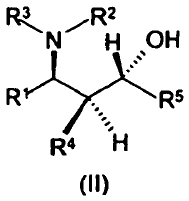 les radicaux R¹, R², R³, R⁴ et R⁵ dans les composés des formules (IV), (V), (VI) et (VII) étant définis comme dans la revendication 1,
où, dans l'étape de procédé 1, les composés des formules (IV), (V), (VI) et (VII) 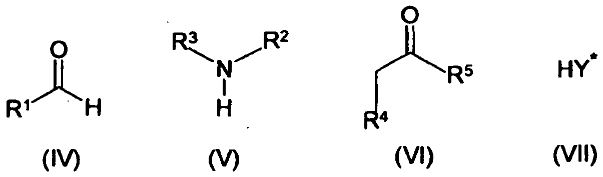 sont transformés dans un solvant approprié en composé de formule (III) 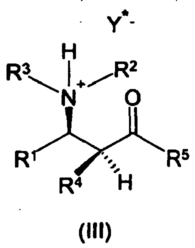 Y^{*-} étant défini comme dans la revendication 1,
soit les composés des formules (IV), (V), (VI) et (VII) étant transformés simultanément dans une réaction de Mannich directe,
soit les composés des formules (IV) et (V) réagissant d'abord en une imine de formule (X) ou en un aminal de formule (XI), qui peut éventuellement être isolé(e), 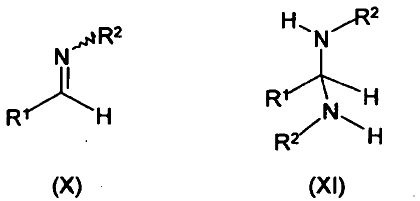 et le composé de formule (X) ou (XI) étant ensuite transformé, en ajoutant les composés de formule (VI) et (VII), en un composé de formule (III),
et dans l'étape de procédé 2 consécutive à l'étape de procédé 1, le composé de formule (III) est transformé en ajoutant une base appropriée dans un solvant approprié en un composé de formule (I) 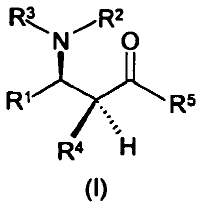 et dans une étape de procédé 3 consécutive à l'étape de procédé 2, le composé de formule (I) est réduit avec un réducteur approprié, puis éventuellement traité et isolé ;
ou, en variante des étapes de procédés 2 et 3, dans une étape de procédé 4 consécutive à l'étape de procédé 1, le composé de formule (III) est réduit avec un réducteur approprié en composé de formule (II), puis éventuellement traité et isolé.

11. Procédé selon la revendication 10, le réducteur étant un réactif de borane ou de borohydrure, éventuellement en présence d'un catalyseur chiral.

12. Procédé selon l'une quelconque des revendications 10 à 11, le réducteur étant un réducteur achiral ou un réducteur contenant un ou plusieurs catalyseurs optiquement actifs.

13. Procédé selon la revendication 12, le réducteur achiral étant choisi dans le groupe formé par :
1. un complexe borane-sulfure ;
2. un éthérate de borane ;
3. le catécholborane ;
4. un complexe borane-sulfure ou un éthérate de borane ou le catécholborane en présence d'un acide de Lewis;
5. un complexe borane-amine ;
6. un complexe borane-amine en présence d'un acide de Lewis ;
7. un complexe borane-phosphine ;
8. une combinaison d'un borohydrure avec un réacteur qui conduit à une génération in situ de borane ;
9. un borohydrure d'un cation métallique monovalent ou divalent ou
10. le diborane (B₂H₆).

14. Procédé selon la revendication 12, le réducteur contenant un ou plusieurs catalyseurs optiquement actifs étant choisi dans le groupe formé par
1. un borohydrure d'un cation métallique monovalent ou divalent en présence de quantités catalytiques d'un complexe aldiminato-cobalt (II) optiquement actif en présence ou non d'alcool tétrahydrofurfurylique comme coligand
2. un borohydrure d'un cation métallique monovalent ou divalent, catalysé par un complexe au rhodium, qui est formé par coordination de deux molécules de 1,3-aminoalcool optiquement actif (II) par molécule de dimère [(µ⁵)-pentaméthylcyclopentadiényl]-dichlorure de rhodium ;
3. les catalyseurs CATHy™ constitués par le dimère cyclopentadiényl-dichlorure de rhodium et des 1,2-aminoalcools dimères.

15. Procédé selon l'une quelconque des revendications 10 à 14, le réducteur étant un complexe borane-sulfure, un éthérate de borane, le borohydrure de sodium ou un réducteur de type borohydrure de sodium asymétrique contenant un catalyseur in situ, qui est généré par coordination de dimère [(µ⁵)-pentaméthylcyclopentadiényl]-dichlorure de rhodium sur le 1,3-aminoalcool optiquement actif (II).

16. Procédé selon l'une quelconque des revendications 10 à 15, le réducteur étant un complexe borane-diméthylsulfure ou un complexe borane-tétrahydrofuranne.

17. Procédé selon l'une quelconque des revendications 10 à 16, le composé de formule (II) étant traité par solvolyse acide et/ou par cristallisation.
